# EUROPEAN PATENT SPECIFICATION

(11) **EP 4 139 482 B1**
(45) Date of publication and mention of the grant of the patent: **14.08.2024**
(21) Application number: 21721905.4
(22) Date of filing: 23.04.2021
(51) Int. Cl.: C12Q 1/6806, C12Q 1/6853, C12N 15/10

(54) **SINGLE STEP SAMPLE PREPARATION FOR NEXT GENERATION SEQUENCING**
EINSTUFIGE PROBENVORBEREITUNG ZUR SEQUENZIERUNG DER NÄCHSTEN GENERATION
PRÉPARATION D'ÉCHANTILLON EN UNE SEULE ÉTAPE POUR SÉQUENÇAGE DE NOUVELLE GÉNÉRATION

(30) Priority: 24.04.2020 EP 20171403; 20.05.2020 EP 20175796
(43) Date of publication of application: 01.03.2023
(73) Proprietor: Medicover GmbH, 12247 Berlin (DE)
(72) Inventor: WACHTER, Oliver Gediminas, 86981 Kinsau (DE); HIRV, Kaimo, 82061 Neuried (DE); KLEIN, Hanns-Georg, 81479 München (DE)
(74) Representative: CH Kilger Anwaltspartnerschaft mbB
(86) International application number: PCT/EP2021/060704
(87) International publication number: WO 2021/214307

(56) References cited:
- WO-A1-2010/115154
- WO-A1-2014/100866
- WO-A1-2017/087873
- WO-A1-2019/204588
- HOSSAIN AYAAN ET AL: "A Massively Parallel COVID-19 Diagnostic Assay for Simultaneous Testing of 19200 Patient Samples", 20 March 2020 (2020-03-20), XP055822933, Retrieved from the Internet <URL:https://docs.google.com/document/d/1kP2w_uTMSep2UxTCOnUhh1TMCjWvHEY0sUUpkJHPYV4/edit> [retrieved on 20210709]
- VINZENZ LANGE ET AL: "Cost-efficient high-throughput HLA typing by MiSeq amplicon sequencing", BMC GENOMICS, BIOMED CENTRAL LTD, LONDON, UK, vol. 15, no. 1, 24 January 2014 (2014-01-24), pages 63, XP021175404, ISSN: 1471-2164, DOI: 10.1186/1471-2164-15-63
- FEI JI-FENG ET AL: "Application and optimization of CRISPR-Cas9-mediated genome engineering in axolotl (Ambystoma mexicanum)", NATURE PROTOCOLS, NATURE PUBLISHING GROUP, GB, vol. 13, no. 12, 14 November 2018 (2018-11-14), pages 2908 - 2943, XP036687564, ISSN: 1754-2189, [retrieved on 20181114], DOI: 10.1038/S41596-018-0071-0
- ERICJ DE MUINCK ET AL: "A novel ultra high-throughput 16S rRNA gene amplicon sequencing library preparation method for the Illumina HiSeq platform", MICROBIOME, BIOMED CENTRAL LTD, LONDON, UK, vol. 5, no. 1, 6 July 2017 (2017-07-06), pages 1 - 15, XP021246865, DOI: 10.1186/S40168-017-0279-1

## Description

### FIELD OF THE INVENTION

The present invention relates to a method for preparing an RNA sample for a target specific next generation sequencing comprising performing a one-step target enrichment in a single reaction vessel or in a single reaction mixture, as well as a kit for preparing an RNA sample for next generation sequencing in a one-step target enrichment. Further envisaged is the use of the method or the kit for an enrichment for a rapid virus detection, or for an enrichment for a rapid leukocyte antigen-associated gene identification or for an enrichment for a rapid blood group associated gene identification.

### BACKGROUND OF THE INVENTION

In the past 15 years, a variety of Next Generation Sequencing (NGS) technologies have been developed after the founding sequencing method of Sanger dideoxy synthesis in 1977. Next Generation Sequencing (NGS), also known as high-throughput sequencing, represents an assortment of sequencing methods which transcend the capacity of traditional DNA sequencing technologies in respect to cost, speed and data output. This technology supports a massively parallel sequencing and thus, allowing rapid analysis of a multitude of samples. There is a variety of NGS platforms using different sequencing technologies which can be grouped into two major categories, sequencing by hybridization and sequencing by synthesis (SBS).

Sequencing by hybridization uses arrayed DNA oligonucleotides of known sequences on filters that were hybridized to labelled fragments of the DNA to be sequenced. By repeatedly hybridizing and washing away the unwanted non-hybridized DNA, it is possible to determine whether the hybridizing labelled fragments matches the sequence of the DNA probes on the filter. This technology depends on using specific probes to interrogate sequences, such as in diagnostic applications for identifying disease-related SNPS (single-nucleotide polymorphisms) in specific genes or identifying chromosome abnormalities (Slatko et al., Curr Protoc Mol Biol., 2018, 122(1): e59).

SBS methods are a further development of Sanger sequencing, without the dideoxy terminators, in combination with repeated cycles of synthesis, imaging, and methods to incorporate additional nucleotides in the growing chain. Two major SBS technologies are prevalent on the market, the Ion Torrent technology and the Illumina technology.

The Illumina technology is defined by their use of terminator molecules that are similar to those used in Sanger sequencing, in which the ribose 3'-OH group is blocked, thus preventing elongation. The technology is based on a so-called "bridge amplification" wherein DNA molecules with appropriate adaptors ligated on each end are used as substrates for repeated amplification synthesis reaction on a solid support (i.e. glass slide) that contains oligonucleotide sequences complementary to a ligated adaptor. The oligonucleotides on the slide are spaced such that the DNA, which is then subjected to repeated rounds of amplification, creates clonal "clusters" consisting of about 1000 copies of each oligonucleotide fragment. During the synthesis, the nucleotides carrying each a different fluorescent label are incorporated and then detected by direct imaging (Slatko et al., Curr Protoc Mol Biol., 2018, 122(1): e59). The nucleotide label serves as a terminator for polymerization, so after each dNTP incorporation, the fluorescent dye is imaged to identify the base and then enzymatically cleaved to allow incorporation of the next nucleotide.

A similar well-known sequencing-by-synthesis technology is ion semiconductor sequencing, often referred to as Ion Torrent technology. This method is based on the detection of hydrogen ions that are released during the polymerization of DNA. As such, no images are created and analysed, as opposed to various other techniques. Unlike the Illumina technology, this approach relies on a single signal to mark the incorporation of a dNTP into an elongating strand. As a consequence, an iterative addition of each of the four nucleotides to a sequencing reaction is necessary to ensure only one dNTP is responsible for the signal. Another difference of the Ion Torrent technology lies in the dNTPs themselves, which do not require to be blocked, as the absence of the next nucleotide in the sequencing reaction prevents elongation (Goodwin et al., Nat Rev Genet, 2016; 17(6), 333-51).

The sample preparation for many sequencing-by-synthesis approaches are generally rather similar and may comprise a) fragmentation of DNA sequences into suitable sizes (between 25-600 bps), b) target enrichment, c) adapter ligation, and d) attachment of indices or barcodes to distinguish between the multitudes of samples. However, the sample preparation is highly time-consuming due to the many steps and error-prone if not performed with due care. A major bottleneck and speed-limiting step for NGS sample preparation has been the selective enrichment of a target, the attachment of required sequences, such as indices and adaptors and the various purification steps between each of these steps. As a result, it usually takes as long as 2-4 days to generate a sample that is ready to be sequenced and another 1-2 days to complete an entire sequencing process. WO2017087873A1 discloses dual indexing of mRNA by providing the reverse transcription primer and the indexing primers in separate steps.

In times when a rapid and accurate method of analysing samples is required, such as seen with the outbreak of the COVID-19 pandemic, time-consuming and complex sample preparation proves to be rather challenging in view of the flood of samples to be analysed.

Hence, there is a need for a fast sample preparation for NGS application, which avoids time consuming process steps and can be automated.

### OBJECTS AND SUMMARY OF THE INVENTION

The present invention addresses these needs and provides in one aspect a method for preparing an RNA sample for a target specific next generation sequencing comprising performing a one-step target enrichment in a single reaction vessel or in a single reaction mixture, wherein said enrichment comprises the steps: (i) exposing the RNA to be sequenced in a single reaction vessel to a mixture comprising a reverse transcriptase, a DNA polymerase, and (a) one or more target-specific reverse primers, suitable for the preparation of a target specific cDNA; and (b) a forward target specific primer comprising a first forward adaptor sequence, and a reverse target specific primer comprising a first reverse adaptor sequence, and (c) a forward indexing primer comprising a first forward adaptor sequence, a forward indexing sequence and a second forward adaptor sequence; and a reverse indexing primer, comprising a first reverse adaptor sequence, a reverse indexing sequence, wherein the reverse indexing primer sequence is different from the forward indexing primer sequence, and a second reverse adaptor sequence; and desoxyribonucleoside triphosphates (dNTPs); and (ii) subjecting the reaction mixture of (i) to a series of temperature changes under conditions sufficient to yield a first strand cDNA copy of at least a portion of the RNA to be sequenced, preferably a gene sequence, and subsequently a target specific amplicon comprising starting from the 5'- to the 3'-end a second forward adaptor sequence, a forward index sequence, a first forward adaptor sequence, a forward target specific primer sequence, target sequence, a reverse target specific primer sequence, a first reverse adaptor sequence, a reverse index sequence and a second reverse adaptor sequence.

The following aspect referring to a method for preparing a DNA sample for a target specific next generation sequencing is not encompassed by the wording of the claims but is considered as useful for understanding the invention. In a further aspect the present invention relates to a method for preparing a DNA sample for a target specific next generation sequencing comprising performing a one-step target enrichment in a single reaction vessel or in a single reaction mixture, wherein said enrichment comprises the steps: (i) exposing the DNA to be sequenced in a single reaction vessel to a mixture comprising a DNA polymerase, and (a) one or more target-specific forward primers and one or more target specific reverse primers, suitable for the preparation of a target specific DNA; and (b) a forward target specific primer comprising a first forward adaptor sequence, and a reverse target specific primer comprising a first reverse adaptor sequence, and (c) a forward indexing primer comprising a first forward adaptor sequence, a forward indexing sequence and a second forward adaptor sequence; and a reverse indexing primer, comprising a first reverse adaptor sequence, a reverse indexing sequence, wherein the reverse indexing primer sequence is different from the forward indexing primer sequence, and a second reverse adaptor sequence; and desoxyribonucleoside triphosphates (dNTPs); and (ii) subjecting the reaction mixture of (i) to a series of temperature changes under conditions sufficient to yield a first strand cDNA copy of at least a portion of the RNA to be sequenced, preferably a gene sequence, and subsequently a target specific amplicon comprising starting from the 5'- to the 3'-end a second forward adaptor sequence, a forward index sequence, a first forward adaptor sequence, a forward target specific primer sequence, target sequence, a reverse target specific primer sequence, a first reverse adaptor sequence, a reverse index sequence and a second reverse adaptor sequence.

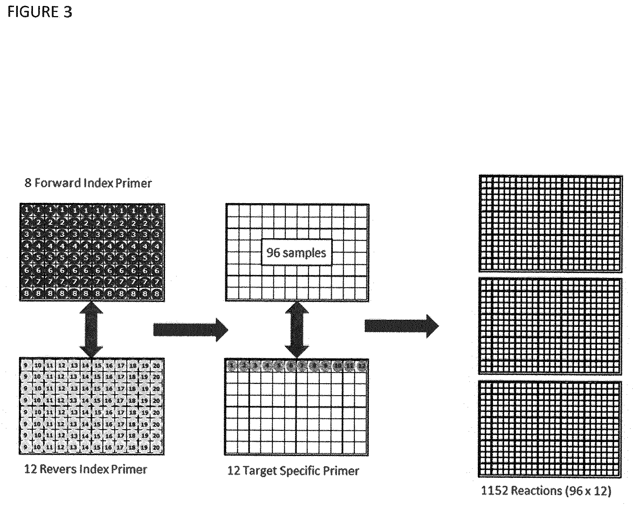

The currently claimed one-step target enrichment strategy for RNA and DNA samples (a DNA sample is not encompassed by the wording of the claims but is considered as useful for understanding the invention) can advantageously be used for an infinite number of applications, as target specific primers can be designed for any kind of genetic targets, similar to the conventional PCR. Further, since adaptor sequences in Target Specific Primer and Indexing Primer can be modified, the one-step target enrichment strategy of the present invention is also applicable for the use on different sequencing platforms. A further relevant advantage of the one-step target enrichment strategy of the present invention is the usage of the two separated primer sets (i.e. a Target Specific Primer and an Indexing Primer). The same Indexing Primer can thus be combined with different Target Specific Primers in different applications. There is hence no need to design and synthesize new Indexing Primers, if new target region must be sequenced. Furthermore, the use of dual indexing and forward Indexing Primers in combination with reverse Indexing Primers advantageously allows for the unambiguous assignment of the sequence reads to the samples. More importantly, only a relatively low number of Indexing Primers is required to analyze a high number of targets and samples. Accordingly, the costs for primer design and synthesis can be significantly reduced. Further details of important embodiments can also be derived from Figure 3. Another important advantage of the currently claimed technology is that the same combination of forward and reverse indexing primers can be used with multiple target specific primer pairs in only one vessel or well for a single patient.

In one set of embodiments, the method additionally comprises as first step the extraction of RNA or DNA from a sample obtained from a subject. In a preferred embodiment, the RNA or DNA is made accessible for further steps by cell lysis. DNA in this context is not encompassed by the wording of the claims but is considered as useful for understanding the invention.

In another embodiment, the sample is a liquid sample such as a cell culture, cell suspension, whole blood, blood plasma, urine, lavage, smear, mouth swab, throat swab, cerebrospinal fluid, saliva or stool sample, or a tissue or biopsy sample.

In a further embodiment, the target sequence is, or is derived from, a target gene or a part of the target gene, such as an exon or intron or part of both, a target intergenic region, or a genomic sequence or a part of it.

In yet another embodiment, the method additionally comprises a control amplification of one or more additional target sequences. In a preferred embodiment, the control amplification is performed with an independent subject-based target such as a mammalian house-keeping gene. It is particularly preferred to use an RNase gene.

In another preferred embodiment, the control amplification is an extraction control yielding information on the amount and/or quality of the sample.

In one embodiment, the method additionally comprises a step of sample registration, which is performed previous to the enrichment.

In a preferred embodiment, the sample registration comprises the unambiguous linking of the sample to a digital code or number.

In another preferred embodiment, the sample registration comprises a step of sample registration, which is performed previous to the enrichment.

In yet another preferred embodiment, the sample registration comprises the unambiguous linking of the sample to a digital code or number.

In a further embodiment, the sample registration is performed by a subject providing the sample.

In one embodiment, the sample registration is performed online, preferably with a mobile digital device such as a cellphone, tablet computer, smartwatch, or a laptop computer; or with any non-mobile computer system.

In a further embodiment, the method further comprises a purification of the amplicon as obtained in step (ii)

In another embodiment, the method further comprises a step of quantifying the amplicon.

In yet another embodiment, the method comprises a step of sequencing the amplicon as obtained in step (ii), preferably with a NGS system such as Illumina, Ion Torrent, Oxford Nanopore, or SMRT Sequencing.

In one embodiment, the method additionally comprises assembling sequence reads.

In another embodiment, the obtained sequence is aligned and/or compared with one or more reference sequences.

In yet another embodiment, the method additionally comprises a phylogenetic comparison of the obtained sequence(s) with one or more reference sequences.

In one embodiment, the obtained sequence is stored in, and optionally retrievable from, a computer system, a database, a public sequence repository, a cloud system, a hospital computer system, a doctor's association computer system, a local health organization database, a regional health organization database, a national health organization database, an international health organization database.

In a further embodiment, the preparation of sample for a target specific next generation sequencing is for the detection of a virus, microbe or a genotype of a higher eukaryote.

In another embodiment, the detection of a virus or microbe additionally includes an identification of said virus or microbe, preferably of sub-species, strain or variant or mutant version of said virus or microbe.

In yet another embodiment, the virus is a positive strand ssRNA virus, preferably belonging to the order of Nidovirales, Picornavirales or Tymovirales, or to the family of Coronaviridae, Picornaviridae, Caliciviridae, Flaviviridae or Togaviridae, wherein said virus is more preferably a rhinovirus, Norwalk-Virus, Echo-Virus or enterovirus, or a Coronavirus or belongs to the group of Coronaviruses, or belongs to the group of alpha or beta coronaviruses, such as human or Microchiroptera (bat) coronavirus, most preferably a SARS-CoV-2 virus.

In one embodiment, the detection of a genotype of a higher eukaryote comprises the identification of a blood group antigen or of a leukocyte antigen.

In a further embodiment, said blood group is a human blood group, preferably an ABO, MNS, Rhesus, Lutheran, Kell, Lewis, Duffy, Kidd, Diego, Yt, Scianna, Dombrock, Colton, Cromer, or Vel blood group.

In another embodiment, the said leukocyte antigen is a human leukocyte antigen, preferably HLA-A, HLA-B, HLA-C, HLA-E, HLA-F, HLA-G, HLA-DRA1, HLA-DRB1, HLA-DRB3/4/5, HLA-DQA1, HLA-DQB1, HLA-DPA1, or HLA-DPB1, or variants thereof.

In yet another embodiment, the method is performed computer-based, preferably automatically or semi-automatically.

The present invention provides in a further aspect, a kit for preparing an RNA sample for next generation sequencing in a one-step target enrichment comprising: a) a reverse transcriptase (RT); b) one or more target-specific reverse primers, suitable for the preparation of a target specific cDNA, c) a forward target specific primer comprising a first forward adaptor sequence, and a reverse target specific primer comprising a first reverse adaptor sequence; d) a forward indexing primer comprising a first forward adaptor sequence, a forward indexing sequence and a second forward adaptor sequence; and a reverse indexing primer, comprising a first reverse adaptor sequence, a reverse indexing sequence, wherein the reverse indexing sequence is different from the forward indexing sequence, and a second reverse adaptor sequence; e) desoxyribonucleoside triphosphates (dNTPs); and f) a DNA polymerase.

The following aspect referring to a kit for preparing a DNA sample for next generation sequencing in a one-step target enrichment is not encompassed by the wording of the claims but is considered as useful for understanding the invention. In yet another aspect the present invention relates to a kit for preparing a DNA sample for next generation sequencing in a one-step target enrichment comprising: a) one or more target-specific forward primers and one or more target specific reverse primers, suitable for the preparation of a target specific DNA, b) a forward target specific primer comprising a first forward adaptor sequence, and a reverse target specific primer comprising a first reverse adaptor sequence; c) a forward indexing primer comprising a first forward adaptor sequence, a forward indexing sequence and a second forward adaptor sequence; and a reverse indexing primer, comprising a first reverse adaptor sequence, a reverse indexing sequence, wherein the reverse indexing sequence is different from the forward indexing sequence, and a second reverse adaptor sequence; e; d) desoxyribonucleoside triphosphates (dNTPs); and e) a DNA polymerase.

In one embodiment, the adaptor sequence has length of about 8 to 45 nucleotides.

In another embodiment, the indexing primer sequence has length of about 4 to 20 nucleotides.

In yet another embodiment, the adaptor sequence is capable of binding to substrate, preferably a sequence chip or flow cell.

In a further embodiment, the target-specific primer or said target-specific primer pair is specific for a target sequence. Preferably said target sequence is a viral gene or a part of a viral genome, a leukocyte antigen-associated gene, or a blood group associated gene.

In another embodiment, the target sequence is a viral gene of a coronavirus, preferably a SARS-CoV-2 virus gene or genomic portion, or a part of it. More preferably the viral gene is a 5'UTR, 3'UTR, ORF1ab, Orf3a, Orf6, Orf7a, Orf7b, Orf8, Orf10, M gene region, E gene region, N gene region, or S gene region of SARS-CoV-2 virus.

In yet another embodiment the target sequence comprises one or more of the following nucleotide positions according to the nucleotide numbering of the reference genome of SARS-CoV-2 (reference genome with NCBI Reference Sequence No: NC_045512.2; SEQ ID NO: 63): 100, 733, 1264 , 2749, 3267, 3828, 5388, 5648, 6319, 6573, 6613, 6954, 7600, 7851, 10667, 11078, 11288-11296, 11824, 12964, 12778, 13860, 17259, 19602, 19656, 21614, 21621, 21638, 21765-21770, 21974, 21991-21993, 22132, 22812, 23012, 23063, 23271, 23525, 23604, 23709, 24506, 24642, 24914, 26149, 27853, 27972, 28048, 28111, 28167, 28253, 28262, 28280, 28512, 28628, 28877, 28975, 28977, 29722, 29754.

In one embodiment, the target sequence is a leukocyte antigen-associated gene selected from HLA-A, HLA-B, HLA-C, HLA-E, HLA-F, HLA-G, HLA-DRA1, HLA-DRB1, HLA-DRB3/4/5, HLA-DQA1, HLA-DQB1, HLA-DPA1, or HLA-DPB1.

In another embodiment, the target sequence is a blood group associated antigen associated with the ABO, MNS, Rhesus, Lutheran, Kell, Lewis, Duffy, Kidd, Diego, Yt, Scianna, Dombrock, Colton, Cromer, or Vel blood group antigens.

In yet another embodiment, the kit additionally comprises synthetic RNA-spike-ins.

In a further preferred embodiment of the method or kit as defined above said forward indexing primer is a primer selected from the group comprising primers of SEQ ID NO: 32 to SEQ ID NO: 39.

In another preferred embodiment of the method or kit as defined above, said reverse indexing primer is a primer selected from the group comprising primers of SEQ ID NO: 40 to SEQ ID NO: 51.

In another preferred embodiment of the method or kit as defined above, said enrichment comprises a multiplexing amplification.

In yet another preferred embodiment of the method or kit as defined above, 2, 3, 4, 5, 6, 7, 8, 9, 10 or more target sequences are simultaneously amplified. It is particularly preferred that 2 or 3 target sequences are simultaneously amplified.

In yet another preferred embodiment of the method defined above, the method allows for a qualitative detection of the target sequence and/or an organism or virus comprising said target sequence or a sequence being highly similar to the target sequence, preferably having a sequence identity of 97% or more.

In further preferred embodiment, method according to the invention comprises the detection of one or more of the following nucleotide exchanges or modifications at positions of the reference genome of SARS-CoV-2 (reference genome with NCBI Reference Sequence No: NC_045512.2; SEQ ID NO: 63): C100T, T733C, G1264T, C2749T, C3267T, C3828T, C5388A, A5648C, A6319G, C6573T, A6613T, T6954C, C7600T, C7851T, T10667G, T11078C, de!11288-11296, C11824T, A12964G, C12778T, C13860T, G17259T, C19602T, G19656T, C21614T, C21621A, C21638T, del21765-21770, G21974T, del21991-21993, G22132T, A22812C, G23012A, A23063T, C23271A, C23525T, C23604A, C23709T, T24506G, C24642T, G24914C, T26149C, A27853C, C27972T, G28048T, A28111G, G28167A, C28253T, insG28262GAACA, G28280C, C28512G, G28628T, AGTAGGG28877-28883TCTAAAC, G28975T, C28977T, C29722T, and C29754T.

A further aspect of the present invention relates to a use of the method or the kit as defined above for an enrichment for a rapid virus detection.

In yet another aspect the present invention relates to a use of the method or the kit as defined above for an enrichment for a rapid leukocyte antigen-associated gene identification.

In yet another aspect the present invention relates to a use of the method or the kit for an enrichment for a rapid blood group associated antigen identification.

In a further embodiment the method as defined above additionally comprises a step of sequence comparison with a reference sequence.

### BRIEF DESCRIPTION OF THE DRAWINGS

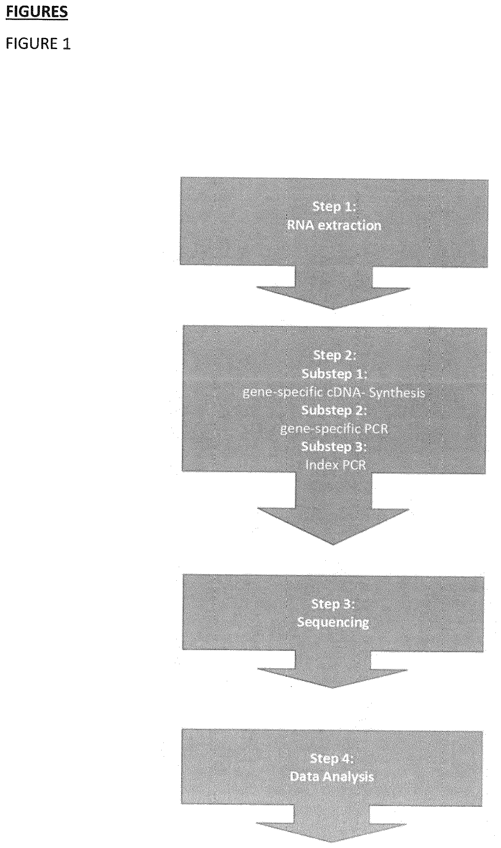

**Figure 1** provides an overview of the workflow according to the present invention.

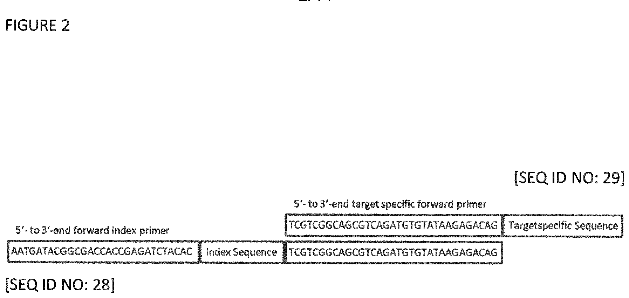

**Figure 2** shows characteristics of oligonucleotides which are used in an embodiment of the present invention.
**Figure 3** provides a schematic illustration of an exemplary combination of forward and reverse indexing primer sets with different target-specific primers.

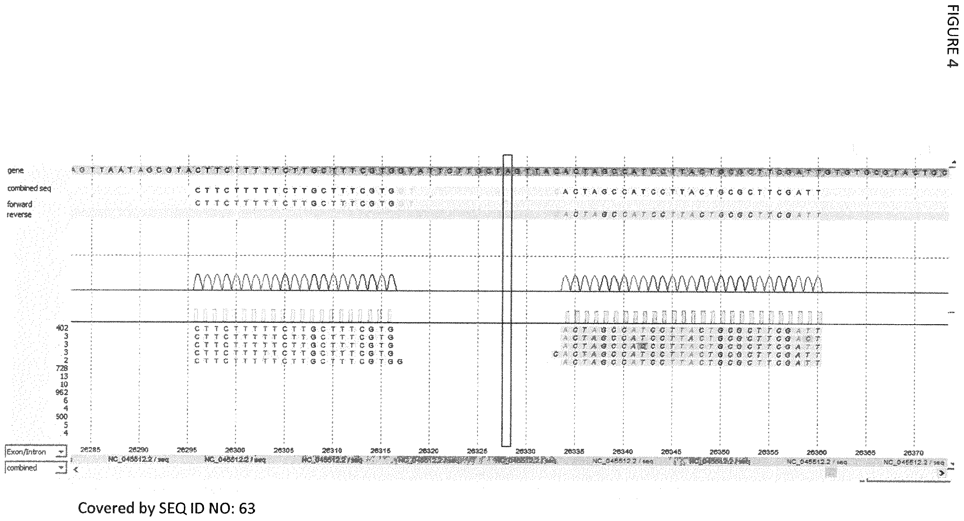

**Figure 4** shows a Wetlab feasibility MiSeq run for E_Sarbeco.

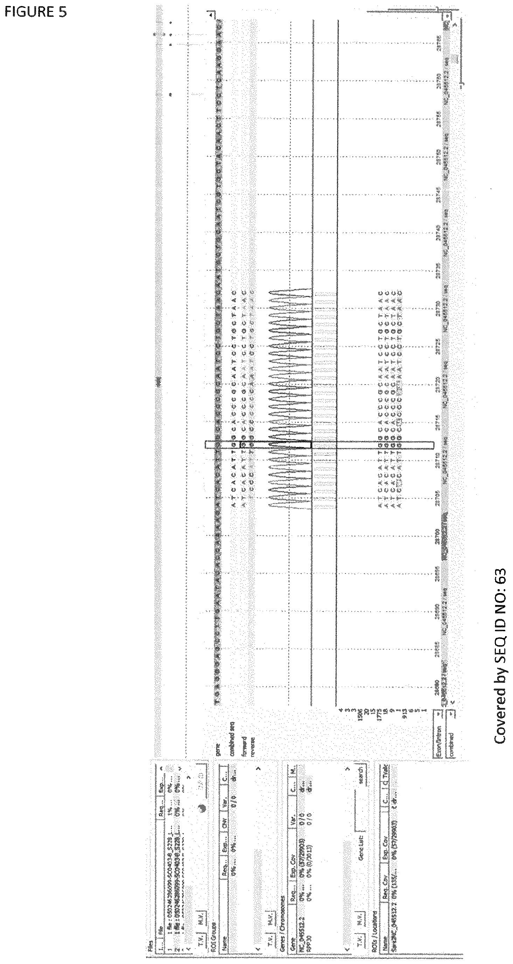

**Figure 5** shows a Wetlab feasibility MiSeq run for 2019_nCoV_N3.
**Figures 6** **and** **7** show sequencing results for Illumina sequencing experiments on Corona virus samples.
**Figure 8** shows the RavenC2 workflow. The workflow ensures a smooth transition between citizen and testing.
**Figure 9** depicts a sample and library preparation and sequencing embodiment according to the present invention. Samples are received and registered in the laboratory. Viral and human RNA targets are either amplified from extracted RNA or directly from lysate and indexing and adapter sequences are incorporated in a 1-step assay approach. Samples are combined into a pool and subjected to sequencing on an Illumina platform.
**Figure 10** depicts sample registry and result communication through the Raven App according to certain embodiments of the present invention. Citizens register via barcode on pre-labelled swab collection tubes anonymously in the Raven App. Once the result is ready a push notification informs the tested individual about an available result for download.
**Figure 11** shows results of a POP study. Individuals with confirmed CoV2 infections determined by PCR could be identified by RAVENC2 testing. All samples were run in duplicates with 2 different enrichment amplicons targeting 1 viral amplicon (darker grey / RP) or 5 viral amplicons (lighter grey / SC).

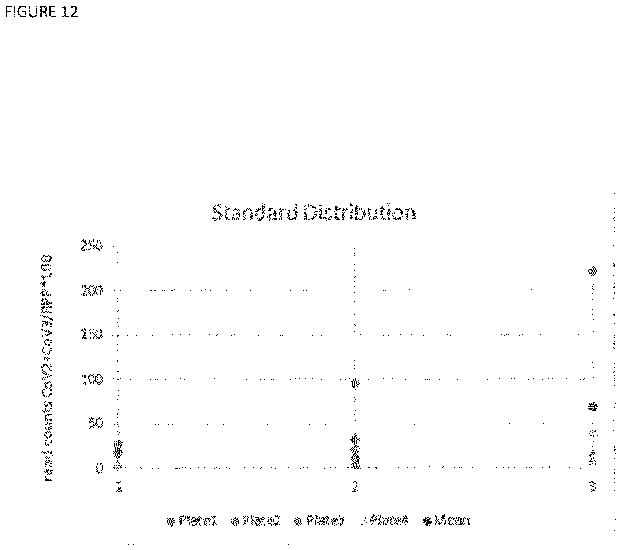

**Figure 12** shows standard distribution results. Column #1 (left hand side) depicts the distribution of the 50 copies standard, column #2 (middle) depicts the 100 copies standard and column #3 (right hand side) depicts the 200 copies standard. These standards were processed in the run to generate a baseline and are used to determine the cutoff and the sensitivity of the method.

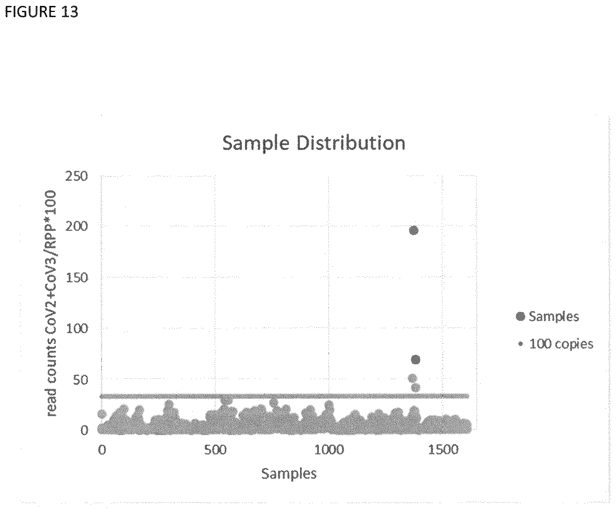

**Figure 13** shows the 100 copies threshold as thick horizontal line. The sample distribution of the individual samples is depicted as circles. Read counts of the 100 copy standard were chosen as cutoff. Samples that exceed the threshold are rated as covid positive samples.

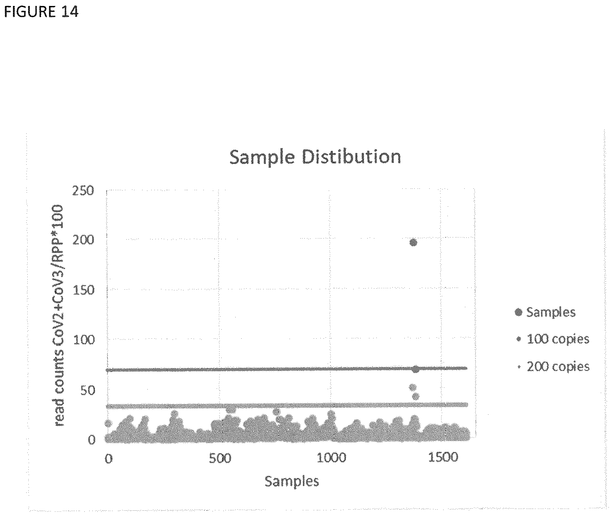

**Figure 14** shows the 100 copies threshold as lower thick horizontal line. The 200 copies threshold is shown as upper thick horizontal line. The sample distribution of the individual samples is depicted as circles. Read counts of the 100 copy standard and 200 copy standard were chosen as cutoff. Samples that exceed the threshold are rated as covid positive samples.

### DETAILED DESCRIPTION OF THE EMBODIMENTS

Although the present invention will be described with respect to particular embodiments, this description is not to be construed in a limiting sense.

Before describing in detail exemplary embodiments of the present invention, definitions important for understanding the present invention are given.

As used in this specification and in the appended claims, the singular forms of "a" and "an" also include the respective plurals unless the context clearly dictates otherwise.

In the context of the present invention, the terms "about" and "approximately" denote an interval of accuracy that a person skilled in the art will understand to still ensure the technical effect of the feature in question. The term typically indicates a deviation from the indicated numerical value of ±20 %, preferably ±15 %, more preferably ±10 %, and even more preferably ±5 %.

It is to be understood that the term "comprising" is not limiting. For the purposes of the present invention the term "consisting of" or "essentially consisting of" is considered to be a preferred embodiment of the term "comprising of". If hereinafter a group is defined to comprise at least a certain number of embodiments, this is meant to also encompass a group which preferably consists of these embodiments only.

Furthermore, the terms "(i)", "(ii)", "(iii)" or "(a)", "(b)", "(c)", "(d)", or "first", "second", "third" etc. and the like in the description or in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein. In case the terms relate to steps of a method or use, there is no time or time interval coherence between the steps, i.e. the steps may be carried out simultaneously or there may be time intervals of seconds, minutes, hours, days, weeks, etc. between such steps, unless otherwise indicated.

It is to be understood that this invention is not limited to the particular methodology, protocols, reagents, etc. described herein as these may vary. It is also to be understood that the terminology used herein is for the purpose of describing particular embodiments only, and is not intended to limit the scope of the present invention that will be limited only by the appended claims. Unless defined otherwise, all technical and scientific terms used herein have the same meanings as commonly understood by one of ordinary skill in the art.

As has been set out above, the present invention concerns in one aspect a method for preparing an RNA sample for a target specific next generation sequencing comprising performing a one-step target enrichment in a single reaction vessel or in a single reaction mixture, wherein said enrichment comprises the steps: (i) exposing the RNA to be sequenced in a single reaction vessel to a mixture comprising a reverse transcriptase, a DNA polymerase, and (a) one or more target-specific reverse primers, suitable for the preparation of a target specific cDNA; and (b) a forward target specific primer comprising a first forward adaptor sequence, and a reverse target specific primer comprising a first reverse adaptor sequence, and (c) a forward indexing primer comprising a first forward adaptor sequence, a forward indexing sequence and a second forward adaptor sequence; and a reverse indexing primer, comprising a first reverse adaptor sequence, a reverse indexing sequence, wherein the reverse indexing primer sequence is different from the forward indexing primer sequence, and a second reverse adaptor sequence; and desoxyribonucleoside triphosphates (dNTPs); and (ii) subjecting the reaction mixture of (i) to a series of temperature changes under conditions sufficient to yield a first strand cDNA copy of at least a portion of the RNA to be sequenced, preferably a gene sequence, and subsequently a target specific amplicon comprising starting from the 5'- to the 3'-end a second forward adaptor sequence, a forward index sequence, a first forward adaptor sequence, a forward target specific primer sequence, target sequence, a reverse target specific primer sequence, a first reverse adaptor sequence, a reverse index sequence and a second reverse adaptor sequence.

The term "in a single reaction vessel" as used herein means that due to the innovative combination of primers and ingredients, reverse transcription, target enrichment, index ligation, and sequencing adaptor ligation can be performed in a single place, e.g. vessel, without any additional intervention for purification or similar steps. Accordingly, all ingredient necessary for the preparation of a RNA sample, or in a further embodiment a DNA sample, can be mixed in said single vessel. This advantageously minimizes the time for the sample processing steps, minimizes the risk for sample mix-up or cross-contamination and can be controlled by temperature and cycle duration conditions during amplification steps.

The term "in single reaction mixture" as used herein means that due to the innovative combination of primers and ingredients, all steps of the method of the present invention can be performed in one mixture of ingredient without the necessity of adding further ingredient or inactivating ingredient after a certain step. This advantageously reduced the time and effort for performing the method and minimizes the risk for sample mix-up or cross-contamination.

For many applications, wherein whole genome sequencing is not required, it is often desirable to only sequence a specific subset of genes or regions of the genome. The term "target enrichment" refers to the amplification or multiple reproduction of such specific gene regions, usually by means of polymerase chain reaction amplification, or similar techniques.

Generally, the amplification processes are carried out on DNA target. For the present invention, it might is desirable to not only analyse DNA but also RNA, for example extracted viral RNA. For this purpose, synthesis of DNA from an RNA template via reverse transcription, also known as cDNA-synthesis, needs to be carried out prior to sequencing of the RNA sample. According, as described herein below in more detail, complementary DNA (cDNA) copies are created by using a reverse transcriptase (RT) or DNA polymerase having RT activity, which results in the production of single-stranded cDNA molecules.

The method thus envisages in a first step (i) the exposure of the RNA to be sequenced in a single reaction vessel to a mixture comprising a reverse transcriptase, a DNA polymerase, several different primers and a dNTPs. The term "exposure" a used herein means a contacting of at least one RNA molecule, preferably 1 to 1000 RNA molecules to an enzyme and dNTPs. The contacting may performed for any suitable time period, e.g. during the entire method, or until an amplicon has been obtained. The exposure may further be performed in a suitable buffer or reagent. The buffer may comprise KCI, MgCl₂, Tris HCL, DTT, Tween, DMSO, betain, BSA, urea, gelatine, spermidine, or any other suitable component in any suitable concentration known to the skilled person. The buffer may, in non-limiting examples, comprise TrisHCl e.g.in a concentration of 250 mM, KCI, e.g. in a concentration of 375 mM, MgClz, e.g. in a concentration of 15 mM and DTT, e.g. in a concentration of 0.1 M, preferably at a pH of 8.3. In addition, a suitable amount of dNTPs, e.g. dATP, dCTP, cGTP and cTTP has to be used, e.g. in a suitable concentration such as 10 mM. The buffer may further preferably comprise RNAse blocking compounds or RNase inhibitors such as RNaseZap, Superase, RNaseOUT, ribonuclease inhibitor, RNasin or the like.

The term "reverse transcriptase (RT)" as used herein refers to a class of polymerases characterized as RNA dependent DNA polymerases. All known RTs require a primer to synthesize a DNA transcript from an RNA template. The reverse transcriptase to be included in the mixture may be any suitable reverse transcriptase capable of producing cDNA known to the skilled person. Examples of such suitable reverse transcriptases include MMLV reverse transcriptase without RNase H activity, avian myeloblastosis virus (AMV) RT, or commercially available reverse transcriptases such as SuperScript, SuperScript II, Superscript III, Superscript IV, StrataScript, One step PrimeScript, Qiagen OneStep RT-PCR kit (Qiagen), Luna Universal Probe One-Step RT-qPCR Kits (NEB), TaqPath 1-Step RT-qPCR Master Mix (ThermoFisher) etc. The reverse transcriptase may further be a thermostable transcriptase such as Superscript IV or a non-thermostable transcriptase such as PrimeScript. This property of the transcriptase may have an influence on the reaction conditions, e.g. the reaction temperature for reverse transcription. It is preferred to use One Step PrimeScript (Takarabio), Qiagen OneStep RT-PCR kit (Qiagen), Luna Universal Probe One-Step RT-qPCR Kits (NEB), or TaqPath 1-Step RT-qPCR Master Mix (ThermoFisher).

The DNA polymerase to be included in the mixture may be any suitable DNA polymerase capable of producing amplicons known to the skilled person. Suitable examples include Taq-DNA polymerase, SuperFi DNA polymerase (Thermo Fisher), Q5 High Fidelity DNA polymerase (NEB), One Taq-DNA polymerase (NEB), Bst DNA polymerase (NEB), Pfu DNA polymerase (Promega), GoTaq polymerase (Promega), Taq DNA Polymerase (Thermofisher), Platinum II Taq Hot-Start DNA Polymerase (ThermoFisher), and FastStart Taq DNA Polymerase (Roche).

The mixture further comprises an innovative selection of primers. The term "primer" as used herein refers to a short single-stranded nucleic acid which serves as a starting point for replicating enzymes, such as DNA polymerase or RT, during DNA or cDNA synthesis. The selection of primers according to the method of the present invention comprises as a first group of primers (a) one or more target-specific reverse primers, suitable for the preparation of a target specific cDNA. The term "reverse primer" as used herein relates to a primer that is complementary to the RNA strand. It accordingly allows for the provision of a DNA copy (cDNA) of the RNA strand after synthesis by a reverse transcriptase. The target-specificity or complementarity may be complete or 99%, 98%, 97% or lower. For example, it may allow for one or two mismatches. It is preferred that the complementarity is complete. The target specific reverse primer for cDNA preparation may have any suitable length, e.g. 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39 or 40 or more nucleotides. It is preferred that the target specific reverse primer can distinguish between different targets, e.g. different virus strains, as well one or more internal controls. In one mixture one or more target-specific reverse primers may be present, e.g. 2, 3, 4, 5, 6, 7, 8, 9, 10 or more. This group of primers may be used for a multiplexing of target sequences, thus yielding a group of cDNA molecules which can subsequently be further processed or enriched. If more than one target-specific reverse primer is used, at least one primer may bind to a sequence of a target entity, whereas the other primer may bind to a control sequence, e.g. a sequence from the host or any other suitable sequence. This step thus allows for a multiplexing amplification during the enrichment procedure.

In certain embodiments 2, 3, 4, 5, 6, 7, 8, 9, 10 or more target sequences are simultaneously amplified. The mixture may accordingly comprise a corresponding number of target specific primers. It is particularly preferred that 2 or 3 target sequences are simultaneously amplified.

The term "target sequence" as used in the context of the present invention relates to any sequence of interest. It may, for example, be a sequence which is derived from a gene, i.e. a specific target gene, or a part of said gene, such as an exon or intron or part of both, an intergenic region, a transcript (RNA), a genomic sequence or a part of it, a splice site, a functional domain, a regulatory sequence such as a promoter sequence, a sector with known SNPs, a mutational hotspot, a sequence associated with a disease, with a resistance to a drug, with an immunological deficit etc. The target sequence may be of RNA or DNA origin. The target sequence being of DNA origin is not encompassed by the wording of the claims but is considered as useful for understanding the invention.

In preferred embodiments the target-specific primer (in particular in case of an RNA sample where cDNA is produced) or the target-specific primer pair (in particular in case of a DNA sample where a dsDNA amplicon is produced) is specific for a target sequence. The target sequence may have any suitable length, e.g. 100 nt, 200 nt, 300 nt, 400 nt, 500 nt, 1000 nt, 2000 nt, 3000 nt, 4000 nt, 5000 nt, 10 000 nt 15 000 nt or more or any value in between the mentioned values. The target sequence is, in particularly preferred embodiments, a viral gene or a part of a viral genome. It may also be a leukocyte antigen-associated gene, or a blood group antigen associated gene.

The viral gene may, in certain embodiments, be a viral gene of a coronavirus, in particular of SARS-CoV-2 virus gene. It may also be a genomic portion, or it may a part or sub-section of a gene, e.g. a region spanning any 100, 150, 200, 300, 400, 500 nt etc. The target sequence may, for example, comprise, essentially consist of, or consist of the 5'UTR, 3'UTR, ORF1ab, Orf3a, Orf6, Orf7a, Orf7b, Orf8, Orf10, M gene region, E gene region, N gene region, or S gene region of SARS-CoV-2, or any 100, 150, 200, 300 nt etc. fragment within these entities, or spanning two or more of these entities.

In further very specific embodiments, the target sequence comprises one or more positions of genomic mutation in the genome of SARS-CoV-2. This may also include not yet know mutations, which are to be detected in the future. These mutations may, in typical situations, lead to synonymous or nonsynonymous amino acid substitutions, or deletions or other changes in the genome. The mutations may, in preferred embodiments, be associated with phenotypical changes of virus biology, e.g. lead to a changed binding or infection behavior, a changed mortality, a changed susceptibility of the virus to vaccination induced immune reactions etc. In several cases the mutation may have an influence on the structure and/or conformation of the SARS-CoV-2's spike or S protein. In more specific embodiments the mutation may have an influence on the binding interface of the spike protein and its cognate receptor, e.g. ACE2. The present invention accordingly envisages one or more of the following nucleotide positions according to the nucleotide numbering of the reference genome of SARS-CoV-2 (reference genome with NCBI Reference Sequence No: NC_045512.2; SEQ ID NO: 63), which are to comprised in target sequence, .e.g. of any suitable size such as 100, 150, 200, 300, 400, 500 nt: Position (according to the numbering scheme of NC_045512.2) 100, 733, 1264, 2749, 3267, 3828, 5388, 5648, 6319, 6573, 6613, 6954, 7600, 7851, 10667, 11078, 11288-11296, 11824, 12964, 12778, 13860, 17259, 19602, 19656, 21614, 21621, 21638, 21765-21770, 21974, 21991-21993, 22132, 22812, 23012, 23063, 23271, 23525, 23604, 23709, 24506, 24642, 24914, 26149, 27853, 27972, 28048, 28111, 28167, 28253, 28262, 28280, 28512, 28628, 28877, 28975, 28977, 29722, and/or 29754.

In further specific embodiments the method according to the present invention comprises the detection of one or more of the following nucleotide exchanges or modifications at positions of the reference genome of SARS-CoV-2 (reference genome with NCBI Reference Sequence No: NC_045512.2; SEQ ID NO: 63): C100T, T733C, G1264T, C2749T, C3267T, C3828T, C5388A, A5648C, A6319G, C6573T, A6613T, T6954C, C7600T, C7851T, T10667G, T11078C, del11288-11296, C11824T, A12964G, C12778T, C13860T, G17259T, C19602T, G19656T, C21614T, C21621A, C21638T, del21765-21770, G21974T, del21991-21993, G22132T, A22812C, G23012A, A23063T, C23271A, C23525T, C23604A, C23709T, T24506G, C24642T, G24914C, T26149C, A27853C, C27972T, G28048T, A28111G, G28167A, C28253T, insG28262GAACA, G28280C, C28512G, G28628T, AG-TAGGG28877-28883TCTAAAC, G28975T, C28977T, C29722T, and C29754T. These nucleotide exchanges or modifications relate to a difference vs. a wild-type sequence enshrined in NC_045512.2. In several instances the presence of a nucleotide exchange indicated a mutated virus. In such cases a specific alert may be started. Furthermore, information may be aggregated into statistics, sequence information may be provided to local, regional, national or international health organization or decision makers.

In further embodiments the target sequence is a leukocyte antigen-associated gene. Envisaged examples include HLA-A, HLA-B, HLA-C, HLA-E, HLA-F, HLA-G, HLA-DRA1, HLA-DRB1, HLA-DRB3/4/5, HLA-DQA1, HLA-DQB1, HLA-DPA1, or HLA-DPB1, or variants thereof. Corresponding genetic information may be known to the skilled person or can be derived from suitable literature or internet sources. For example, information may be derived from the PD-IMGT/HLA Database (https://www.ebi.ac.uk/ipd/imgt/ hla/; last visited on April 20, 2021); see also Robinson et al., Nucleic Acids Research, 2020), 48:D 948-55.

In a further specific embodiment the target sequence is a blood group associated antigen. Envisaged blood group antigens comprise ABO, MNS, Rhesus, Lutheran, Kell, Lewis, Duffy, Kidd, Diego, Yt, Scianna, Dombrock, Colton, Cromer, and Vel blood group. Corresponding genetic information may be known to the skilled person or can be derived from suitable literature or internet sources. For example, information may be derived from the BGMUT database or the dbRBC (database Red Blood Cells) resource of NCBI at the NIH. (see also Patnaik et al., Nucleic Acids Res., 2012, 40, D1023-D1029).

The innovative selection of primers in the method of the invention further comprises as a second group of primers (b) a forward target specific primer comprising a first forward adaptor sequence, and a reverse target specific primer comprising a first reverse adaptor sequence. The group of primers is envisaged for an enrichment step, which specifically enriches a target sequence. The term "forward primer" as used herein relates to a primer that is complementary to the sequence of the reverse strand. Accordingly, the forward primer allows for providing copies of the template strand, e.g. of the cDNA and subsequently derived DNA molecules. The forward target specific primer advantageously comprises two sections or portions, a target specific portion and an adaptor portion. The target specific portion is complementary to a target sequence on the cDNA. The target specific forward primer portion may have any suitable length, e.g. 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39 or 40 or more nucleotides. It may be fully complementary or allow for one or two mismatches. The adaptor portion is located at the 5' end of the primer. It corresponds to a sequencing primer sequence and, at the same time, may be used as adaptor for binding to primers of group (c), i.e. to indexing primers. The adaptor portion may have any suitable length, e.g. 15, 16, 17, 18, 19, 20, 21, 22, 23, 24, 25, 26, 27, 28, 29, 30, 31, 32, 33, 34, 35, 36, 37, 38, 39 or 40 or more nucleotides. It is preferred that the adaptor sequence has a length of 8 to 45 nucleotides. The reverse target specific primer comprising a first reverse adaptor sequence as used herein is constructed in a similar way as the forward primer. Accordingly, the reverse primer allows for providing copies of the complementary strand, e.g. a DNA molecule derived from cDNA. The reverse target specific primer advantageously comprises two sections or portions, a target specific portion and an adaptor portion. The target specific portion is complementary to a target sequence on the cDNA-derived complementary DNA strand. The target specific reverse primer portion may have any suitable length, e.g. 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39 or 40 or more nucleotides. It may be fully complementary or allow for one or two mismatches. The adaptor portion is located at the 5' end of the primer. It corresponds to a sequencing primer sequence and, at the same time, may be used as adaptor for binding to primers of group (c), i.e. to indexing primers. The adaptor portion may have any suitable length, e.g. 15, 16, 17, 18, 19, 20, 21, 22, 23, 24, 25, 26, 27, 28, 29, 30, 31, 32, 33, 34, 35, 36, 37, 38, 39 or 40 or more nucleotides. It is preferred that the adaptor sequence has a length of 8 to 45 nucleotides. This group of primers may be used for sequence enrichment. By using target specific forward and reverse primers which bind to the cDNA sequence (and its complement) which can be obtained with the primers of group and which is unique for every target of interest, it is possible to distinguish between different targets and even allows for including an internal control. Accordingly, a huge number of double stranded DNA molecules, which are fully or at least highly complementary to the cDNA template sequence, can be synthesized by with the help of a DNA polymerase, e.g. via PCR steps.

The innovative selection of primers in the method of the invention further comprises as a third group of primers (c) a forward indexing primer comprising a first forward adaptor sequence, a forward indexing sequence and a second forward adaptor sequence and a reverse indexing primer, comprising a first reverse adaptor sequence, a reverse indexing sequence, wherein the reverse indexing primer sequence is different from the forward indexing primer sequence, and a second reverse adaptor sequence. The forward primer of said group (c) accordingly comprises three sections of which the most 3' oriented section is a first forward adaptor sequence, which may be identical to the first forward adaptor sequence of the forward primers of group (b) and thus complementary to a corresponding portion of the enriched molecules. Also envisaged are sequences which are partially identical, e.g. 80% or more identical. This adaptor sequence is designed to be bound by a sequencing primer for a subsequent sequencing activity. The second section, more 5' oriented, is an indexing sequence. The term "indexing sequence" as used herein relates to a sequence which is artificially included in a polynucleotide and which serves for identification purposes after a characterization step, e.g. after sequencing. The indexing sequence may, thus, inform the user which of several samples is being characterized, e.g. sequenced. An indexing section accordingly comprises a unique sequence which is provided only once, i.e. for one type of molecule/polynucleotide, e.g. within one sample. The indexing sequence is preferably different from known naturally occurring sequence motifs. In other embodiments, it is preferably long enough to avoid mix-ups with naturally occurring sequences or different indexing sequences. According to preferred embodiments, the indexing sequence has a length of at least 4 to about 25 or more nucleotides, preferably a length of about 4 to 20 nucleotides. Further details would be known to the skilled person, or can be derived from suitable literature sources such as Kozarewa et al., 2011, Methods Mol. Biol.733, 279-298. The third section, at the 5' terminus, is a further, second adaptor sequence. This second adaptor sequence is capable of interacting with a substrate or device, e.g. a flow cell, to facilitate sequencing. The second adaptor sequence may have any any suitable length, e.g. 15, 16, 17, 18, 19, 20, 21, 22, 23, 24, 25, 26, 27, 28, 29, 30, 31, 32, 33, 34, 35, 36, 37, 38, 39 or 40 or more nucleotides. It is preferred that said adaptor sequence has a length of 8 to 45 nucleotides. The sequence is preferably complementary to a fishing sequence at a substrate or device, e.g. at the surface of a sequence chip or flow cell such as an Illumina sequencing flow cell. It is particularly preferred that the forward indexing primer is a primer selected from the group comprising primers of SEQ ID NO: 32 to SEQ ID NO: 39

The reverse indexing primer of said group (c) has an identical arrangement of three sections as described above for the forward indexing primer. Accordingly, it comprises a first adaptor sequence, an indexing sequence and a second adaptor sequence. Importantly, it is advantageously envisaged that the indexing sequence of the reverse primer of said group (c) is not identical to the indexing sequence of the forward primer. This allows for a distinction of both strand upon sequencing and thus provides two differentiable and separately identifiable strands of a molecule. It is particularly preferred that the reverse indexing primer is a primer selected from the group comprising primers of SEQ ID NO: 40 to SEQ ID NO: 51.

Accordingly, the primer of group (c) allows for a preparation of the molecule for a subsequent sequencing step. This preparation includes a dual indexing. Thus, to every sample, a unique combination of forward and reverse index sequences is added which advantageously allows a pooling of high number of samples and their simultaneous sequencing. Accordingly, the number of samples, sequenced in parallel in a single run, is not limited to the possibilities to design sample specific indexes, but may rather depend on the sequencing platform and potentially data output.

The method according to the invention comprises, upon contacting the RNA molecule with the primer groups as described above, (ii) subjecting the reaction mixture of (i) to a series of temperature changes. These temperature changes are designed to successionally make use of primer groups (a), (b) and (c).

The first set of conditions is designed to allow for the production of a cDNA molecule. Conditions for this step may vary according to the primer length and sequence and the reverse transcriptase used. For example, for thermostable reverse transcriptase, e.g. Superscript IV, a suitable annealing temperature for the primer and a reaction temperature of about 50°C may be used. For non-thermostable reverse transcriptases a lower temperature, e.g. of 25°C may be used, preferably with e.g. OneStep PrimeScript. It is preferred to use a low temperature of about 25°C for the reverse transcription. The reverse transcription step may be performed for any suitable length of time, e.g. for about 3 to 15 min, preferably for about 5 minutes.

The second set of conditions is designed to allow for the enrichment of target sequences from the cDNA molecule with the primers of group (b) and the preparation of molecules for sequencing according with the primers of group (c). Conditions for this step may vary according to the primer length, the target sequence and the DNA polymerase used. Typically, a denaturation step, a primer annealing step and an extension or polymerisation step is used. These steps are repeated for several times, e.g. for 15 to 35 times. For example, the denaturation may be performed at temperatures of about 95°C. The annealing step may be performed in the range of about 50 to 60°C. The extension may be performed, depending on the DNA polymerase, at a temperature of about 55 to 72°C. Time periods may be adapted to the target sequence length or the number of cycles. Typically, denaturation periods are about 15 to 30 sec and annealing periods are about 15 to 30 sec. The extension period may vary considerably. Typically, about 1 min of extension time may be calculated for about 1000 base pairs to be produced.

After having finished the enrichment and preparation steps, a target specific amplicon is obtained. This amplicon comprises the following segments from 5' to 3' end: (1) a second forward adaptor sequence which is suitable to binding to a substrate, (2) a forward indexing sequence, (3) a first forward adaptor sequence which is complementary to a sequencing primer, (4) a forward target specific primer sequence, (5) a target sequence of variable length according to the selected target and the selected primers, (6) a reverse target specific primer sequence, (7) a first reverse adaptor sequence, which is complementary to a sequencing primer, (8) a reverse index sequence, (9) a second reverse adaptor sequence, which is suitable to binding to a substrate. The double stranded amplicon can thus be sequenced and identified according to the indexing sequence on both strands in parallel.

Advantageously, this resulting product can be obtained in single vessel and thereby allows for a very efficient high-throughput and less time consuming overall sequencing approach. The approach thus minimizes the time for the sample processing steps, minimizes the risk for sample mix-up or cross-contamination and the process can be controlled by specific parameter such as temperature and cycle duration conditions during amplification steps. As further advantageous feature of the invention, PCR products can, in certain embodiments, be pooled after the target enrichment since every single sample is combined with a sample specific index. Consequently, the number of vessels can by reduced by pooling of samples. Further steps such as PCR product purification and normalization can be performed with a significantly reduced number of vessels, which saves time and reagents. Furthermore, subsequent sequencing steps may directly be performed with the obtained product. The present invention envisages a pooling of 10, 20, 20, 40, 50, 60, 70, 80, 90, 100, 200, 300, 400, 500, 1000, 2000, 2500, 3000 or more or any value in between the mentioned values of different enrichment products (e.g. derived from a corresponding number of single vessels). In specific embodiments the maximum number of pooled different enrichment products may be limited by the number of available different indexing sequences. The maximum number of pooled different enrichment products can accordingly be adjusted to the choice and amount of different indexing sequences, or to any other suitable parameter.

The following aspect referring to a method for preparing a DNA sample for a target specific next generation sequencing is not encompassed by the wording of the claims but is considered as useful for understanding the invention. In an alternative aspect the present invention relates to a method for preparing a DNA sample for a target specific next generation sequencing comprising performing a one-step target enrichment in a single reaction vessel or in a single reaction mixture, wherein said enrichment comprises the steps: (i) exposing the DNA to be sequenced in a single reaction vessel to a mixture comprising a DNA polymerase, and (a) one or more target-specific forward primers and one or more target specific reverse primers, suitable for the preparation of a target specific DNA; and (b) a forward target specific primer comprising a first forward adaptor sequence, and a reverse target specific primer comprising a first reverse adaptor sequence, and (c) a forward indexing primer comprising a first forward adaptor sequence, a forward indexing sequence and a second forward adaptor sequence; and a reverse indexing primer, comprising a first reverse adaptor sequence, a reverse indexing sequence, wherein the reverse indexing sequence is different from the forward indexing sequence, and a second reverse adaptor sequence and (ii) subjecting the reaction mixture of (i) to a series of temperature changes under conditions sufficient to yield a target specific amplicon comprising starting from the 5'- to the 3'-end a second forward adaptor sequence, a forward indexing sequence, a first forward adaptor sequence, a forward target specific primer sequence, target sequence, a reverse target specific primer sequence, a first reverse adaptor sequence, reverse indexing sequence and a second reverse adaptor sequence.

The method for preparing a DNA sample for a target specific next generation sequencing largely corresponds to the method for preparing an RNA sample for target specific next generation sequencing. The above explained features and details thus apply also to the method for preparing a DNA sample, with the exception that the innovative group of primers (a) is designed for the amplification of a DNA molecule. Accordingly, the mixture does not comprise a reverse transcriptase, but only a DNA polymerase as mentioned above. Accordingly, the primers of group (a) may comprise one or more forward and reverse primers for the target sequence, thus allowing an amplification of both strands of the target DNA at the same time. The initial amplification may be followed by a target enrichment and sample indexing step which fully corresponds to the RNA based method mentioned above. As to the elements of (ii), i.e. subjecting the reaction mixture of (i) the method relates to a series of temperature changes under conditions sufficient to yield a target specific amplicon. These steps differ from the steps mentioned in the context of the RNA based method by the omission of a first reverse transcription step. Accordingly, a series of temperature changes including denaturation, annealing and extension as explained above may be used.

Accordingly, after having finished the enrichment and preparation steps for the DNA sample, a target specific amplicon is obtained. This amplicon comprises the following segments from 5' to 3' end: (1) a second forward adaptor sequence which is suitable to binding to a substrate, (2) a forward indexing sequence, (3) a first forward adaptor sequence which is complementary to a sequencing primer, (4) a forward target specific primer sequence, (5) a target sequence of variable length according to the selected target and the selected primers, (6) a reverse target specific primer sequence, (7) a first reverse adaptor sequence, which is complementary to a sequencing primer, (8) a reverse index sequence, (9) a second reverse adaptor sequence, which is suitable to binding to a substrate. The double stranded amplicon can thus be sequenced and identified according to the indexing sequence on both strands in parallel.

The method as defined above, additionally comprising as first step the extraction of RNA from a sample obtained from a subject, preferably by sample lysis, or, alternatively, the extraction of DNA from a sample obtained from a subject. DNA extraction in this context is not encompassed by the wording of the claims but is considered as useful for understanding the invention.

For the extraction of RNA maintaining RNA integrity is critical and requires special precautions during extraction, processing, storage, and experimental use. It is accordingly preferred to perform the method with nuclease-free labware and reagents. To isolate and purify RNA, a variety of strategies are available depending on the type of source materials. It is, in particular, envisaged to stabilize RNA molecules, to inhibit RNases, and to maximize yield. Envisaged purification methods typically remove endogenous compounds, such as complex polysaccharides that may interfere with enzyme activity; and common inhibitors of reverse transcriptases, such as salts, metal ions, ethanol, and phenol. Typically, the extraction is performed with a suitable cell lysis buffer, e.g. a commercially available cell lysis buffer such as RNeasy (Qiagen) or RLA (Promega). Typically, the cell lysis buffer for RNA extraction is highly denaturing and is usually composed of guanidine isothiocyanate. RNase inhibitors are usually present in the lysis buffer, since RNases can be resistant to denaturation and remain active. Also envisaged is the use of paramagnetic beads, e.g. SPRI beads.

For extraction of DNA (DNA extraction in this context is not encompassed by the wording of the claims but is considered as useful for understanding the invention) a similar approach may be used. However, the employment of RNA stabilizers and RNase inhibitors is not necessary. Typically, cells in a sample are separated from each other, often by a physical means such as grinding or vortexing, and put into a solution containing salt. The positively charged sodium ions in the salt help protect the negatively charged phosphate groups that run along the backbone of the DNA. Subsequently, as much of the cellular debris as possible needs to be removed. This is typically done by using a protease to degrade DNA-associated proteins and other cellular proteins. Alternatively, some of the cellular debris can be removed by filtering the sample. Finally, the DNA is precipitated by adding isopropanol to the mixture. Further, magnetic beads-based methods or column-based methods can be used. For cell lysis typically a lysis buffer which commonly contains SDS is used. Also envisaged are commercial extraction kits such as DNAzol (ThermoFisher), PureLink (ThermoFisher), Monarch (NEB) or Wizard (Promega).

In embodiments of the invention the sample may be a liquid sample.

The term "liquid sample" refers to a liquid material obtained via suitable methods from one or more biological organisms or comprising one or more biological organisms, or processed after having been obtained. The liquid sample may further be material obtained from contexts or environments in which biological organisms are present, or processed variants thereof. Typically, the liquid sample is an aqueous sample. In preferred embodiments, it may comprise a bio-organic fluid obtained from the body of a mammal that is taken for analysis, testing, quality control, or investigation purposes. In a preferred embodiment, said liquid sample may be a cell culture sample, a cell suspension, whole blood, blood plasma, urine, lavage, smear, mouth swab, throat swab, cerebrospinal fluid, saliva or stool sample, or a tissue or biopsy sample. It may further be a blood components or banked blood sample, a bile, saliva, nasal fluid, ear fluid sweat, sputum, semen, breast fluid, milk, colostrum, pleural fluid, ascites, cerebrospinal fluid, amniotic fluid or bronchoalveolar lavage fluid, gastric fluid, aqueous humor, vitreous humor, gastrointestinal fluid, exudate, transudate, pleural fluid, pericardial fluid, upper airway fluid, peritoneal fluid, or a liquid stool sample. Also envisaged are a fluid harvested from a site of an immune response, or fluid harvested from a pooled collection site. Furthermore, the liquid sample may contain a tissue extract derived from body tissues, e.g. tissues obtained via biopsy or resections, preferably from a eukaryotic organism, more preferably from a mammalian organism, even more preferably from a human being. The biopsy material may be derived, for example, from all suitable organs, e.g. the lung, the muscle, brain, liver, skin, pancreas, stomach, heart, stomach, intestine etc., a nucleated cell sample, a fluid associated with a mucosal surface, or skin. In order to be extracted, the biopsy material is typically homogenized and/or resuspended in a suitable buffer solution as known to the skilled person. Such samples may, in specific embodiments, be pre-processed e.g. by enrichment steps and/or dilution steps etc. Typically, the sample is processed by lysis and subsequent RNA or DNA extraction as outlined above. The sample may, in further embodiments, be a solid sample. A solid sample, e.g. a solid tissue sample or a solid cell accumulation may subsequently be diluted in a suitable buffer for further processing steps. In addition, any suitable sample derived from the environment, food sources, organic or biological sources (e.g. animals, in particular mammals, plants etc.) may be used, e.g. after processing. It is preferred that the sample is a swab sample, e.g. taken from nose and/or mouth and/or throat zones of the body. Also preferred are blood and processed blood samples, tissue sample or cell culture sample.

For control purposes one or more additional sequences of interest may be analysed. These additional sequences are selected among genes or transcripts which are typically expected to a be present in a sample. Such sequences are further expected to be present in a wide variety o tissues, cell types and samples and to show no or only minimal changes in expression levels between individual samples and experimental conditions. For RNA detection typically expressed genes are used. A suitable example is a mammalian house-keeping gene such as RNase. Also envisaged is the use of Glyceraldehyde 3-phosphate dehydrogenase (GAPDH). For DNA detection, although not encompassed by the claims, any genomic sequence may be used.

The use of additional sequences as mentioned above is advantageously used for different purposes. It can be used as extraction control yielding information on the amount and/or quality of the sample. It may further be used as process control to show whether amplification steps have properly worked. In typical embodiments the control readout is the generation of a sequence read for the envisaged control sequence.

In preferred embodiments of the invention a sample obtained from a subject is registered. The term "sample registration" as used herein means that the sample is unambiguously connected to a subject, as well as to a date and optionally a place, a time, a subject's birth date, email address, telephone number, street address, the subject's responsible general practitioner, the subject's emergency contact, a subject's health insurance information etc. The sample registration is preferably performed previous to the enrichment. The registration of a subject's data may be rendered anonymous. For example, the data may be connected to a number and be stored in a separate place or system. It is particularly preferred to register samples with digital code or number. This digital code or number is preferably chosen to be unambiguous, i.e. should have a suitable length or complexity.

The registration may performed by the subject during the sample taking process. For example, the subject is asked to provide all necessary information. It is preferred to collect the information electronically, e.g. via a mobile digital device such as a cell phone, tablet computer, smartwatch, or a laptop computer, preferably an app working on the device or a suitable interface, e.g. a web interface. Alternatively, the information may also be collected with any non-mobile computer system, e.g. on paper or as audio data.

In further embodiments, the method additionally comprises a step of purification of the amplicon as obtained in step (ii). This step is envisaged in order to avoid quality and efficiency problems during a subsequent sequencing step. The purification of the DNA amplicons may be performed according to any suitable method known to the skilled person. For example, obtained amplifications may be purified with a column-based technique or magnetic/streptavidin bead based methods. For example, spin columns may be used to quickly and efficiently purify PCR products from enzymes, dNTPs, salts and primers. The DNA is typically eluted from the spin column with a buffer and can subsequently be used for sequencing steps. An envisaged commercial example is the QIAquick PCR purification kit (Qiagen), GenCatch (Epoch).

The method as described herein additionally comprises a step of quantifying the amplicon. This step may, for example, be performed spectrophotometrically, e.g. by measuring intrinsic absorptivity properties of nucleic acids (DNA or RNA), or with fluor-ophore based methods, a fragment analyzer or by real-time PCR. When an absorption spectrum is measured, nucleic acids absorb light with a characteristic peak at 260 nm. A corresponding signal is typically measured by spectrophotometers or spectrometers. Alternatively, a quantification measurement may be performed via electrophoresis of an amplicon sample and a subsequent staining, e.g. with ethidium bromide.

In further specific embodiments the method allows for a qualitative detection of a target sequence. The method thus provides a diagnostically relevant answer to the question whether a sequence is present or not. The qualitative detection may, for example, be based on a predefined cut-off amount of detected molecules in a specific volume and/or after a specific number of PCR cycles. Should the detected number be below said threshold, a negative answer is given, vice versa if the threshold is surpassed. The exact threshold value may depend on the equipment and reagents used. It my further be calibrated with specific control and calibration solutions, e.g. comprising a predefined amount of target sequence. In a corresponding embodiment, the qualitative detection of a target sequence may also provide a diagnostically relevant answer to the question whether an organism or virus comprising said target sequence is or was, or parts of it are or were, present or not in the sample. In further embodiments, the method allows for a qualitative detection of a sequence having a sequence identity of 97% or more with a certain, e.g. predefined target sequence.

As used herein, the term "next generation sequencing" or "deep sequencing are related terms that describe a DNA sequencing technology which allows multi-million DNA samples to be sequenced simultaneously. This next generation sequencing approach is typically a massively parallel sequencing approach which may include any suitable sequencing method that determines the nucleotide sequence of the amplicon according to the present invention in a highly parallel fashion. For example, more than 10⁸ molecules may be sequenced simultaneously. The sequencing may be performed according to any suitable massive parallel approach. Typical platforms include Roche 454, GS FLX Titanium, Illumina, Life Technologies, Ion Torrent, Oxford Nanopore Technolgies, Solexa, Solid or Helicos Biosciences Heliscope systems, MGI Tech or SMRT Sequencing. Preferred is the Illumina platform. The sequencing may further include subsequent imaging and initial data analysis steps.

It is further envisaged that the method steps according to the invention, including or excluding steps such as nucleic acid extraction, NGS sequencing, imaging and initial data analysis be performed in a semi-automatic or automatic manner. For example, the core steps of preparing an RNA sample or a DNA sample (a DNA sample is not encompassed by the wording of the claims but is considered as useful for understanding the invention) for sequencing and optionally also the other steps such as nucleic acid extraction, NGS sequencing, imaging and initial data analysis may be performed in a sample analyzer or robotic or liquid sample handling system. The analyzer or handling system may, for example, comprise modules for one or more different assay(s) or activities, e.g. an RNA or DNA preparation module and a sequencing module, a pH sensor, a sensor for ionic concentrations etc. Also envisaged is the presence of reaction zones, which comprise one or more reagent(s) necessary for the performance of a method, e.g. buffers, ions, nucleotides, dyes etc. The analyzer may further or alternatively be equipped with an image recognition module. The analyzer may accordingly also be equipped with microfluidic elements, which allow to transport samples or sample portions to different areas of the device. Furthermore, robotic components including robotic arms etc. may be included. In further embodiments, the analyzer may be used in combination with one or more further analyzer(s). For example, a chain or conveyer structure may be provided in which a sample is analyzed by two or more analyzers, e.g. in a row. These analyzers may further be connected and/or share data with each other and/or an external database or the like. In further embodiments, the analyzers may be integrated in a laboratory management system, e.g. a laboratory information management system.

Correspondingly obtained data are typically provided in the form of sequencing reads which may be single-end or paired-end reads. Obtaining such sequencing data may further include the addition of assessment steps or data analysis steps.

The sequencing length may be any suitable sequencing read length. It is preferred to make use of sequencing reads of a length of about 50 to about 1000, preferably about 150 to about 500 nucleotides, e.g. 50, 60, 70, 80, 90, 100, 110, 120, 130, 140, 150, 160, 170, 180, 190, 200, 250, 300, 350, 400, 450, 500 or more nucleotides or any value in between the mentioned values. The length may vary depending on the specific target sequence, organism form which it is derived, genetic or genomic structure of the target sequence or scientific/diagnostic problem to be solved.

In certain embodiments the obtained sequence is aligned and/or compared with one or more reference sequences. The terms "alignment", "aligning" or "comparing" as used herein relate to the process of sequence comparison and matching a sequencing read with one or more predefined sequence, e.g. with one or more reference sequences or a part thereof. In the context of the present invention alignment exclusively relates to nucleotide sequences. For the performance of an alignment operation or sequence comparison any suitable algorithm or tool can be used. Preferred is an algorithm such as the Burrows-Wheeler Aligner (BWA), e.g. as described by Li and Durbin, 2009, Bioin-formatics, 25, 1754-1760.

It is preferred that the alignment is performed in the form of a phylogenetic comparison of the obtained sequence(s) with one or more reference sequences. Suitable algorithms for a phylogenetic comparison include PGLS (phylogenetic generalized least squares) which is used to test whether there is a relationship between two (or more) variables while accounting for the fact that lineage are not independent, or Monte Carlo simulations. An outcome of the phylogenetic comparison is typically a phylogenetic tree, which indicates relationship and lineage of compared sequences. This approach is particularly advantageous if different samples, or samples from different subjects are sequenced at the same time. Also a comparison of the outcome of a phylogenetic comparison with earlier comparison runs or literature data or independent data is envisaged.

Sequence reads may, in certain embodiments also be assembled. The assembly is typically performed with the help of reference sequence which is used as scaffold or framework allowing for a placement of the sequence reads at corresponding position after sequence comparison, e.g. in the form of contigs. A suitable tool is, for example, GAML (Boza et al., Algorithms Mol Biol. 2015, 10:18).

Also envisaged is an assembly of sequence reads without the use of a reference genome, i.e. a de novo assembly. Reads with sufficient amount of overlapping parts at the start or the end positions may be used to form contigs, i.e. sets of mutually overlapping reads. Examples of corresponding algorithms include Cortex (Iqbal et al., Nature Genetics, 2012) and SPAdes (Bankevich et al., Journal of Computational Biology, 2012). A suitable tool is, for example, ABySS (Simpson et al., Genome Res. 2009, 19(6), 1117-23).

The term "reference sequence" as used herein relates to a sequence, which is used for alignment purposes within the context of the present invention. The reference sequence is typically an organism's or entity's genomic sequence or part of a genomic sequence, e.g. a virus genome or part of it, a mammalian genome or part of it, e.g. the genomic sequence of a chromosome or a sub-section thereof. The reference sequence may further be limited to certain sectors of the genome, e.g. specific chromosomes, or parts of a chromosome, or certain genes, groups of genes or gene clusters etc. Particularly preferred are sectors, which correspond to known mutational hotspots or which have been described as being involved in the etiology of diseases. The sequence may either be provided in any suitable direction or orientation. The reference sequence may be selected as any suitable genomic sequence derivable from databases as known the skilled person. For example, a reference sequence may be derived from the reference assembly provided by the Human Genome Reference Consortium, or from the depository of genomic sequences at NCBI, e.g. for viruses (https://www.ncbi.nlm.nih.gov, last visited on April 20, 2021). For example, the genomic sequence of SARS-CoV-2 may be derived as NCBI Reference Sequence: NC_045512.2 from NCBI as mentioned above.

The present invention further envisages, in certain embodiments, a step of of sequence comparison with a reference sequence, e.g. a reference sequence as mentioned above. The comparison may be performed with any suitable tool or program, e.g. an algorithm as mentioned above. The comparison may yield results as to the presence of a sequence deviation from the reference sequence, e.g. the presence of a mutated or changed nucleotide. For a massive parallel sequencing approach, the comparison results may further be groups and/or fed into a phylogenetic algorithm or program to detect relationships between the sequences.

The present invention further envisages that the sequence information obtained from a subject's sample and/or the result of a sequence comparison as mentioned above is stored on a computer system, a database, a public sequence repository, a cloud system, a hospital computer system, a doctors association computer system, a local health organization database, a regional health organization database, a national health organization database and/or an international health organization database. The sequence information may be stored in any suitable format. It may further be linked to a subject's registration data, e.g. as defined above. The information may further be linked to one or more aggregated or derived statistical values. The information may, preferably, be evaluated with respect to a specific diagnostic or clinical question, e.g. infection by an organism, infection by a specific type of organism, presence of a certain genotype etc. Also envisaged is a linkage to an alert system comprising a connection to a subject's registration data.

It is further envisaged to connect the obtained information to a diagnostic database which may comprise information on the disease and/or on potential therapeutic options. Also included may be a conclusion on the most promising treatment, or a potential therapy plan. The corresponding information may also be derived from suitable literature sources, e.g. an electronic literature depository.

It is further envisaged that the information can be retrieved from any of the mentioned systems, e.g. by the subject, or medical practitioner, or a hospital, or a health office etc.

In specific embodiments, the preparation of sample for a target specific next generation sequencing is for the detection of a virus, a microbe or a genotype of a higher eukaryote. The virus may be any virus, preferably a positive strand ssRNA virus. It may, in particular, belong to the order of Nidovirales, Picornavirales or Tymovirales, or to the family of Coronaviridae, Picornaviridae, Caliciviridae, Flaviviridae or Togaviridae. In more preferred embodiments the virus is a rhinovirus, Norwalk-Virus, Echo-Virus or enterovirus. It may further be a Coronavirus or belong to the group of Coronaviruses, or belongs to the group of alpha or beta coronaviruses. Particularly preferred is a human or Microchiroptera (bat) coronavirus, in particular a SARS-CoV-2 virus or any mutational derivative thereof.

Further envisaged are PHEV, FcoV, IBV, HCoV-OC43 and HcoV HKU1, JHMV, HCoV NL63, HCoV 229E, TGEV, PEDV, FIPV, CCoV, MHV, BCoV, SARS-CoV, MERS-CoV or any mutational derivative thereof. The term "mutational derivative thereof" as used herein relates to virus variants, which do not have the same genomic sequence as the mentioned viruses (e.g. as defined by reference sequences such as those stored at NCBI, mentioned above) but is derived therefrom by mutational events which are typical for this virus group. These events may lead to changes in the infectious behavior of the virus, but still allows for a classification of the virus, thus identification of the virus as belonging to the group, e.g. of coronaviruses.

Also envisaged are other viruses such as a negative strand ssRNA virus including RSV, metapneumovirus, or an influenza virus; a dsRNA virus including a rotavirus; an ssDNA virus including Smacoviridae or Spiraviridae; a dsDNA virus including human papillomavirus (HPV), an adenovirus, or Herpes simplex virus Type 1 and Type 2 (HSV-1, HSV-2).

A "microbe" as envisaged by the present invention may be a bacterium, e.g. a bacterium which is pathogenic for mammals, in particular for human beings, or a fungus. Examples of bacteria to be analysed according to the present invention include Streptococcus pneumoniae, Haemophilus influenzae, Staphylococcus aureus, in particular MRSA, Escherichia coli, Salmonella spp. and Neisseria meningitidis.

The term"genotype of a higher eukaryote" as used herein relates to any part of the transcriptome or genome of a higher eukaryotic organism, e.g. a mammal, preferably a human being. Such genotype may, preferably, be associated with a diagnostically or clinically relevant or interesting situation, e.g. a disease or predisposition for a disease, or a therapeutically relevant or interesting situation. In particularly preferred embodiments, the genotype is linked to or comprises blood-group antigens or leukocyt antigens. Envisaged blood groups comprise ABO, MNS, Rhesus, Lutheran, Kell, Lewis, Duffy, Kidd, Diego, Yt, Scianna, Dombrock, Colton, Cromer, and Vel blood group. Corresponding genetic information may be known to the skilled person or can be derived from suitable litetrature or internet sources, e.g. as mentioned above.

In further particularly preferred embodiments, the genotype is linked to or comprises a human leukocyte antigen. Envisaged examples include HLA-A, HLA-B, HLA-C, HLA-E, HLA-F, HLA-G, HLA-DRA1, HLA-DRB1, HLA-DRB3/4/5, HLA-DQA1, HLA-DQB1, HLA-DPA1, or HLA-DPB1, or variants thereof. Corresponding genetic information may be known to the skilled person or can be derived from suitable literature or internet sources, e.g. as mentioned above.

In a further aspect the present invention relates to a kit for preparing an RNA sample for next generation sequencing in a one-step target enrichment. The RNA sample kit according to the present invention comprises a) a reverse transcriptase (RT); b) one or more target-specific reverse primers, suitable for the preparation of a target specific cDNA, c) a forward target specific primer comprising a first forward adaptor sequence, and a reverse target specific primer comprising a first reverse adaptor sequence; d) a forward indexing primer comprising a first forward adaptor sequence, a forward indexing sequence and a second forward adaptor sequence; and a reverse indexing primer, comprising a first reverse adaptor sequence, a reverse indexing sequence, wherein the reverse indexing sequence is different from the forward indexing sequence, and a second reverse adaptor sequence; e) desoxyribonucleoside triphosphates (dNTPs); and f) a DNA polymerase. In a preferred embodiment the kit typically comprises all these elements in one vessel. The vessel may be provided in suitable form, e.g. refrigerated or at any suitable temperature or humidity. In further embodiments, the kit may comprise the above listed components in different containers which may, for example, mixed when used, e.g. when starting the method.

The following aspect referring to a kit for preparing a DNA sample for next generation sequencing in a one-step target enrichment is not encompassed by the wording of the claims but is considered as useful for understanding the invention. In another aspect the present invention relates to a kit for preparing a DNA sample for next generation sequencing in a one-step target enrichment. The DNA sample kit comprises a) one or more target-specific forward primers and one or more target specific reverse primers, suitable for the preparation of a target specific DNA, b) a forward target specific primer comprising a first forward adaptor sequence, and a reverse target specific primer comprising a first reverse adaptor sequence; c) a forward indexing primer comprising a first forward adaptor sequence, a forward indexing sequence and a second forward adaptor sequence; and a reverse indexing primer, comprising a first reverse adaptor sequence, a reverse indexing sequence, wherein the reverse indexing sequence is different from the forward indexing sequence, and a second reverse adaptor sequence; e; d) desoxyribonucleoside triphosphates (dNTPs); and e) a DNA polymerase. In a preferred embodiment the kit typically comprises all these elements in one vessel. The vessel may be provided in suitable form, e.g. refrigerated or at any suitable temperature or humidity. In further embodiments, the kit may comprise the above listed components in different containers which may, for example, mixed when used, e.g. when starting the method.

In specific embodiments, the kit may comprise a forward indexing primer selected from the group comprising primers of SEQ ID NO: 32 to SEQ ID NO: 39. In further specific embodiments the kit may or may additionally comprise a reverse indexing primer selected from the group comprising primers of SEQ ID NO: 40 to SEQ ID NO: 51.

The kit may be formulated as diagnostic composition and may comprise suitable carriers, diluents etc. The components or ingredients of the kit may, according to the present invention, be comprised in one or more containers or separate entities. The nature of the agents is determined by the method of detection for which the kit is intended.

In further embodiments the kit may comprise synthetic RNA spike-ins. The term "synthetic RNA spike-in" as used herein relates to an RNA molecule of known sequence and quantity which is used to calibrate measurements assays. The spike-in is typically designed to bind to a DNA molecule with a matching sequence, i.e. a control probe. Since a known quantity of RNA spike-in is mixed with the experiment sample during preparation, the degree of hybridization between the spike-ins and the control probes can be used to normalize hybridization measurements of sample RNA.

The kit may optionally comprise a package insert or a leaflet with instructions. The term "package insert" or "leaflet with instructions" is used to refer to instructions customarily included in commercial packages of diagnostic or biochemical products that contain information about the usage, calibration and/or warnings concerning the use etc. The leaflet with instructions may be part of the kit.

In a further aspect the present invention relates to a use of the method or the kit as defined above for an enrichment for a rapid virus detection. The enrichment, which may be implemented with primers of group b) as mentioned above, allows for a very efficient amplification of relevant sequences and thus provided for rapid and massively performable virus detection. This approach is hence capable of saving time and resources and provides essential sequence information for a huge number of samples in a very short period of time.

In a further aspect the present invention relates to the use of the method or the kit as defined above for an enrichment for a rapid leukocyte antigen-associated gene identification. The enrichment, which may be implemented with primers of group b) as mentioned above, allows for a very efficient amplification of relevant sequences and thus provided for rapid and massively performable leukocyte antigen-associated gene detection. This approach is hence capable of saving time and resources and provides essential sequence information for a huge number of samples in a very short period of time.

In yet another aspect the relates to the use of the method or the kit as defined above for an enrichment for a rapid blood group associated gene identification. The enrichment, which may be implemented with primers of group b) as mentioned above, allows for a very efficient amplification of relevant sequences and thus provided for rapid and massively performable blood group associated gene identification. This approach is hence capable of saving time and resources and provides essential sequence information for a huge number of samples in a very short period of time.

The examples and figures provided herein are intended for illustrative purposes. It is thus understood that examples and figures are not to be construed as limiting. The skilled person in the art will clearly be able to envisage further modifications of the principles laid out herein.

### EXAMPLES

### Example 1

### Reagent composition and thermocycling conditions for one-step target enrichment

The following master-mix and cycle profile was used:

| | 12.5 | | Number of samples |
|---|---|---|---|
| **Mastermix** | | | 400 |
| H₂0 | | 0 | |
| 2x Reaction mix | 6 | 2400 | |
| MgSO₄ (50mM) | 0.2 | 80 | |
| BSA (1mg/ml) | 0.5 | 200 | |
| Prime Target F+R (10µM Stock) | 0.5 | 200 | |
| SSIII Taq Mix | 0.5 | 200 | |
| | | 0 | |
| Index F | 1.15 | 460 | |
| Index R | 1.15 | 460 | |
| RNA Template | 2.5 | | |
| Total | 12.5 | | |

### The used Cycler Profile:

| | | |
|---|---|---|
| 25°C | 2:00 | |
| 55°C | 10:00 | |
| 95°C | 3:00 | |
| 95°C | 0:15 | 45 |
| 58°C | 0:30 | cycles |

### Example 2

### Single reaction in a single well

The following components were added to the single reaction in a single well of a 96-well plate or 384-well plate:

| | |
|---|---|
| DNA or equivalent* | 4 µl |
| Index Primer, forward | 1 µl |
| Index Primer, reverse | 1 µl |
| Target-specific Primer, forward | 0.3 µl |
| Target-specific Primer, reverse | 0.3 µl |
| Master-Mix** | 4.9 µl |

| | |
|---|---|
| * Instead of extracted DNA, blood, cell suspension or lysates from buccal swabs and similar can be used. ** Master-Mix contains: H₂O, Reaction-Buffer, MgClz, DMSO, Nucleotide Mix, Taq-Polymerase | |

### Thermocycler profile

| **Cycles** | **Temp. °C** | **Time** |
|---|---|---|
| **Hold** | **95** | **10:00** |
| **10** | **95** | **00:30** |
| | **62** | **00:20** |
| | **72** | **01:30** |
| **24** | **95** | **00:30** |
| | **61,5-59,1*** | **00:20** |
| | **72** | **01:30** |
| **1** | **72** | **10:00** |
| **Hold** | **10** | **∞** |

### Example 3

### MiSeq run 12

142 samples labeled with _RP or _SR0403 and (i) include 2 different amplicons (1 virus specific + 1 internal control), (ii) in every sample the internal control and the virus specific region should be counted separately, (iii) the primer sequence is highlighted in yellow, and (iv) the target region is highlighted in red.

**Table 1. Primers used in the MiSeq run**

| **Primer name** | **Sequence 5' → 3'** | **SEQ ID NO** |
|---|---|---|
| E_Sarbeco_F1 | ACAGGTACGTTAATAGTTAATAGCGT | 1 |
| E_Sarbeco_R2 | ATATTGCAGCAGTACGCACACA | 2 |
| RP-F | AGATTTGGACCTGCGAGCG | 3 |
| RP-R | GAGCGGCTGTCTCCACAAGT | 4 |

RNAseP primers (i.e. those designated with "RP") were used for internal control. The product length based on these primers was 113 base pairs for the primer pair E_Sarbeco_F1 and E_Sarbeco_R2, and 65 base pairs for the primer pair RP-F and RP-R.Resulting products:
1) ACAGGTACGTTAATAGTTAATAGCGT ACTTCTTTTTCTTGCTTTCGTGGT ATTCTTGCTAG-TTACACTAGCCATCCTTACTGCGCTTCGAT TGTGTGCGTACTGCTGCAATAT (for E_sarbeco F1 and E_Sarbeco R2 primers) (SEQ ID NO: 5)
   and
2) AGATTTGGACCTGCGAGCG GGTTCTGACCTGAAGGCTCTGCGCGG ACTTGTGGAGA-CAGCCGCTC (for RP-F and RP-R primers). (SEQ ID NO: 6)

### Example 4

### MiSeq run 2

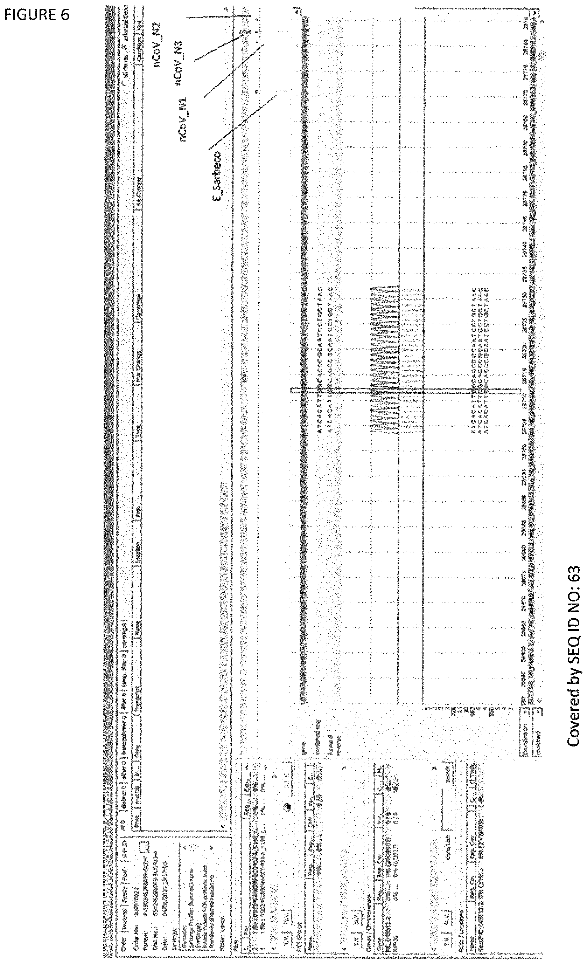

142 samples were labeled with _SC or _SC0403 and (i) includes 5 different amplicons (4 virus specific + 1 internal control), (ii) in every sample the internal control and the virus specific region should be counted separately, (iii) the primer sequence is highlighted in yellow, and (iv) the target region is highlighted in red. The MiSeq run 2 results are shown in Figure 6.

**Table 2. Primers used in the MiSeq run**

| **Primer name** | **Sequence 5' → 3'** | **SEQ ID NO** |
|---|---|---|
| 2019-nCoV_N1-F | GACCCCAAAATCAGCGAAAT | 7 |
| 2019-nCoV_Nr-R | TCTGGTTACTGCCAGTTGAATCTG | 8 |
| E_Sarbeco_F1 | ACAGGTACGTTAATAGTTAATAGCGT | 1 |
| E_Sarbeco_R2 | ATATTGCAGCAGTACGCACACA | 2 |
| RP_F | AGATTTGGACCTGCGAGCG | 3 |
| RP_R | GAGCGGCTGTCTCCACAAGT | 4 |
| 2019-nCoV_N2-F | TTACAAACATTGGCCGCAAA | 9 |
| 2019-nCov_N2-R | GCGCGACATTCCGAAGAA | 10 |
| 2019-nCov_N3-F | GGGAGCCTTGAATACACCAAAA | 11 |
| 2019-nCov_N3-R | TGTAGCACGATTGCAGCATTG | 12 |

RNAseP primers (i.e. those designated with "RP") were used for internal control. The product length based on these primers was 113 base pairs for the primer pair E_Sarbeco_F1 and E_Sarbeco_R2, 72 base pairs for the primer pair 2019-nCoV_N1-F and 2019-nCoV_N1-R, 67 base pairs for the primer pair 2019-nCoV_N2-F and 2019-nCoV_N2-R, 72 base pairs for primer pairs 2019-nCoV_N3-F and 2019-nCoV_N3-R, and 65 base pairs for the primer pair RP-F and RP-R. Resulting products:
1) ACAGGTACGTTAATAGTTAATAGCGT ACTTCTTTTTCTTGCTTTCGTGGT ATTCTTGCTAG-TTACACTAGCCATCCTTACTGCGCTTCGAT TGTGTGCGTACTGCTGCAATAT (for E_sarbeco F1 and E_Sarbeco R2 primers), (SEQ ID NO: 5)
2) AGATTTGGACCTGCGAGCG GGTTCTGACCTGAAGGCTCTGCGCGG ACTTGTGGAGA-CAGCCGCTC (for RP-F and RP-R primers), (SEQ ID NO: 6)
3) GACCCCAAAATCAGCGAAAT GCACCCCGCATTACGTTTGGTGGACCCT CAGAT-TCAACTGGCAGTAACCAGA (for 2019-nCoV_N1-F and 2019-nCoV_N1-R primers), (SEQ ID NO: 13)
4) TTACAAACATTGGCCGCAAA TTGCACAATTTGCCCCCAGCGCTTCAGCG TTCTTCG-GAATGTCGCGC (for 2019-nCoV_N2-F and 2019-nCoV_N2-R primers), (SEQ ID NO: 14) and
5) GGGAGCCTTGAATACACCAAAA GATCACATTGGCACCCGCAATCCTGCTAA CAATGCTG-CAATCGTGCTACA (for 2019-nCoV_N3-F and 2019-nCoV_N3-R primers) (SEQ ID NO: 15).

### Example 5

### NovaSeq6000 run

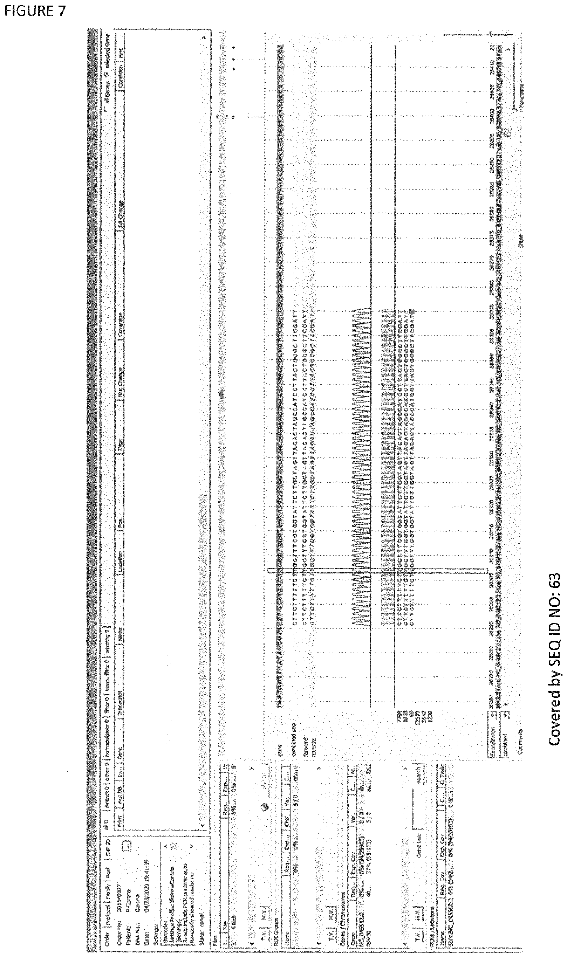

The primer sequence is highlighted in yellow, and the target region is highlighted in red. The NovaSeq results are shown in Figure 7.

**Table 3. Primers used in the MiSeq run**

| **Primer name** | **Sequence 5' → 3'** | **SEQ ID NO** |
|---|---|---|
| E_Sarbeco_F1 | ACAGGTACGTTAATAGTTAATAGCGT | 1 |
| E_Sarbeco_R2 | ATATTGCAGCAGTACGCACACA | 2 |
| RP_F | AGATTTGGACCTGCGAGCG | 3 |
| RP_R | GAGCGGCTGTCTCCACAAGT | 4 |
| nCov_N3-F | GGGAGCCTTGAATACACCAAAA | 11 |
| nCov_N3-R | TGTAGCACGATTGCAGCATTG | 12 |

RNAseP primers (i.e. those designated with "RP") were used for internal control. The product length based on these primers was 113 base pairs for the primer pair E Sarbeco_F1 and E_Sarbeco_R2, 65 base pairs for primer pairs 2019-nCoV_N3-F and 2019-nCoV_N3-R, and 65 base pairs for the primer pair RP-F and RP-R. Resulting products:
1) ACAGGTACGTTAATAGTTAATAGCGT ACTTCTTTTTCTTGCTTTCGTGGT ATTCTTGCTAG-TTACACTAGCCATCCTTACTGCGCTTCGAT TGTGTGCGTACTGCTGCAATAT (for E_sarbeco F1 and E_Sarbeco R2 primers) (SEQ ID NO: 5),
2) AGATTTGGACCTGCGAGCG GGTTCTGACCTGAAGGCTCTGCGCGG ACTTGTGGAGA-CAGCCGCTC (for RP-F and RP-R primers) (SEQ ID NO: 6), and
3) GGGAGCCTTGAATACACCAAAA GATCACATTGGCACCCGCAATCCTGCTAA CAATGCTG-CAATCGTGCTACA (for 2019-nCoV_N3-F and 2019-nCoV_N3-R primers) (SEQ ID NO: 15)

### Example 6

### Primerdesign Illumina

The following primers were designed/used in the context of Illumina NGS approaches.

**Table 4. Primer design**

| **Primer name** | **Sequence 5' → 3'** | **SEQ ID NO** |
|---|---|---|
| Nextera-Transposase + CTG modification | | |
| 2019-nCoV_N1.F | TCGTCGGCAGCGTCAGATGTG- TATAAGAGACAGCTGGAC- CCCAAAATCAGCGAAAT | 16 |
| 2019-nCoV_N2.F | TCGTCGGCAGCGTCAGATGTG- TATAAGAGACAGCTGTTA- CAAACATTGGCCGCAAA | 17 |
| 2019-nCoV_N3.F | TCGTCGGCAGCGTCAGATGTG- TATAAGAGACAGCTGGGGAGCCTT- GAATACACCAAAA | 18 |
| RP-F.F | TCGTCGGCAGCGTCAGATGTG- TATAAGAGACAGCTGAGATTTGGAC- CTGCGAGCG | 19 |
| RdRP_SARS.F | TCGTCGGCAGCGTCAGATGTG- TATAAGAGACAGCTGGTGARAT- GGTCATGTGTGGCGG | 20 |
| E_Sarbeco.F | TCGTCGGCAGCGTCAGATGTG- TATAAGAGACAGCTGACAGG- TACGTTAATAGTTAATAGCGT | 21 |
| 2019-nCoV_N1.R | GTCTCGTGGGCTCGGAGATGTG- TATAAGAGACAGCCGTCTGGTTA- CTGCCAGTTGAATCTG | 22 |
| 2019-nCoV_N2.R | GTCTCGTGGGCTCGGAGATGTG- TATAAGAGACAGCCGGCGCGACATT- CCGAAGAA | 23 |
| 2019-nCoV_N3.R | GTCTCGTGGGCTCGGAGATGTG- TATAAGAGACAGCCGTG- TAGCACGATTGCAGCATTG | 24 |
| RP.R | GTCTCGTGGGCTCGGAGATGTG- TATAAGAGACAGCCG- GAGCGGCTGTCTCCACAAGT | 25 |
| RdRP_SARSr.R | GTCTCGTGGGCTCGGAGATGTG- TATAAGAGACAGCCGCARAT- GTTAAASACACTATTAGCATA | 26 |
| E_Sarbeco.R | GTCTCGTGGGCTCGGAGATGTG- TATAAGAGACAGCCGATATT- GCAGCAGTACGCACACA | 27 |
| Index primer Forward | AATGATACGGCGACCACCGA- GATCTACAC [i5 e. g. N501 TA- GATCGC]TCGTCGG- CAGCGTCAGATGTGTATAAGAGA- CAG | 28 |
| TS-Primer Forward: | TCGTCGGCAGCGTCAGATGTG- TATAAGAGACAG + targetspec. se- quence | 29 |
| Index primer Reverse | CAAGCAGAAGACGGCATACGA- GAT [i7 e. g. 701 TCGCCTTA] GTCTCGTGGGCTCGGAGATGTG- TATAAGAGACAG | 30 |
| TS-Primer Reverse | GTCTCGTGGGCTCGGAGATGTG- TATAAGAGACAG + targetspec. sequence | 31 |

### Example 7

### Indexing primers

The following primers can be used as indexing primers in the context of the present invention.

**Table 5. Forward Indexing primers**

| **Primer name** | **Sequence 5' → 3'** | **SEQ ID NO** |
|---|---|---|
| F-Illu-IndexN509 | AATGATACGGCGACCACCGA- GATCTACACGATCTGACTCGTCGG- CAGCGTCAGATGTG- TATAAGAGACAG | 32 |
| F-Illu-IndexN502 | AATGATACGGCGACCACCGA- GATCTACACCTCTCTATT- CGTCGGCAGCGTCAGATGTG- TATAAGAGACAG | 33 |
| F-Illu-IndexN503 | AATGATACGGCGACCACCGA- GATCTACACTATCCTCTT- CGTCGGCAGCGTCAGATGTG- TATAAGAGACAG | 34 |
| F-Illu-IndexN504 | AATGATACGGCGACCACCGA- GATCTACACAGAGTAG- ATCGTCGGCAGCGTCAGATGTG- TATAAGAGACAG | 35 |
| F-Illu-IndexN505 | AATGATACGGCGACCACCGA- GATCTACACGTAAG- GAGTCGTCGGCAGCGTCAGATGTG- TATAAGAGACAG | 36 |
| F-Illu-IndexN506 | AATGATACGGCGACCACCGA- GATCTACACACTGCATA- TCGTCGGCAGCGTCAGATGTG- TATAAGAGACAG | 37 |
| F-Illu-IndexN507 | AATGATACGGCGACCACCGA- GATCTACACAAGGAG- TATCGTCGGCAGCGTCAGATGTG- TATAAGAGACAG | 38 |
| F-Illu-IndexN508 | AATGATACGGCGACCACCGA- GATCTACACCTAAGCCTT- CGTCGGCAGCGTCAGATGTG- TATAAGAGACAG | 39 |

**Table 6. Reverse Indexing primers**

| **Primer name** | **Sequence 5' → 3'** | **SEQ ID NO** |
|---|---|---|
| R-Illu-IndexN701 | CAAGCAGAAGACGGCATACGA- GATTCGCCT- TAGTCTCGTGGGCTCGGAGATGTG- TATAAGAGACAG | 40 |
| R-Illu-IndexN702 | CAAGCAGAAGACGGCATACGA- GATCTAG- TACGGTCTCGTGGGCTCGGAGAT- GTGTATAAGAGACAG | 41 |
| R-Illu-IndexN713 | CAAGCAGAAGACGGCATACGA- GATTGTGAT- GAGTCTCGTGGGCTCGGAGATGTG- TATAAGAGACAG | 42 |
| R-Illu-IndexN704 | CAAGCAGAAGACGGCATACGA- GATGCTCAG- GAGTCTCGTGGGCTCGGAGATGTG- TATAAGAGACAG | 43 |
| R-Illu-IndexN705 | CAAGCAGAAGACGGCATACGA- GATAG- GAGTCCGTCTCGTGGGCTCGGA- GATGTGTATAAGAGACAG | 44 |
| R-Illu-IndexN706 | CAAGCAGAAGACGGCATACGA- GATCAT- GCCTAGTCTCGTGGGCTCGGAGAT- GTGTATAAGAGACAG | 45 |
| R-Illu-IndexN707 | CAAGCAGAAGACGGCATACGA- GATGTAG- AGAGGTCTCGTGGGCTCGGAGAT- GTGTATAAGAGACAG | 46 |
| R-Illu-IndexN708 | CAAGCAGAAGACGGCATACGA- GATCCTCTCTGGTCTCGTGGGCTCG- GAGATGTGTATAAGAGACAG | 47 |
| R-Illu-IndexN709 | CAAGCAGAAGACGGCATACGA- GATAGCG- TAGCGTCTCGTGGGCTCGGAGAT- GTGTATAAGAGACAG | 48 |
| R-Illu-IndexN710 | CAAGCAGAAGACGGCATACGA- GATCA- GCCTCGGTCTCGTGGGCTCGGA- GATGTGTATAAGAGACAG | 49 |
| R-Illu-IndexN711 | CAAGCAGAAGACGGCATACGA- GATTGCCTCTTGTCTCGTGGGCTCG- GAGATGTGTATAAGAGACAG | 50 |
| R-Illu-IndexN712 | CAAGCAGAAGACGGCATACGA- GATTCCTCTACGTCTCGTGGGCTCG- GAGATGTGTATAAGAGACAG | 51 |

### Example 8

### Bat SARS-like coronavirus isolate bat-SL-CoVZC45 genome sequences and primer location

### GenBank: MG772933.1

>MG772933.1 Bat SARS-like coronavirus isolate bat-SL-CoVZC45, complete genome
SEQ ID NO. 52

ATATTAGGTTTTTACCTTCCCAGGTAACAAACCAACTAACTCTCGATCTCTTGTAGATCTGTTCTCTAAA
CGAACTTTAAAATCTGTGTGACTGTCACTTAGCTGCATGCTTAGTGCACTCACGCAGTTTAATTATAATT
AATTACTGTCGTTGACAGGACACGAGTAACTCGTCTATCTTCTGCAGGTTGCTTACGGTTTCGTCCGTGT
TGCAGCCGATCATCAGCATACCTTGGTTTCGTCCGGGTGTGACCGAGAGGTAAGATGGAGAGCCTTGTCC
CTGGTTTCAACGAGAAAACACACGGTCCACTCAGTTTGCCTGTTTTACAGGTTCGTGACGTGCTTGTACG
TGGCTTTGGAGACTCCGTAGAGGAAGCTTTATCAGAGGCACGTCAACATCTTAAAGATGGCACTTGTGGC
TTAGTAGAAGTTGAAAAAGGTGTTTTACCTCAACTTGAACAGCCCTATGTGTTCATCAAACGTTCTGATG
CCCGAACTGCACCTCACGGCCATGTTATGGTCGAATTAGTAGCAGAACTCGATGGCATTCAGTATGGTCG
TAGTGGTGAGACACTTGGTGTTCTTGTCCCTCATGTAGGAGAGGTACCAGTTGCTTACCGTAAAGTTCTT
CTTCGTAAGAACGGTAATAAAGGAGCTGGTGGCCATAGTTACGGCGCCGATCTAAAGTCTTTTGACTTAG
GCGACGAGCTTGGTACTGATCCTATTGAAGATTTTCAAGAAAATTGGAACACTAAACATGGCAGTGGTGT
TACCCGTGAACTCAAGCGTGAGCTTAACGGAGGTGCATACACTCGCTATGTAGACAACAACTTTTGTGGC
CCAGATGGCTACCCTCTTGAGTGCATTAAAGACCTTCTGGCTCGTGCTGGTAAGGCTTCTTATGCTTTGT
CTGAACAATTGGATTTTATTGACACTAAGAGAGGTGTGTACTGCTGCCGTGAACACGAGCATGAAATTGC
TTGGTACACGGAACGCTCTGAAAAGAGCTATGAATTGCAGACACCTTTTGAAATTAAGTTGGCAAAGAAA
TTTGACACCTTCAATGGGGAATGTCCAAATTTTGTATTTCCTCTAAATTCAACAATCAAGACCATTCAAC
CAAGGGTTGAAAAGAAAAAGCTTGATGGTTTCATGGGTAGAATTCGATCTGTCTATCCTGTTGCTTCACC
AAATGAATGCAACCAAATGTGCCTCTCAATTCTCATGAAGTGTGACCATTGTGGTGAAACTTCATGGCAG
ACGGGTGATTTTGTTAGAGCCACTTGCGAATTCTGTGGTACTGAAAATTTGACTAAAGAAGGTGCCACAA
CTTGTGGTTACTTACCTCAAAATGCTGTTGTAAAACTTTATTGTCCAGCATGTCATAATCCAGAAGTAGG
ACCTGAGCATAGTCTTGCTGAATATCATAATGAGTCTGGTTTGAAAACCGTTCTTCGTAAGGGTGGTCGT
ACCATTGCTTATGGGGGCTGTGTGTTTGCTTATGTTGGTTGCTACAACAAGTGTGCCTATTGGGTTCCAC
GTGCTAGTGCTAACATAGGCTGTAATCACACAGGTGTTGTTGGAGAAGGTTCTGAAAGTCTAAACGACAA
CCTTCTTGAAATATTGCAAAAGGAGAAAGTCAACATCAATATTGTTGGTGACTTTAAACTTAATGAAGAG
ATTGCCATTATTTTGGCATCTTTTTCTGCCTCTATAAGTGCTTTTGTAGAAACTGTAAAAGGTTTGGATT
ACAAAACATTCAAACAAATTGTTGAATCCTGTGGTAACTTTAAAGTTACGAAGGGAAAAGCAAAGAAAGG
TGCCTGGAACATAGGTGAACAAAGTTCAATACTGAGTCCGCTCTATGCGTTCCCTTCAGATGCTGCTCAT
GTTGTACGGTCTATCTTCTCACGCACACTAGAAACTGCTCACCATTCTGTGCATGTCTTACAAAAGGCTG
CTATAATTATCTTAGATGGAATTTCACAGTATTCATTGAGACTCATTGATGCTATGATGTTCACATCTGA
TTTGGTTACTAACAATCTAGTTGTAATGGCTTACATTACGGGTGGTGTTGTACAAATGACTTCACAGTGG
CTAACAAATATCTTTGGCACTGTTTATGAAAAACTTAAACCGGTTCTTGATTGGCTCGAAGAGAAATTCA
AAGAAGGTATAGAGTTTCTTAGAGACGGTTGGGAAATTGTTAAATTTATCTCAACTTGTGCTTGTGAAAT
AGTCGGTGGACAAATTGTCACCTGTGCAAAGGAAATTAAGGAGAGTGTTCAGACATTCTTTAAGCTCGTA
AATAAATTTTTGGCTTTGTGTGCTGACTCCATCATTATTGGTGGAGCTAAACTTAAAGCCTTGAATTTAG
GTGAAACATTTGTCACACACTCAAAGGGATTGTACAGAAAGTGTGTTAGATCCAGAGAAGAAACTGGCTT
ACTCTTGCCTCTGAAAGCTCCAAAAGAAATTATTTTCTTAGAGGGAGAAACACTTCCCACAGAAGTGTTA

ACAGAGGAAGTTGTCTTGAAAACTGGTGTTTTACAACCATTAGAACAACCTACTAATGAGGCTGTTGAAG
CTCCATTGATTGGTACACCAGTCTGTATTAACGGGCTCATGTTGCTCGAAATTAAAGACACAGAAAAGTA
CTGTGCCCTTGCACCTAATATGATGGTAACAAATAATACCTTCACACTTAAAGGTGGTGCACCAACAAAA
GTCACTTTTGGTGATGACACTGTGATTGAAGTGCAGGGTTACAAGAGTGTAAACATCACTTTTGAACTTG
ATGAAAGGATTGATAAAGTACTTAATGAGAAGTGCTCTAATTACACAGTTGAACTCGGTACAGAGGTAAA
CGAGTTCGCTTGTGTTGTTGCTGATGCTGTCATAAAAACTTTACAACCAGTATCTGAACTAATCATACCA
CTGGGCATTGATTTAGACGAGTGGAGTATGGCTACATACTACTTGTTTGATGAGTCCGGTGAATTTAAAT
TGTCTTCACATATGTACTGTTCTTTCTACCCTCCTGAAGATGAAGGGGAAGATGATTGTGAAGAAGGACA
GTGTGAACCATCAACTCAATATGAGTATGGTACTGAGGATGACTACCAAGGTAAACCTTTGGAGTTTGGT
GCTACTTCTTTTTCTTCTTCTTCACAGGAAGAAGAACAAGAAGAGGATTGGTTAGAATCTGATAGTCAGG
ACGGCCAAGAGACTGCAGTTGAAGAAAATAAAATACCGAGTGTTGAAGTTCCACCTGTTTTGCAGGTGGA
ATCAACACCAGTTGTTACTGAAACTAGTGAACAAAATAATTTCACAGGTTATTTAAAATTAACTGACAAT
GTCTTCATTAAAAATGCTGACATTGTAGAAGAAGCTAAAAAGGTAAAGCCTACAGTAGTTGTTAATGCAG
CTAATGTTTACCTTAAACATGGAGGAGGTGTTGCTGGAGCTTTAAATAAGGCAACTAACAACGCCATGCA
GGTTGAATCTGATAAGTACATAACTACCAATGGGCCACTAATTGTGGGTGGTGGTTGTGTTTTGAGTGGA
CATAACCTTGCTAAAAATTGTCTTCATGTTGTTGGCCCTAATGTCAACAGAGGTGAAGACATTCAATTGC
TTAAAAATGCTTATGAAAATTTCAATCAACATGAGATTTTACTCGCACCATTATTATCAGCTGGTATTTT
TGGTGCTGATCCTGTACATTCTTTAAGAGTTTGTGTAGAAACTGTTCACACAAATGTCTATCTAGTTGTC
TTTGACAAAAATCTCTATGACAAACTTGTTTCAAGCTTTTTAGAGATGAAGAGTGAAAAACAAGTAGAAC
AAAAAGTTGCTGAAAATCCTAAAGAGGAAGTTAAGCCATTTTTTACTGAAAATAAACCTTCAGTTGAACA
AAGACAACAAGCTGAAGAGAAGAAAATCAAAGCCAGTATTGAAGAAGTTACAACTACTCTAGAGGAGACC
AAGTTCCTTACAGAAAACTTGTTACTTTATATTGACATCAATGGCAATCTTCACCCAGATTCTGCCACTC
TTGTTAAAGATATTGACACCACTTTCTTGAAGAAAGATGTTCCATATATAGTGGGTGATGTTATTAAAGA
AGGCGCTTTAACTGCTGTAGTTATACCTACTAAAAAGGCTGGTGGCACTACCGAAATGCTTGCTAAAGCT
TTAAGAAAAGTGCCAACAGATAATTATATAACCACCTACCCTGGTCAGGGTTTAAATGGTTACACTGTAG
AAGAAGCAAAGACAGTGCTTAAAAAGTGTAAAAGTGCTTTTTACATTTTACCATCTATTATCTCTAATGC
GAAGCAAGAAATTCTAGGAACTGTTTCTTGGAACTTGCGAGAGATGCTCGCACATGCAGAAGAAACACGT
AAGTTAATGCCTGTTTGTATGGAGACTAAAGCTATAGTTTCAACTATACAACGTAAGTACAAAGGCATTA
AAATACAGGAGGGTGTGGTTGATTATGGTGCTAGATTTTACTTTTACACTAGTAAAACTACTGTAGCATC
ACTTATTAACACACTTAACAATCTAAATGAGACTCTTGTCACAATGCCATTAGGATATGTGACGCACGGT
CTAAATTTAGAAGAAGCTGCTCGGTACATGAGGTCTCTCAAAGTACCGGCTACAGTCTCTGTTTCTTCAC
CAGATGCTGTTACAGCATATAATGGTTATCTTACTTCTTCTTCAAAAACACCTGAAGAACACTTTATTGA
AACCGTTTCACTTGCTGGTTCCTATAAAGATTGGTCTTATTCTGGACAGTCTACACAACTAGGCATAGAA
TTTCTTAAGAGAGGTGATAAGAGTGTATATTACACCAGTAATCCCACTACATTTCACCTAGATGGTGAAA
CTATCACCTTTGATAATCTTAAGACACTTCTCTCTTTGAGAGAAGTGAGGAATATTAAAGTGTTTACAAC
AGTAGACAACATTAACCTCCACACGCAAGTTGTGGATATGTCTATGACATATGGACAACAGTTCGGTCCA
ATTTATTTGGATGGAGCTGATGTTACTAAAATAAAACCTCACAATTCACATGAAGGTAAAACATTTTATG
TTTTGCCTAATGATGATACCTTACGTGCAGAGGCTTTTGAGTACTACCATACAACTGATCCTAGTTTTCT
GGGTAGGTACATGTCAGCTTTAAACCACACTAAAAAGTGGAAATACCCACAAGTAAATGGTTTAACTTCT
ATAAAATGGGCAGATAACAATTGTTATCTTGCTACTGCATTGTTAACAATCCAACAAATAGAGTTGAAAT
TTAATCCACCAGCTTTACAAGACGCCTATTATAGGGCAAGAGCTGGTGAGGCTGCTAATTTTTGTGCACT
TATCCTGGCCTATTGTAATAAGACAGTAGGTGAGTTAGGTGATGTCAGAGAAACAATGAATTATTTGTTT
CAACATGCCAATTTAGATTCTTGTAAAAGAGTCTTGAATGTGGTGTGTAAAACTTGTGGACAACAGCAAA
CAACTCTCAAGGGTGTAGAAGCTGTTATGTATATGGGCACACTTTCTTATGAACAACTTAAGAAGGGTGT
GCAGATACCTTGTATGTGTGGTAAACAAGCTACACAATATCTGGTACAACAAGAGTCACCTTTTGTTATG
ATGTCTGCACCGCCCGCCCAATATGAACTTAAGCATGGTACATTTGTTTGTGCTAGTGAGTATACTGGTA
ATTACCAGTGTGGTCACTACAAACATATAACTTCTAAAGAAACCTTGTATTGCATAGATGGCGCTTTACT
TACAAAGTCCTCTGAGTATAAAGGTTCTATTACAGATGTTTTCTATAAAGAAAACAGTTATACAACAACC
ATAAAACCAGTTACATACAAGTTGGATGGTGTTGTTTGTACAGAAATTGATCCTAAGTTGGATGGTTATT
ATAAGAAAGATAATTCTTATTTCACAGAGCAACCAATTGATCTTGTACCAAACCAACCTTACCCGAATGC
AAGCTTTGACAATTTTAAGTTTGTATGTGATAATACCAAATTTGCCGATGATTTAAATCAATTGTCTGGT
TATAAGAAACCTGCCTCGAGAGAGCTTAAGGTTACATTCTTTCCTGACTTAAATGGTGATGTAGTGGCTA
TTGATTATAAGCACTACACACCTTCTTTTAAGAAAGGAGCTAAATTGCTGCATAAGCCAATTGTTTGGCA
TGTTAACAATGCAACTAACAAAGCAACGTACAAACCAAATATTTGGTGCATACGTTGTCTTTGGAGTACA
AAACCGGTTGAAACATCAAATTCTTTTGATGCACTGGAATTAGGGGACACACAGGGAATGGATAATCTTG

CCTGTGAAGTTCTAAAACCAGTCTCTGAAGAAGTAGTGGAAAATCCTACCATACAGAAAGACATTCTTGA
GTGTAATGTGAAAACTACCGAAGTTGTAGGAGACATTATACTTAAACCGGCAAGTGATGGTCTAAAAATT
ACAAAAGAGGTTGGTCATACAGACCTAATGGCTGCTTATGTTGACAATTCAAGTCTTACTATTAAGAAAC
CTAATGAATTATCCAGAGTATTAGGTTTGAAAACTTTAGCCACTCATGGCTTGGCTGCTATTAATAGTGT
TCCTTGGGACACTATAGCTAATTATGTTAAGCCTTTCCTTAATAAGGTTGTTAGCACAACTACTAACATA
GTTACACGGTGTCTAAACCGTGTTTGTACTAATTATATGCCTTATTTGTTTACTTTATTGCTACAATTGT
GTACTTTTACTAAAAGTACAAATTCTAGAATAAGAGCATCTATGCCAACCACTATAGCAAAGAATACTGT
TAAAAGTGTTGGTAAATTTTGTATAGAGGCTTCATTTAATTATTTGAAGTCACCTAATTTTTCTAAATTG
ATAAATATTGTAATTTGGTTTTTATTATTAAGTGTTTGCCTAGGTTCTTTAATCTATTCAACTGCTGCTT
TAGGTGTCTTAATGTCTAATTTAGGCATGCCTTCTTATTGTACTGTTTACAGAGAAGGTTATTTGAACTC
TACTAATGTCACTACTGCAACCTACTGCACTGGTTCTATACCTTGTAGTGTTTGTCTTAGTGGTTTAGAT
TCTTTGGATACTTACCCATCCTTAGAAACTATACAAATTACCATTTCGTCTTTTAAATGGGATTTAACTG
CTTTTGGTCTAGTTGCAGAGTGGTTTTTGGCATATATTCTTTTTACTAGGTTCTTTTATGTACTTGGATT
GGCTGCAATCATGCAATTGTTTTTCAGCTATTTTGCAGTACATTTTATTAGTAATTCTTGGCTTATGTGG
TTAATAATTAATCTTGTACAAATGGCCCCAATTTCAGCTATGGTTAGAATGTATATTTTCTTTGCATCAT
TTTATTATGTATGGAAAAGTTATGTGCATGTTGTAGATGGTTGTACTTCATCAACTTGTATGATGTGTTA
TAAACGTAATAGAGCAACAAGAGTTGAATGTACAACTATTGTTAATGGTGTTAGAAGGTCCTTTTATGTC
TATGCTAATGGAGGTAAAGGCTTTTGCAAACTACATAACTGGAATTGTATTAATTGTGATACATTCTGTG
CTGGTAGTACATTTATTAGTGACGAAGTTGCTAGAGACTTATCACTACAGTTTAAAAGACCAATAAATCC
TACTGACCAGTCTTCCTATATTGTTGATAGTGTTACAGTGAAGAATGGTTCCATCCATCTTTACTTTGAT
AAGGCTGGTCAAAAGACTTATGAAAGACATTCTCTCTCTCATTTTGTTAACTTAGACAATCTGAGAGCTA
ATAACACTAAGGGTTCATTGCCTATTAATGTTATAGTTTTTGATGGTAAATCAAAATGTGAAGAATCATC
TGCTAAATCAGCGTCTGTTTATTATAGTCAGCTTATGTGTCAACCTATACTGTTACTGGACCAGGCATTA
GTGTCTGATGTTGGTGACAGTGCAGAAGTTGCAGTTAAGATGTTTGATGCTTATGTTAATATATTTTCAT
CAACTTTCAATGTTCCAATGGAAAAACTCAAAGCATTAGTTGCGACTGCAGAAGCTGAACTTGCAAAGAA
TGTGTCTTTAGACAACGTCTTATCTACTTTTATTTCAGCAGCTCGTCAAGGGTTTGTTGATTCAGATGTA
GAAACTAAAGATGTTGTTGAATGTCTTAAATTGTCACACCAATCTGACATAGAAGTTACAGGTGACAGTT
GTAACAATTACATGCTCACTTATAACAAAGTTGAAAACATGACACCTCGGGATCTTGGTGCTTGTATTGA
TTGTAGTGCACGTCATATCAATGCACAGGTGGCAAAAAGTCATAACATAGCTTTGATTTGGAATGTTAAA
GATTTCATGTCATTGTCTGAACAACTACGAAAACAAATACGCAGTGCTGCTAAGAAGAATAACTTGCCTT
TTAGATTGACATGTGCAACCACTAGACAAGTTGTTAATGTTGTTACAACAAAAATAGCACTTAAGGGTGG
TAAAATTGTTAACAACTGGTTGAAGCAGCTGATTAAGGTTACACTTGTGTTTCTTTTCATCACTGTTATC
TTCTATTTAATAACACCTGTTCATGTCATGTTTAAACACAATGACTTTTCAAGTGAAATTATAGGATACA
AGGCTATTGATGGTGGTGTCACTCGTGACATAGCGTCAACAGATACTTGTTTTGCTAACAAACATGCTGA
CTTTGACTCTTGGTTTAGTCAGCGCGGTGGTAGTTATACTAATGATAAAGCTTGCCCATTGGTAGCAGCT
GTTATCACAAGGGAAGTTGGCTTTGTTGTGCCCGGTTTGCCTGGCACAATATTACGCACAATTAATGGTG
ATTTTTTGCATTTTCTTCCTAGAGTGTTTAGTGCGGTAGGTAACATTTGTTACACTCCTTCTAAACTTAT
AGAGTACACTGACTTTGCAACATCGGCATGCGTTTTAGCTGCTGAATGTACCATTTTTAAAGATGCTTCT
GGTAAACCAGTACCTTATTGTTATGATACTAATGTACTAGAAGGTTCTGTTGCGTATGAAAGTCTCCGCC
CTGACACACGCTATGTGCTCATGGACGGTTCTATAATTCAATTCCCTAACACTTACCTTGAAGGTTCTGT
TAGAGTAGTAACAACTTTTGATTCAGAGTATTGTAGACATGGTACTTGCGAAAGATCAGAGGCTGGCATT
TGTGTATCTACTAGTGGTAGATGGGTACTTAATAATGATTATTACAGATCCTTGCCAGGAGTTTTTTGTG
GTGTAGATGCTGTGAATTTACTTACTAATATGTTCACGCCATTAATTCAACCTATTGGTGCTTTGGACAT
ATCTGCATCTATTGTAGCAGGTGGTGTTGTAGCTATTATAGTAACTTGTCTAGCCTACTACTTCATGAGG
TTTAGAAGAGCTTTTGGTGAATACAGTCATGTAGTTGCCTTTAACACTCTACTATTCTTTATGTCATTCA
CTGTACTCTGTTTAACACCAGTCTATTCATTCTTACCTGGTGTTTATTCTGTTATTTACTTGTACTTGAC
ATTTTATCTTACTAATGATGTTTCTTTCTTAGCACATATCCAATGGATGGTTATGTTCACACCCTTAGTG
CCTTTCTGGATGACAATTGTTTATGTCATTTGCATTTCCACAAAGCATTTTTATTGGTTCTTTAGTAACT
ACCTAAAGAGACGTGTAGTCTTTAATGGTGTTTCCTTTAGTACATTTGAGGAGGCTGCATTATGTACCTT
TTTGTTAAATAAAGAAATGTATCTGAAATTGCGTAGTGATGTACTTCTACCTCTTACGCAATACAATAGA
TATTTAGCTCTTTATAATAAGTACAAGTATTTTAGTGGGGCCATGGACACTACCAGTTATAGAGAAGCAG
CTTGCTGTCATCTGGCTAAGGCTATAAATGATTTCAGTAATTCAGGTTCTGATGTCCTCTACCAACCACC
ACAAACTTCAATCACATCAGCGGTTTTGCAGAGTGGTTTTAGAAAAATGGCATTCCCATCTGGTAAAGTT
GAAGGTTGCATGGTACAAGTTACTTGTGGTACCACTACACTTAATGGTCTTTGGCTTGATGATGTAGTTT
ACTGTCCACGACATGTGATCTGCACTTCTGAAGACATGCTCAATCCTAATTATGAAGATTTACTTATACG

TAAATCTAACCATAATTTTTTAGTTCAGGCTGGTAATGTTCAACTTAGAGTTGTTGGACATTCTATGCAA
AATTGTGTTCTTAAGCTTAAAGTAGATACAGCTAATCCTAAGACACCTAAGTATAAGTTTGTGCGCATTC
AACCCGGACAGACTTTTTCAGTATTAGCCTGTTACAATGGTTCACCATCTGGTGTTTACCAATGTGCCAT
GAGACCTAATTTTACTATTAAGGGTTCATTCCTTAATGGTTCATGTGGTAGTGTTGGTTTTAATATAGAC
TATGACTGTGTCTCTTTTTGTTATATGCATCATATGGAGTTACCAACGGGAGTTCATGCTGGCACAGACT
TAGAAGGTACCTTCTACGGACCTTTTGTTGACAGACAGACAGCACAAGCGGCTGGTACTGACACAACTAT
TACAGTTAATGTTCTAGCTTGGTTGTATGCAGCTGTTATAAACGGAGATAGATGGTTCCTTAATAGGTTT
ACCACAACTCTAAACGATTTTAATCTTGTGGCTATGAAGTATAATTATGAACCTCTAACACAAGACCATC
TTGACATACTAGGACCTCTTTCAGCTCAAACTGGAATTGCAGTCCTAGATATGTGTGCTTCATTAAAAGA
ATTATTACAAAATGGTATGAATGGACGTACCATATTGGGTAGTGCTTTATTAGAAGATGAATTTACACCT
TTCGATGTTGTTAGACAATGTTCAGGTGTCACCTTTCAAAGTGCAGTGAAAAGGACAATCAAGGGCACGC
ACCATTGGTTGTTGCTTACAGTTTTGACTTCACTCTTAGTTTTAGTTCAGAGTACTCAATGGTCTTTGTT
CTTCTTTGTGTATGAAAATGCCTTTATGCCTTTTGCTATGGGTATTATTGCTATGTCTGCTTTTGCTATG
ATGTTTGTCAAACATAAGCATGCATTCCTCTGTTTGTTCCTGTTACCTTCTCTTGCTACTGTAGCTTATT
TTAATATGGTCTACATGCCTGCTAGTTGGGTGATGCGTATTATGACATGGTTGGATATAGTTGATACTAG
TTTGTCTGGTTTCAAGCTAAAGGACTGTGTTATGTATGCATCAGCTGTAGTGTTATTAATCCTCATGACA
GCAAGAACCGTATATGATGATGGTGCTAGAAGAGTTTGGACACTTATGAATGTCCTGACACTCGTTTATA
AAGTTTATTATGGTAATGCTTTAGACCAAGCTATTTCCATGTGGGCTCTTATAATCTCTGTTACTTCTAA
CTACTCAGGTGTAGTTACAACTGTCATGTTTTTGGCCAGAGGTATTGTTTTTATGTGTGTTGAGTATTGT
CCTATCTTCTTTATAACTGGCAATACACTCCAGTGTATAATGCTAGTTTATTGTTTCTTAGGTTATTTCT
GTACTTGTTATTTTGGTCTCTTCTGTTTACTCAACCGCTATTTTAGGCTTACTCTTGGTGTTTATGACTA
TTTAGTCTCTACACAAGAATTTAGGTACATGAACTCTCAGGGGCTCCTGCCACCTAAGAGTAGTATTGAC
GCTTTCAAGCTTAACATTAAATTGTTGGGCATTGGAGGTAAACCTTGTATTAAGGTTGCTACTGTACAGT
CTAAAATGTCTGACGTAAAGTGCACATCAGTAGTGCTTCTCTCAGTTCTTCAGCAACTTAGAGTAGAGTC
ATCTTCTAAATTGTGGGCACAGTGTGTACAACTTCACAATGATATTCTTCTTGCCAAGGACACTACTGAA
GCTTTTGAGAAGATGGTTTCACTTTTGTCTGTTTTGCTGTCCATGCAGGGTGCTGTAGACATTAACAAGT
TGTGCGAGGAAATGCTCGACAACCGTGCTACCCTTCAGGCTATTGCTTCAGAATTCAGTTCTTTACCTTC
ATATGCTGCTTATGCCACTGCTCAAGAGGCTTATGAGCAGGCTGTAGCAAATGGTGATTCTGAAGTTGTT
CTTAAAAAGTTAAAGAAATCTTTGAATGTGGCTAAATCTGAGTTTGACCGTGATGCTGCCATGCAACGTA
AGTTGGAAAAGATGGCGGATCAGGCTATGACCCAAATGTACAAGCAGGCAAGATCTGAGGACAAGAGGGC
AAAAGTAACTAGTGCAATGCAAACTATGCTTTTCACTATGCTTAGAAAACTTGATAATGATGCACTTAAC
AACATTATCAACAATGCACGTGATGGTTGTGTACCACTCAACATCATACCACTCACAACAGCAGCAAAAC
TCATGGTTGTTGTCCCTGACTATGGAACCTACAAGAATACTTGTGATGGTAACACTTTTACATATGCATC
AGCACTCTGGGAAATCCAGCAAGTTGTTGATGCAGATAGTAAAATTGTGCAGCTTAGTGAAATCAACATG
GACAACTCACCAAATTTGGCTTGGCCTCTTATTGTTACTGCTTTAAGAGCCAATTCAGCTGTCAAACTAC
AGAACAATGAGCTGAGTCCAGTAGCACTACGACAGATGTCCTGTGCGGCTGGTACTACACAGACAGCTTG
TACTGATGACAACGCACTTGCCTACTATAACAATTCTAAGGGAGGTAGGTTTGTGCTTGCATTACTATCA
GACCACCAGGATCTCAAATGGGCTAGATTCCCTAAGAGTGATGGTACAGGTACTATCTATACAGAATTGG
AACCACCTTGCAGGTTTGTTACAGACACACCTAAAGGACCTAAAGTGAAGTATTTGTACTTTATTAAGGG
TCTTAACAACCTAAATAGAGGTATGGTACTGGGTAGTTTGGCTGCCACAGTACGTCTTCAGGCTGGTAAT
GCGACAGAAGTGCCTGCCAATTCAACTGTGCTTTCTTTTTGTGCTTTTGCTGTAGACCCAGCTAAAGCTT
ACAAAGATTACCTAGCAAGTGGTGGACAACCAATCACGAATTGTGTGAAGATGTTGTGTACACACACTGG
TACAGGACAGGCAATCACGGTAACACCAGAAGCCAATATGGATCAAGAATCCTTTGGTGGTGCTTCATGC
TGTCTGTATTGTAGATGCCACATTGATCATCCAAATCCTAAGGGATTTTGTGACTTGAAAGGTAAGTATG
TTCAAATACCTACCACTTGTGTTAATGACCCTGTGGGGTTTACACTCAGAAACACAGTCTGTACCGTCTG
CGGAATGTGGAAAGGTTATGGCTGTAGTTGTGATCAACTCCGCGAACCCATGATGCAGTCTGCGGATGCG
TCAACGTTTTTAAACGGGTTTGCGGTGTAAGTGCGGCCCGTCTTACACCGTGCGGCACAGGCACTAGCAC
TGATGTCGTTTATAGGGCTTTTGATATTTACAACGAGAAAGTTGCTGGTTTTGCAAAGTTCCTAAAAACT
AATTGCTGTCGCTTCCAAGAAAAGGATGAGGAAGGCAATTTATTAGACTCTTATTTCGTAGTTAAGAGGC
ACACAATGTCCAACTACCAACATGAAGAGACTATTTACAACTTGGTTAAAGGGTGTCCAGCCGTTGCTGT
TCATGACTTTTTCAAGTTTAGAGTAGATGGTGACATGGTACCACATATATCACGTCAACGTCTAACTAAA
TACACAATGGCTGATTTAGTCTATGCTCTACGTCATTTTGACGAGGGCAATTGTGATACATTAAAGGAAA
TACTTGTCACATACAAATGTTGTGATGACAACTATTTCAATAAGAAGGATTGGTATGACTTTGTAGAAAA
TCCTGATGTTCTACGCGTATACGCGAACCTAGGTGAGCGTGTACGTCAAGCCTTATTGAAAACTGTGCAA
TTCTGCGATGCTATGCGCGATGCGGGTATCGTAGGTGTACTGACACTAGACAATCAGGATCTGAATGGGA

ATTGGTACGACTTCGGAGATTTCGTACAGGTAGCACCAGGCTGCGGAGTCCCTATTGTGGATTCATACTA
TTCTTTGCTGATGCCTATTCTGACACTCACAAGGGCTTTGGCTGCTGAGTCCCATATGGACGCTGATCTC
GCGAAGCCACTTATTAAGTGGGATTTGCTGAAATATGATTTCACGGAAGAGAGACTATGTCTTTTCGACC
GTTATTTTAAATATTGGGACCAGACGTACCATCCTAATTGTATTAATTGTTTGGATGACAGGTGTATCCT
TCATTGTGCAAACTTTAATGTGTTATTTTCTACTGTGTTTCCACCTACGAGTTTTGGACCACTAGTAAGA
AAGATATTTGTAGATGGTGTACCTTTTGTTGTTTCAACGGGATACCATTTCCGTGAGCTAGGGGTTGTAC
ATAATCAGGATGTAAACTTACATAACTCGCGTCTCAGTTTTAAGGAACTTTTAGTGTACGCTGCTGATCC
AGCTATGCATGCTGCCTCTGGCAATTTGTTGTTAGACAAACGCACTACATGCTTTTCAGTAGCTGCACTA
ACAAACAATGTTGCTTTTCAAACTGTCAAACCCGGTAATTTTAATAAAGACTTTTATGACTTTGCTGTGT
CTAAAGGCTTCTTTAAGGAAGGAAGTTCTGTTGAATTAAAACACTTCTTCTTTGCTCAGGATGGCAATGC
TGCTATCAGTGATTATGACTATTACCGTTATAATCTGCCAACAATGTGTGATATTAGACAACTCCTATTC
GTAGTTGAGGTTGTCGATAAATATTTTGATTGTTACGACGGTGGCTGTATCAATGCCAACCAAGTTATCG
TTAACAATCTGGACAAATCAGCCGGTTTCCCATTTAATAAGTGGGGTAAGGCTAGACTTTATTATGACTC
AATGAGTTATGAGGATCAAGATGCACTGTTCGCATACACTAAGCGTAACGTCATCCCTACAATAACTCAA
ATGAATCTTAAGTATGCCATTAGTGCAAAGAATAGAGCTCGCACTGTTGCTGGTGTCTCTATCTGTAGTA
CTATGACCAATAGACAGTTTCATCAGAAATTATTAAAGTCAATAGCCGCCACTAGAGGAGCTACTGTGGT
AATTGGAACAAGCAAATTTTATGGTGGCTGGCATAACATGTTAAAAACTGTTTACAGTGATGTAGAAAGT
CCTCACCTTATGGGTTGGGACTACCCAAAATGTGACAGAGCCATGCCTAATATGCTTAGAATCATGGCTT
CCCTCGTTCTTGCTCGCAAACATAGCACTTGTTGTAACTTGTCACACCGTTTCTATAGATTAGCTAATGA
GTGTGCACAAGTATTAAGTGAGATGGTCATGTGTGGCGGCTCATTATATGTGAAACCAGGTGGAACATCA
TCCGGTGATGCCACAACTGCTTATGCTAATAGTGTGTTTAACATCTGTCAAGCAGTAACAGCTAATGTAA

**Table 7. Primers used to obtain above sequence**

| **Primer name** | **Sequence 5' → 3'** | **SEQ ID NO.** |
|---|---|---|
| RdRP_SARSr-F2 | GTGARATGGTCATGTGTGGCGG | 56 |
| RdRP_SARSr-R1 | CARATGTTAAASACACTATTAGCATA | 57 |
| RdRP_SARSr-P2 | FAM-CAGGTGGAACCTCATCAGGA-GATGC-BBQ | 58 |
| RdRP_SARSr-P1 | FAM-CCAGGTGG-WACRTCATCMGGTGATGC-BBQ | 59 |

SEQ ID NO. 53:

ATGCACTTCTTTCAACTGATGGTAATAAGATTGCTGATAAGTATGTCCGCAACCTTCAACACAGACTTTA
CGAGTGTCTCTATAGAAATAGAGACGTAGATCAGGAATTCGTGGATGAATTTTATGCATATTTGCGTAAG
CATTTCTCCATGATGATTCTTTCTGATGATGCCGTCGTATGCTATAATAGTAATTACGCGGCACAGGGTC
TAGTAGCTAGCATTAAGAACTTTAAAGCAGTTCTTTATTACCAAAATAATGTATTCATGTCTGAGGCAAA
ATGTTGGACTGAGACTGACCTTACTAAAGGACCTCATGAATTTTGTTCTCAGCATACCATGCTAGTTAAA
CAGGGAGATGATTATGTGTACCTGCCTTATCCAGATCCATCCAGAATATTAGGCGCAGGCTGTTTTGTCG
ATGACATTGTCAAAACAGATGGTACACTTATGATTGAAAGGTTTGTGTCATTAGCAATTGACGCCTATCC
ACTTACAAAGCACCCTAATCAAGAGTATGCTGATGTTTTCCATTTATACTTACAGTATATTAGGAAATTA
CATGATGAGCTTACTGGTCACATGTTGGACATGTACTCTGTAATGCTAACTAATGACAACACCTCGAGGT
ACTGGGAACCTGAGTTTTATGAGGCAATGTACACACCACACACAGTTTTGCAAGCTGTAGGTGCGTGTGT
GTTATGCAATTCACAGACTTCACTTCGTTGCGGTGCTTGTATTAGGAGACCTTTCCTTTGTTGCAAGTGC
TGCTATGATCACGTCATCTCAACATCACATAAATTAGTGTTGTCTGTTAATCCCTATGTTTGCAATGCAC
CCGGTTGTGATGTCACAGACGTAACACAACTCTATTTGGGAGGTATGAGCTATTACTGCAAGTCACATAA
ACCACCCATTAGTTTTCCGTTGTGTGCTAATGGTCAGGTTTTTGGTTTGTACAAAAACACATGTGTGGGC
AGTGATAACGTAACTGACTTCAATGCAATAGCGACATGCGACTGGACTAATGCTGGCGATTACATACTTG
CCAACACTTGCACAGAGAGACTCAAACTTTTTGCAGCGGAAACGCTCAAAGCTACTGAGGAAACATTCAA
ACTATCTTATGGTATTGCCACTGTTCGTGAAGTACTGTCAGATAGAGAACTTCATCTTTCATGGGAGGTA
GGAAAACCTAGACCACCATTGAATAGAAATTACGTCTTTACTGGTTACCGTGTGACCAAAAATAGTAAAG
TACAGATTGGAGAGTATACCTTTGAAAAAGGTGACTATGGTGATGCTGTTGTGTACAGAGGTACTACAAC

TTATAAATTGAATGTTGGTGATTACTTTGTGTTAACATCACACACAGTAATGCCACTAAGTGCACCAACA
CTAGTGCCACAAGAGCACTATGTGCGAATAACTGGCTTATACCCTACACTTAATATCTCTGATGAGTTTT
CTAGCAATGTTGCAAATTACCAAAAGGTCGGCATGCAGAAGTACTCCACACTCCAGGGACCACCTGGTAC
TGGTAAGAGTCACTTTGCTATTGGACTTGCCCTCTACTACCCATCTGCCCGCATAGTGTATACAGCTTGC
TCTCATGCTGCTGTTGATGCGCTATGTGAGAAGGCATTAAAATACTTGCCTATAGATAAGTGTAGTAGAA
TTATTCCTGCACGTGCGCGTGTAGAGTGTTTTGACAAATTCAAAGTGAATTCAACCTTAGAACAGTATGT
TTTCTGCACTGTCAATGCGCTGCCTGAAACTACTGCTGATATAGTGGTCTTTGATGAAATTTCAATGGCC
ACTAATTATGATTTGAGTGTTGTCAATGCCAGACTACGTGCAAAACACTACGTTTACATTGGTGATCCGG
CTCAATTACCTGCACCACGCACATTGCTAACAAAAGGCACACTTGAACCAGAATATTTCAATTCAGTGTG
CAGACTTATGAAAACAATAGGTCCAGACATGTTCCTTGGGACTTGTCGTCGTTGTCCCGCTGAAATTGTT
GACACAGTGAGTGCTTTAGTTTATGATAATAAGCTAAAAGCACACAAAGAGAAGTCAGCTCAATGCTTCA
AGATGTTTTACAAGGGTGTGATTACGCATGATGTGTCATCCGCAATCAACAGACCACAAATAGGTGTAGT
AAGAGAATTTCTTACGCGCAATCCAGCTTGGAGAAAAGCTGTTTTTATTTCACCATATAATTCACAGAAT
GCAGTGGCATCAAAGATTTTAGGGTTGCCTACTCAAACTGTTGATTCTTCACAGGGTTCTGAATATGACT
ATGTCATATTCACACAAACCACTGAGACTGCACACTCTTGTAATGTAAACCGCTTTAATGTGGCCATTAC
AAGAGCAAAGATTGGCATTTTGTGCATAATGTCTGATAGAGACCTTTATGACAAGCTTCAATTTATGAGT
CTAGAAGTACCGCGTCGAAATGTGGCTACTTTACAAGCAGAAAATGTGACTGGACTCTTTAAGGACTGTA
GCAAGATCATTACTGGTCTTCATCCAACACAGGCACCTACGCATCTCAGTGTTGATACTAAATTCAAGAC
TGAAGGACTTTGTGTCGACATACCAGGAATACCAAAGGACATGACCTATCGTAGACTCATCTCTATGATG
GGCTTTAAAATGAATTACCAAGTTAATGGTTACCCTAATATGTTTATCACCCGTGAAGAAGCTATTCGTC
ACGTTCGTGCATGGATAGGCTTTGATGTTGAGGGTTGTCATGCGACTAGAGATGCTGTAGGAACAAATCT
ACCACTCCAGTTAGGGTTTTCAACAGGTGTTAACCTAGTGGCTGTACCAACTGGCTATGTTGACACTGAG
CACAGCACAGAATTTACCAGAGTTAATGCAAAACCTCCTCCAGGTGATCAATTTAAGCATCTTATACCAC
TTATGTACAAAGGCTTGCCCTGGAACGTGGTGCGTATTAAGATTGTTCAAATGCTCAGTGATACACTGAA
AGGATTATCAGACAGAGTTGTGTTTGTCCTTTGGGCACATGGCTTTGAACTTACATCGATGAAGTATTTT
GTTAAGATCGGACCAGAAAGAACGTGTTGTCTGTGTGACAAACGCGCGACTTGCTTCTCTACTTCATCTG
ACACTTATGCCTGTTGGAATCACTCTGTGGGCTTTGACTATGTCTATAACCCGTTTATGATTGATGTCCA
GCAGTGGGGTTTTACAGGTAACCTTCAAAGTAACCATGATCAACACTGCCAAGTGCATGGTAATGCCCAT
GTAGCTAGTTGTGATGCTATCATGACTAGATGTCTTGCAGTCCATGAGTGCTTTGTTAAGCGCGTTGATT
GGTCTGTTGAATACCCGATTATTGGAGATGAACTGAAGATTAATGCCGCATGCAGAAAAGTACAGCATAT
GGTTGTTAAATCTGCATTGCTTGCTGATAAATTCCCAGTTCTTCATGATATAGGAAACCCAAAGGCTATT
AGATGTGTGCCGCAGTCTGAAGTGGACTGGAAATTCTACGACGCTCAGCCTTGCAGTGACAAAGCTTATA
AAATAGAAGAACTCTTCTACTCATATGCCACACATCATGACAAGTTCACAGATGGTGTTTGCTTGTTTTG
GAACTGTAACGTTGATCGTTACCCGGCTAATGCTATTGTGTGTAGGTTTGATACTAGAGTGCTTTCTAAT
TTAAACCTACCAGGTTGTGATGGTGGTAGTTTGTATGTTAATAAGCATGCGTTCCACACTCCAGCTTTTG
ATAAGAGTGCATTTACACATTTGAAACAACTGCCTTTCTTTTATTACTCTGACAGTCCGTGTGAGTCTCA
TGGTAAACAGGTTGTGTCAGATATTGATTATGTCCCACTAAAGTCTGCTACGTGTATTACACGATGCAAC
TTAGGTGGTGCCGTTTGTAGACATCATGCAAACGAGTACAGACAGTACTTGGATGCATATAATATGATGA
TTTCTGCTGGATTTAGCCTTTGGATTTATAAACAATTTGATACTTACAACTTGTGGAACACTTTCACCAA
GTTGCAGAGTTTAGAAAATGTGGCTTATAATGTTATCAACAAGGGACACTTTGATGGACAGAATGGTGAA
GCACCTGTGTCTATCGTTAATAATGCTGTTTACACTAAGTTAGATGGTGTTGATGTGGAGATCTTTGAAA
ATAAGACAACACTTCCTGTTAATGTTGCATTTGAGCTTTGGGCTAAACGTAACATTAAACCGGTGCCAGA
GATTAAAATACTCAATAATTTGGGTGTTGATATCGCTGCTAATACTGTTATATGGGACTACAAGAGAGAA
GCGCCAGCGCATGTTTCTACAATAGGTGTCTGTACAATGACTGACATTGCAAAGAAACCTACTGAGAGTG
CTTGTTCATCACTTACTGTCTTATTTGATGGTAGAGTTGAGGGACAGGTAGACCTTTTTAGAAACGCCCG
TAATGGTGTTTTAATAACAGAAGGTTCAGTTAAGGGCTTAACACCTTCGAAAGGACCTGCACAGGCTAGT
GTCAACGGAGTCACATTAATTGGAGAATCAGTAAAAACACAGTTCAATTACTTTAAGAAAGTGGATGGCA
TTATTCAGCAATTGCCAGAAACCTACTTTACTCAAAGCAGAGACTTAGAGGATTTCAAGCCCAGATCACA
AATGGAAACTGATTTCCTTGAGCTCGCTATGGATGAATTCATAGAACGATATAAGCTAGATGGCTATGCT
TTCGAGCACATCGTTTATGGAGATTTTAGTCATGGACAATTAGGCGGACTTCATTTATTGATAGGACTGG
CCAAAAGGTCACAGGACTCACTGTTAAAGCTAGAGGATTTTATTCCTATGGATAGCACAGTGAAAAACTA
CTTCATAACAGATGCGCAAACGGGTTCATCTAAGTGTGTATGCTCTGTTATCGACCTTTTACTTGATGAC
TTTGTTGAAATAATAAAGTCACAAGATCTTTCAGTGGTTTCAAAAGTAGTCAAAGTTACGATTGATTATA
CAGAAATTTCATTTATGCTTTGGTGTAAAGATGGGCATGTGGAAACTTTTTACCCAAAATTACAATCTAG
TCAAGCATGGCAACCAGGTGTTGCTATGCCTAATCTCTATAAAATGCAGAGAATGTTACTGGAAAAGTGT

GATCTTCAAAATTATGGTGATAGTGCTATATTGCCTAAAGGCATAATGATGAATGTCGCAAAGTACACTC
AACTGTGTCAGTATTTAAATACACTTACTTTAGCTGTGCCCTATAATATGAGAGTTATACATTTTGGCGC
GGGCTCTGATAAAGGAGTAGCACCTGGCACAGCTGTTCTTAGACAGTGGTTGCCAACTGGTACACTACTT
GTCGATTCTGATTTAAATGATTTTGTTTCTGATGCAGACTCAACATTAATTGGTCATTGTGCAACCGTAC
ATACGGCTAATAAATGGGATCTCATTATTAGCGATATGTATGATCCTAAGACTAAAAATGTTACAAAAGA
GAATGATTCCAAAGAAGGATTTTTCACTTACATTTGTGGATTTATACAGCAAAAATTAGCCCTCGGAGGT
TCCGTAGCTGTAAAGATAACAGAGCATTCCTGGAATGCTGATCTTTATAAGCTCATGGGACACTTCGCAT
GGTGGACAGCTTTTGTTACTAATGTTAATGCTTCATCTTCAGAGGCTTTCTTAATTGGTTGTAATTATCT
TGGCAAACCACGTGAGCAGATAGATGGTTATGTCATGCATGCAAATTACATATTTTGGAGGAACACAAAT
CCAATACAATTGTCTTCCTACTCATTATTTGACATGAGTAAGTTTCCTCTTAAATTAAGAGGTACTGCTG
TTATGTCATTAAAAGATGGACAAATCAATGATATGATTTTGTCTCTTCTTAGTAAAGGCAGACTTATTGT
TAGAGAGAATAATAGAGTTATTATCTCTAGTGATGTTCTTGTTAACAACTAAACGAACATGTTGTTTTTC
TTGTTTCTTCAGTTCGCCTTAGTAAACTCCCAGTGTGTTAACTTGACAGGCAGAACCCCACTCAATCCCA
ATTATACTAATTCTTCACAAAGAGGTGTTTATTACCCTGACACAATTTATAGATCAGACACACTTGTGCT
CAGCCAGGGTTATTTTCTTCCATTTTATTCTAATGTTAGCTGGTATTACTCATTAACAACCAACAATGCT
GCCACAAAGAGGACTGATAATCCTATATTAGATTTCAAGGACGGCATATACTTTGCTGCCACTGAACACT
CAAATATTATCAGGGGCTGGATCTTTGGAACAACTCTTGACAACACTTCTCAATCTCTCTTGATAGTTAA
CAACGCAACGAATGTTATTATCAAGGTTTGTAATTTTGATTTTTGTTATGATCCCTACCTTAGTGGTTAC
TATCATAACAACAAAACATGGAGCATCAGAGAATTTGCTGTCTATTCTTCTTATGCTAATTGTACTTTTG
AGTATGTTTCGAAATCCTTTATGTTGAACATTTCTGGTAATGGTGGTCTGTTCAACACTCTTAGAGAGTT
TGTTTTCAGAAATGTCGATGGGCATTTCAAGATTTACTCAAAGTTTACACCAGTAAATTTAAATCGTGGC
TTGCCTACTGGTCTCTCAGTGCTTCAGCCATTGGTTGAATTACCAGTTAGCATAAATATTACTAAATTCA
GAACACTCCTCACTATTCATAGAGGAGACCCTATGCCTAATAACGGCTGGACTGCTTTTTCAGCTGCTTA
TTTCGTGGGCTATCTTAAACCACGTACCTTTATGCTGAAATATAATGAGAATGGCACCATTACTGATGCT
GTTGATTGTGCACTTGACCCTCTTTCGGAGACAAAGTGTACGTTAAAATCTCTTACTGTCCAAAAGGGCA
TCTATCAGACTTCTAACTTCCGAGTGCAACCCACTCAGTCTGTAGTTAGATTTCCTAATATTACCAATGT
GTGTCCATTTCACAAGGTTTTTAATGCCACGAGGTTTCCTTCCGTCTATGCGTGGGAAAGAACTAAAATT
TCTGATTGCATTGCAGATTACACTGTTTTCTACAATTCAACTTCTTTTTCTACTTTTAAATGTTATGGTG
TTTCACCTTCTAAATTGATTGATTTGTGCTTTACGAGTGTGTATGCTGATACATTTCTCATAAGATTCTC
AGAAGTCAGACAGGTGGCACCAGGACAAACTGGTGTCATTGCTGACTATAATTATAAATTACCTGATGAT
TTTACAGGTTGTGTCATAGCTTGGAACACTGCCAAACAGGATGTAGGTAATTATTTCTACAGGTCTCATC
GTTCTACCAAATTGAAACCATTTGAAAGAGATCTTTCCTCAGACGAGAATGGTGTCCGTACACTTAGTAC
TTATGACTTCAACCCTAATGTACCACTTGAATACCAAGCTACAAGGGTTGTTGTTTTGTCATTTGAGCTT
CTAAATGCACCAGCTACAGTTTGTGGACCAAAACTATCCACACAACTAGTAAAAAATCAGTGCGTTAATT
TCAACTTTAACGGACTCAAGGGCACTGGTGTCTTGACTGATTCTTCCAAGAGGTTTCAGTCATTCCAACA
ATTTGGTAAAGATGCGTCTGACTTTATTGATTCAGTACGTGATCCTCAAACACTTGAGATACTTGACATT
ACACCTTGCTCTTTTGGTGGTGTCAGTGTTATAACACCAGGAACAAACACTTCTTTAGAGGTGGCTGTTC
TTTACCAAGATGTTAACTGCACTGATGTACCAACTACTATACATGCAGACCAACTAACACCTGCTTGGCG
TATTTATGCTACTGGCACTAATGTGTTTCAAACTCAAGCAGGCTGTCTTATAGGAGCTGAACATGTCAAT
GCTTCTTATGAGTGTGACATCCCAATTGGTGCTGGTATTTGTGCTAGCTACCATACGGCTTCTATATTAC
GCAGTACAAGCCAGAAAGCTATTGTGGCTTATACTATGTCCCTTGGTGCTGAGAACTCTATCGCTTATGC
TAACAATTCTATAGCCATACCTACAAATTTTTCAATTAGTGTTACCACTGAAGTTATGCCTGTATCAATG
GCTAAAACTTCTGTAGATTGTACTATGTATATCTGTGGTGACTCTATAGAGTGTAGCAACTTGTTGTTAC
AATATGGCAGTTTTTGCACACAACTAAATCGTGCTTTAAGTGGGATTGCTATTGAGCAAGACAAGAACAC
CCAAGAGGTTTTTGCTCAAGTTAAGCAAATCTATAAAACACCACCTATTAAGGATTTTGGTGGTTTTAAT
TTTTCACAGATACTACCTGACCCATCTAAACCCAGCAAGAGGTCGTTTATTGAAGACTTACTCTTCAATA
AAGTCACTCTTGCTGATGCCGGTTTTATCAAACAGTACGGTGATTGTTTGGGTGGTATTTCTGCTAGAGA
TTTGATTTGTGCTCAAAAGTTCAATGGACTTACTGTCTTACCACCATTGCTCACAGATGAAATGATCGCT
GCTTATACAGCTGCATTAATTAGCGGCACTGCCACTGCTGGATGGACCTTTGGTGCTGGTGCTGCTCTTC
AAATACCATTTGCCATGCAAATGGCTTATAGGTTTAATGGAATTGGAGTTACTCAGAATGTTCTCTATGA
GAATCAGAAATTAATAGCCAATCAGTTTAATAGTGCTATTGGAAAAATCCAAGAGTCTTTGACATCTACA
GCTAGTGCACTTGGAAAATTGCAGGATGTTGTTAACCAAAATGCACAAGCTTTAAACACGCTTGTTAAAC
AACTTAGTTCCAATTTTGGTGCAATTTCAAGCGTGTTGAATGACATTCTTTCACGCCTTGACAAAGTCGA
GGCTGAGGTTCAGATTGATAGGTTGATCACAGGTAGACTTCAGAGTTTACAGACGTATGTGACTCAACAA
TTAATCAGAGCTGCAGAAATCAGAGCTTCTGCTAATCTTGCTGCGACTAAAATGTCCGAGTGTGTACTAG

GACAATCTAAAAGAGTTGATTTTTGTGGAAAAGGTTATCACCTAATGTCTTTTCCCCAGTCAGCGCCTCA
TGGTGTTGTCTTCTTACATGTGACTTACATTCCTTCGCAAGAAAAGAACTTCACAACAGCTCCTGCCATT
TGCCATGAAGGTAAAGCACACTTCCCACGTGAAGGTGTTTTCGTTTCGAATGGCACACACTGGTTTGTAA
CACAAAGGAACTTTTATGAACCTAAAATTATAACCACTGACAATACATTTGTCTCTGGTAACTGTGATGT
TGTAATTGGAATTATCAACAACACAGTTTATGATCCTTTACAACCAGAACTTGATTCATTTAAGGAGGAG
TTAGATAAATATTTTAAAAATCATACATCACCTGATATTGATCTTGGTGATATTTCTGGCATTAATGCTT
CTGTTGTCAATATTCAAAAGGAAATTGACCGCCTCAATGAGGTTGCCAGAAATTTAAATGAATCACTCAT
TGATCTCCAAGAACTTGGAAAATATGAGCAATATATCAAATGGCCATGGTATGTTTGGCTCGGCTTCATT
GCTGGACTCATTGCTATAGTCATGGTTACAATCCTGCTTTGTTGCATGACAAGTTGTTGCAGTTGTCTCA
AGGGCTGTTGTTCTTGCGGATCTTGCTGTAAATTTGATGAAGACGACTCTGAGCCTGTGCTCAAAGGAGT
CAAATTACATTACACATAAACGAACTTATGGATTTGTTTATGAGAATTTTCACTCTTGGAACTGTTACTC
TTAAACAAGGTGAAATCAAAGGTGCTACTCCTACAAATTCTGTTCGCACTACTGCAACAATACCGATACA
AGCCACACTCCCTTTCGGATGGCTTGTTGTTGGCGTTGCAATTCTTGCTGTTTTTCAAAGCGCTTCAAAA
ATAATTACACTCAAAAAGAGATGGCAGTTAGCCCTCTCTAAAGGTGTTCATTTTGTTTGCAACTTGCTTC
TGCTGTTTTTAACAGTTTATTCTCACTTGTTGCTTCTTGCTGGTGGCTTGGAAGCCACTTTTCTCTTTCT
TTATGCATTAGCTTATTGCTTGCAAACTGTAAATTTTGTGAGAATAATAATGCGATTCTGGTTGTGCTGG
AAGTGCCGTTCCAAGAATCCTTTACTCTATGATGCCAACTACTTTCTTTGTTGGCATACTAATTGTTATG
ACTATTGTATACCATACAATAGTGTAACCTCTTCAATTGTCATCACATGTGGTGATGGTACTACGAATCC
CATTTCTGAGGACGACTACCAAATTGGTGGTTACACGGAAAAGTGGGAGTCTGGTGTTAAGGACTGTGTT
GTATTACATAGTTATTTCACCTCAGATTACTACCAGCTGTACTCAACACAAGTGAGTACAGACACTGGTG
TTGAACATGTTACTTTCTTCATCTACAATAAAATTGTTGATGAGCCTGAAGAACATGTTCAAATTCACAC
AATCGACGGTACATCTGGAGTTGTTAATCCAGCAATGGAACCAATTTATGATGAACCGACGACGACTACT
AGCGTGCCTTTGTAAGCACAAGCTGATGAGTACGAACTTATGTACTCATTCGTTTCGGAAGAGACAGGTA
CGTTAATAGTTAATAGCGTACTTCTTTTTCTTGCTTTTGTGGTATTCTTGCTAGTCACACTAGCCATCCT
TACTGCGCTTCGATTGTGTGCGTACTGCTGCAATATTGTTAACGTGAGTCTTGTAAAACCTTCTTTTTAC

**Table 8. Primers used to obtain above sequence**

| Primer name | Sequence 5' → 3' | SEQ ID NO. |
|---|---|---|
| E_Sarbecto_F1 | ACAGGTACGTTAATAGTTAA-TAGCGT | 1 |
| E_Sarbeco_R2 | ATATTGCAGCAGTACGCACACA | 2 |
| E_Sarbeco_P1 | FAM-ACAC-TAGCCATCCTTACTGCGCTTCG-BBQ | 60 |

SEQ ID NO. 54

GTTTACTCTCGTGTTAAAAATCTGAATTCTTCTAGAGTTCCTGATCTTTTGGTCTAAACGAACTAAATAT
TATATTAGTCTTTCTGTTTGGAACTTTAATTTTAGCCATGTCAGGTGACAACGGTACCATTACCGTTGAA
GAGCTTAAAAAGCTCTTAGAACAATGGAACCTAGTAATAGGATTCTTGTTTCTTACATGGATTTGTTTGT
TACAATTTGCCTATGCCAACAGGAATAGGTTTTTGTACATAATTAAGTTAATTTTCCTCTGGCTGCTTTG
GCCAGTAACTTTAGCTTGCTTTGTGCTTGCTGCTGTTTACAGAATAAACTGGATCACTGGTGGAATTGCC
ATTGCAATGGCCTGTCTTGTAGGCTTGATGTGGCTTAGCTACTTCATTGCTTCTTTCAGGCTGTTTGCTC
GTACGCGTTCCATGTGGTCATTTAACCCAGAAACTAACATTCTTTTGAACGTGCCTCTTCATGGCACAAT
TCTGACCAGGCCGCTTCTAGAGAGTGAACTCGTAATTGGAGCTGTGATCCTTCGTGGACATCTTCGTATT
GCAGGACACCATCTGGGACGCTGTGACATCAAGGACCTGCCCAAAGAAATCACTGTAGCTACATCACGAA
CGCTTTCTTATTACAAATTGGGAGCTTCGCAGCGTGTAGCAGGTGACTCAGGTTTTGCTGCATACAGTCG
CTACAGGATTGGTAATTACAAATTAAATACAGACCATTCCAGTAGCAGTGACAATATTGCTTTGCTTGTA
CAGTAAGTGACAACAGATGTTTCATCTCGTTGACTTTCAGGTTACTATAGCAGAGATATTATTAATTATT
ATGAGGACTTTTAAAGTTTCCATTTGGAATCTTGATTATATCATAAATCTCATAATTAAAAATCTATCTA
AGCCTCCAACTGAGAATAACTGTTCTCAATTAGATGAAGAGCAACCAATGGAGATTGATTAAACTAACAT
GAAAATTATTTTCTTCTTGGTACTGATAACACTTGTTACTGGCGAGCTTTACCACTACCAAGAGTGTATA

AAAGGTACAACTGTACTTTTAAAAGAACCTTGCTCTTCAGGAACATATGAAGGCAATTCACCATTTCATC
CTCTAGCTGATAATAAATTTGCACTGGCTTGCTTTAGCACTCAATTTGCTTTTGCTTGTCCTGACGGTGT
TAGACACACCTTTCAGTTACGTGCGAGATCAGTTTCACCCAAACTGTTTACCAGACAAGAGGAAGTTCAA
GAATTATACTCACCTGTTTTCCTTATCGTTGCAGCTATAGTGTTCATAATACTTTGCTTCACATTCAAAA
GAAAAATAGAATGAGTGAATTTTCATTAATTGACTTCTATTTGTGCTTCTTAGCCTTTCTGCTATTCCTT
GTTTTAATTATGCTCATTATCTTTTGGTTCTCACTAGAACTGCAAGATCATAATGAAACTTTCCACGCCT
AAACGAACATGAAATTTCTTGTTTTCTTAGGAATTCTTACAACAGTAGCTGCATTCCATCAGGAATGTAG
TTTACAGTCATGTGCTCAGCATCAACCCTATGTAGTTGATGACCCTTGTCCAATTCACTTCTACTCACGA
TGGTATATCAGAGTGGGAGCTAGAAAATCAGCACCTTTGATTGAATTGTGTGTTGATGAGGTAGGCTCTA
AGTCACCCATTCAATACATTGACATTGGTAATTACACAGTTTCCTGTTCTCCTTTTACAATTAATTGCCA
GGAACCTAAATTAGGTAGTCTCGTAGTACGGTGTTCGTATTATGAAGACTTTCTAGAGTACCATGACATT
CGTGTTGTCTTAGATTTCATCTAAACGAACTAACTAAAATGTCTGATAATGGACCCCAAAACCAACGTAG
TGCACCCCGCATTACATTTGGTGGACCCTCAGATTCAAGTGACAATAGCAAAAACGGAGAGCGCAATGGT
GCACGACCTAAACAACGTCGACCCCAAGGCTTACCCAATAATACTGCATCTTGGTTCACCGCTCTCACTC
AACATGGCAAGGAAAACCTTACGTTCCCTCGAGGGCAAGGTGTTCCAATCAACACCAATAGCTCTAAAGA
TGACCAAATTGGCTACTACCGTAGAGCTACCAGACGAATTCGTGGTGGTGACGGTAAAATGAAAGAGCTC
AGCCCCAGATGGTATTTTTACTATCTAGGAACTGGACCAGAAGCTGGACTTCCCTATGGTGCTAACAAAG
AAGGCATCATATGGGTTGCAACTGAGGGAGCCTTAAACACACCGAAAGACCACATTGGCACCCGCAATCC
TGCTAACAATGCTGCAATCGTGCTACAACTTCCTCAAGGAACAACATTGCCAAAAGGCTTCTACGCAGAA
GGGAGCAGAGGCGGCAGTCAAGCTTCTTCACGCTCCTCATCACGTAGTCGCAACAGTTCAAGAAACTCAA
CTCCAGGCAGCAGTAGGGGAACTTCTCCTGCTAGAATGGCTGGCAATGGCGGTGACACTGCTCTTGCTTT
GCTGCTGCTAGATAGGTTGAACCAGCTTGAGAACAAAGTATCTGGCAAAGGCCAACAACAACAGGGCCAA
ACTGTCACTAAGAAATCTGCTGCTGAGGCATCTAAAAAGCCTCGCCAAAAACGTACTGCTACAAAACAGT
ACAACGTCACTCAAGCATTTGGGAGACGTGGTCCAGAACAAACCCAAGGAAATTTTGGGGACCAAGAATT
AATCAGACAAGGAACTGATTACAAACATTGGCCGCAAATTGCACAATTTGCTCCAAGTGCCTCTGCATTC
TTTGGAATGTCACGCATTGGCATGGAAGTCACACCTTCGGGAACATGGCTGACTTATCATGGAGCCATTA

**Table 9. Primers used to obtain above sequence**

| Primer name | Sequence 5' → ' | SEQ ID NO. |
|---|---|---|
| 2019-nCoV_N2-F | TTA CAA ACA TTG GCC GCA AA | 9 |
| 2019-nCoV_N2-R | GCG CGA CAT TCC GAA GAA | 10 |
| 2019-nCoV_N2-P | FAM-ACA ATT TGC CCC CAG CGC TTC AG-BHQ1 | 61 |
| 2019-nCoV_N3-F | GGG AGC CTT GAA TAC ACC AAA A | 11 |
| 2019-nCoV_N3-R | TGT AGC ACG ATT GCA GCA TTG | 12 |
| 2019-nCoV_N3-P | FAM-AYC ACA TTG GCA CCC GCA ATC CTG-BHQ1 | 62 |

SEQ ID NO. 55

AATTGGATGACAAAGATCCACAATTCAAAGATAACGTCATACTGCTGAATAAGCACATTGACGCATACAA
AACATTCCCACCAACAGAGCCTAAAAAGGACAAAAAGAAAAAGGCTGATGAACTTCAGGCTTTACCGCAG
AGACAGAAGAAACAACAAACTGTGACCCTTCTTCCTGCTGCAGATTTGGATGAATTCTCCAAACAGTTGC
AACAATCCATGAGTGGTACTGATTCAACCCAGGCTTAAACTCGTGCAGACCACACAAGGCAGATGGGCTA
TATAAACGTTTTCGCTTTTCCGTTTACGATATATAGTCTACTCTTGTGCAGAATGAATTCTCGTAACTAC
ATAGCACAAGTAGATGTAGTTAACTTTAATTTCACATAGCAATCTTTAATCAATGTGTAACATTGGGGAG
GACTTGAAAGAGCCACCACGTTTTCACCGAGGCCACGCGGAGTACGATCGAGGGTACAGCCAATAATGTT
AGGGAGAGCAGCCTATATGGAAGAGCCCTAATGTGTAAAATTAATTTTAGTAGTGCTATCCCCATGTGAT
TTTAATAGCTTCAACCACTCGACAAGAAAAAAAAAAAAAAAAAAAAAAAAAAA

### Example 9

### RAVENC2 a high-throughput solution for COVID-19 Screening on Illumina Systems

A direct to citizen virus screening workflow for rapid detection of infected individuals

Problem: The novel coronavirus has created a pandemic (COVID-19). A safe release of a country lockdown requires fast and reliable identification of all, including *asymptomatic* individuals combined with rapid communication of results.

The solution: the RavenC2 System presents a complete end-to-end and direct to consumer workflow to perform large scale SARS-CoV-2 testing - using pre-barcoded sample collection vials, RNA extraction-free and 1-step targeted library preparation with high throughput Next Generation Sequencing, combined with rapid data analysis and integration with a smartphone app to securely manage the data flow between healthcare providers and citizens.

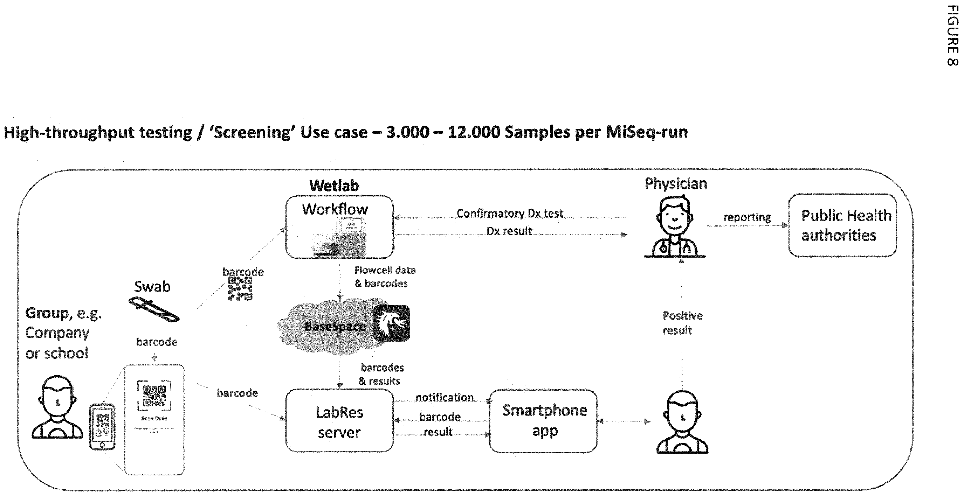

A novel coronavirus has created a pandemic (COVID-19). Until the availability of a safe vaccination opening of our society and economy requires regular testing of *asymptomatic* individuals combined with rapid communication of results. This makes high-throughput testing key to monitoring the outbreak to prevent future waves of the pandemic. For fast and reliable large-scale SARS-CoV-2 testing, a complete end to end workflow was established clinical laboratory using pre-barcoded swap devices that are simple and quick registered by the citizen via a smart phone app at sample collection. Samples are quickly transformed using an RNA extraction free, 1-setp targeted RT-PCR approach. The use of separate target-specific primer sets for 3 viral targets and 1 internal human control genes, in combination with indexing primer sets allows modular combination of molecular barcode (index) kits to ensure scalability from several samples to 3000-12000 samples per sequencing run. Libraries are compatible with all Illumina platforms (tested: MiSeq, HiSeq2500, NovaSeq) and can be directly uploaded and analyzed in Basepace or using a local Dragen Server. Result data are directly communicated via API interface and reported to the citizen via smartphone app (see **Figure 8**). The proposed solution is flexible, it can be applied to both settings to address the needs of healthcare systems and public health programs.

### Experimental methods

To demonstrate the sensitivity and specificity of the sequencing workflow 400 test samples, full virus control controls and Virus target PCR amplicons are interrogated on the MiSeq^{™} System. The full RAVENC2 workflow is tested in a pilot experiment on multitude of individuals.

### Sample collection and registry

Single swabs of citizens are collected in pre-barcoded collection vials and the test and registers in the app via scanning the vial barcode. Once collected samples have reached the laboratory and registered by their barcode via LIMS, they are immediately introduced into the laboratory process.

### Sample extraction, library preparation

The swab samples are transferred in a lysis buffer and forwarded to an automated RNA extraction workflow. In a 1-step approach target-specific cDNA is generated with SARS-CoV-2 specific virus markers and enriched in each sample (targeted RT-PCR). A human control amplicon is used as process control.

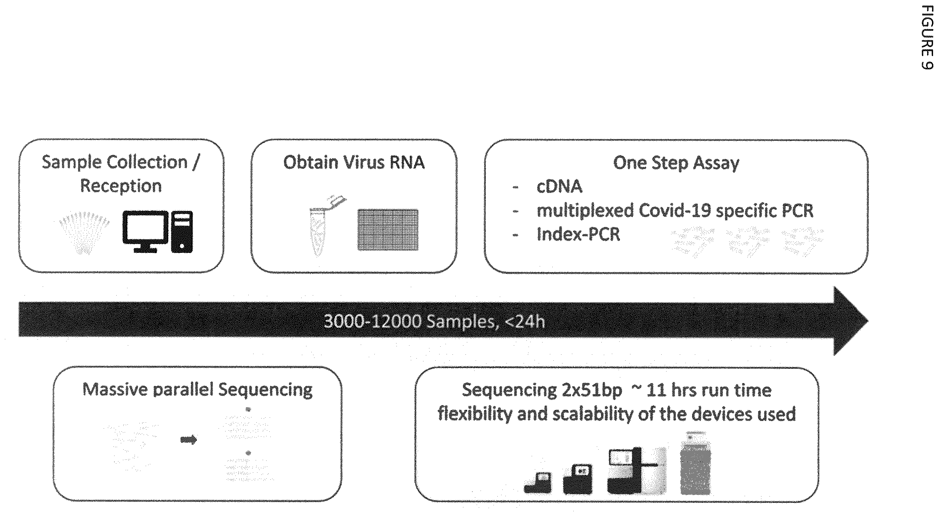

Sequencing barcodes (indices) are introduced simultaneously during the target PCR reaction. Barcodes that identify the individual samples, consist of a unique combination of two 8-nucleotides indices. This enables the parallel analysis of around 3.000 samples per sequencing pool (see **Figure 9**).

### Sequencing and Data Analysis

Library pools are sequenced on a MiSeq using 2 x 51 bp read length with default settings. The solution is scalable and compatible with all supported Illumina Sequencers. Data are either directly uploaded to Illumina Basespace Sequencing Hub and/or stored locally. The RAVENC2 panel includes oligo probes targeting viral sequences and also human genome positive control probes, in the event a sample does not contain any viral target. Using DRAGEN locally or in Basespace, all sequencing data from a run is demultiplexed into FASTQ files for each individual sample. The paired-end reads are then aligned to a reference sequence containing the entire SARS-CoV-2 genome plus human control amplicon sequences. After filtering low-quality reads and alignments purely matching to primer sequences (potential PCR artifacts) DRAGEN counts the number of reads aligning specifically to each amplicon. Sample-level QC, to establish that sample collection and the PCR worked as expected, is done using the counts of reads to the human control RNA (e.g. RPP). On samples passing QC, Virus detection is then performed next by aggregating read counts from all viral amplicons, compared to thresholds established from calibration studies. The DRAGEN system uses an FPGA card for hardware acceleration, enabling processing an entire sequencing run with thousands of samples in under one hour (MiSeq or NextSeq). The overall processing of an entire sequencing run with thousands of samples is completed in under one hour. APIs are defined to transmit results to the laboratory information system as well as the RAVENC2 server communicating with the smartphone app.

### Direct result reporting via Smartphone App

The Raven App provides a decentralized and secure digital solution for direct return of Covid-19 results to individuals tested on the high-throughput RavenC2 laboratory platform.

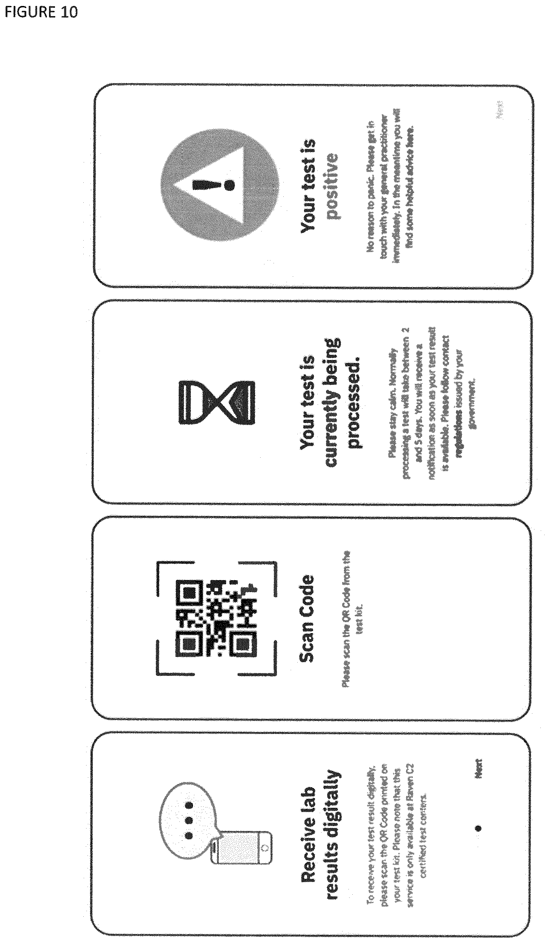

The goal of the RAVENC2 application and infrastructure is to digitally transmit Covid-19 test results directly from the laboratory to each individual at scale, while minimizing the processing of personal data. Individuals using the RAVENC2 Application will be able to scan the unique barcode printed on each test kit, which is later entered into the Laboratory Information Management System (LIMS). Once sequenced, each result can then be transmitted back to the individual based on a unique identifier. The use of temporary access tokens ensures that only users who have scanned the code on a test kit can access the test result assigned to that test kit (see **Figure 10**).

### Validation with control samples

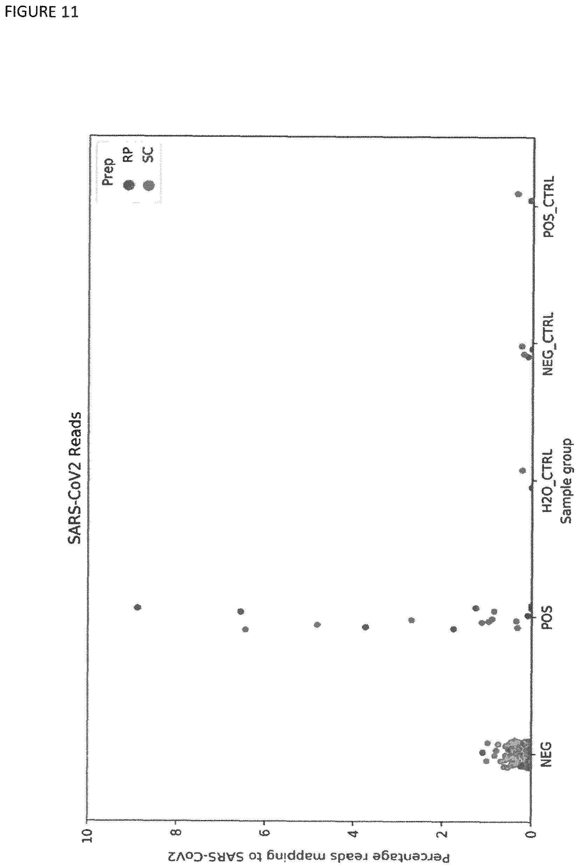

Initial validation of the RAVENC2 Panel is performed with a set of 96 test samples which were pretested via qPCR, one commercially available virus sample and viral and human control PCR amplicons as targets. After library preparation and sequencing, resulting data is aligned and read counts from all viral amplicons are aggregated, compared to established thresholds from calibration studies (see **Figure 11**).

### Summary

Implementing this workflow all around the EU or even worldwide is assumed to multiply the amount of tests that could be performed on a daily basis. Since modern sequencing platforms are able to produce terabytes of data and the virus amplicons only need a few Megabytes, the number of concurrently analysed samples is mostly limited by the number of unique barcodes. Redesigning those to a length of 10 or 12 bp will greatly increase the capacity of each sequencing run to > 1.000.000/run.

With the RavenC2 approach we can see that testing of 3000 samples in a day on a single instrument is highly achievable. This can further be scaled via a number of axes:

Multiple instruments, since there are thousands of sequencing instruments globally.

Higher capacity instruments (newer sequencing instruments produce terabases of data and the virus amplicons only need a few mega bases). Theoretically a single run on the latest sequencing instruments could handle millions of samples in a single day however the ability to individually identify each sample would need some significant advances.

Increasing the barcode length and/or availability of unique dual indexing (both will enable more samples to be combined on a single run and help increase the number of samples per day up). Using our current configuration increasing the barcode complexity will enable at least approximately 12,000 sample in a day per instrument.

### Example 10

### NGS screening pilot project with >1500 samples

### Sample collection

Single pharyngeal swabs of 1571 citizens were collected in pre-barcoded collection vials (Fisher Scientific), prefilled with 800 µl lysis buffer (Macherey Nagel, Roche). Samples were transferred into the laboratory process within 2-48 hrs. of collection

The resuspension took place during transportation and no additional resuspension was necessary

### RNA extraction

200 µl of the swab lysis resuspension were transferred by Hamilton Chemagic Liquid handling System (Hamilton), from the barcoded vials into barcoded 96 deep well extraction plates.

RNA extraction was performed on a MagNA Pure 96 System, using the MagNA Pure 96 DNA and Viral NA Kit (Roche Diagnostics) according to instructor's guide, input volume was 200 µl and the elution volume was set to 100 µl.

### Library preparation

A one-step target enrichment approach was used to generate Illumina Libraries combining cDNA synthesis, target enrichment, sample indexing and sequence adaptor ligation in a single reaction.

Three separate target-specific primer sets for viral targets were used and one internal human target gene as process control. According the CDC Primerset for Covid19 with a mean target amplicon size of 72 bp. In combination with the gene-specific primer sets a sample specific indexing primer set was added containing a dual molecular barcode sequence and the "Nextera"-adapter sequence-overhang, allowing to combine several samples (up to 3000) in one sequencing run.

RT-PCR reaction was prepared in a total volume of 12.5 µl on a Hamilton M Liquid handling System using One Step PrimeScript^{™} III RT-qPCR Mix, with UNG (Takara RR601B) in 384 well plates and either 1.5 µl RNA extract from the test samples.

To every 384 well reaction plate 1 µl of a dilution series with known standard virus copies starting from 200 copies/µl to 3 copies/µl either a no template control was included. (EDX SARS-CoV-2 Standard BioRad). Five known positive samples has been included in the workflow. The standard dilution series is used to define a sequencing run specific cut-off and to distinguish positives from negative samples. RT-PCR was carried out on ProFlex^{™}2 x 384-Well-PCR-Systems (ThermoFisher).

2µL of every RT-PCR well was combined into a single sequencing library pool. Library clean-up was performed with QIAquick PCR Purification Kit according to the instructions in the manual (Qiagen).

The following ingredients and conditions were used:

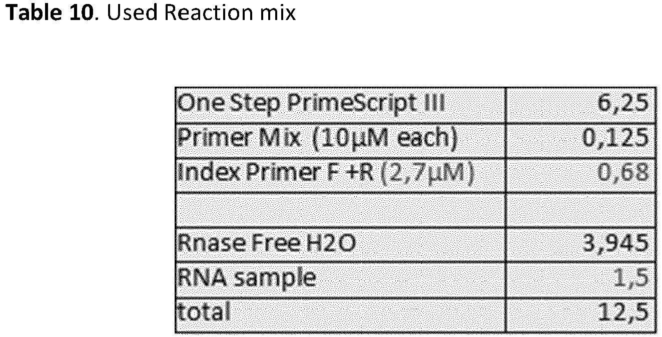

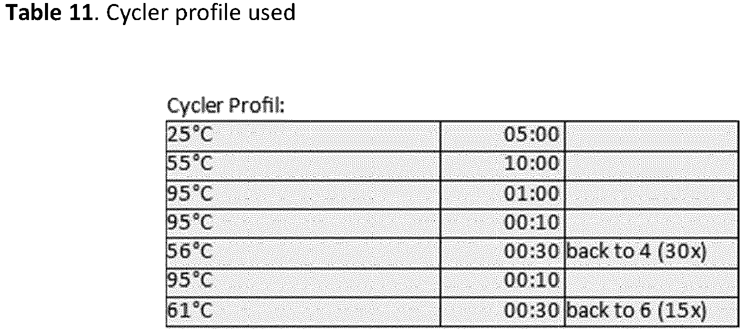

**Table 12. Primers used**

| **Primer name** | **Sequence 5' → 3'** | **SEQ ID NO** |
|---|---|---|
| Nextera-Transposase + CTG modification | | |
| 2019-nCoV_N1.F | TCGTCGGCAGCGTCAGATGTG- TATAAGAGACAGCTGGAC- CCCAAAATCAGCGAAAT | 16 |
| 2019-nCoV_N2.F | TCGTCGGCAGCGTCAGATGTG- TATAAGAGACAGCTGTTA- CAAACATTGGCCGCAAA | 17 |
| 2019-nCoV_N3.F | TCGTCGGCAGCGTCAGATGTG- TATAAGAGACAGCTGGGGAGCCTT- GAATACACCAAAA | 18 |
| RP-F.F | TCGTCGGCAGCGTCAGATGTG- TATAAGAGACAGCTGAGATTTGGAC- CTGCGAGCG | 19 |
| 2019-nCoV_N1.R | GTCTCGTGGGCTCGGAGATGTG- TATAAGAGACAGCCGTCTGGTTA- CTGCCAGTTGAATCTG | 22 |
| 2019-nCoV_N2.R | GTCTCGTGGGCTCGGAGATGTG- TATAAGAGACAGCCGGCGCGACATT- CCGAAGAA | 23 |
| 2019-nCoV_N3.R | GTCTCGTGGGCTCGGAGATGTG- TATAAGAGACAGCCGTG- TAGCACGATTGCAGCATTG | 24 |
| RP.R | GTCTCGTGGGCTCGGAGATGTG- TATAAGAGACAGCCG- GAGCGGCTGTCTCCACAAGT | 25 |

### Library preparation

2µL of every RT-PCR well was combined into a single sequencing library pool. Library clean-up was performed with QIAquick PCR Purification Kit according to the instructions in the manual (Qiagen).

Library pools were sequenced on a MiSeq and on a NovaSeq6000 System using 2 x 51 bp read length with default settings.

Data were stored locally and were uploaded to Illumina Analytics Platform. Using DRAGEN, all sequencing data from a run were de-multiplexed into FASTQ files for each individual sample. The paired-end reads are then aligned to a reference sequence containing the entire SARS-CoV-2 genome plus human control amplicon sequences. After filtering low-quality reads and alignments purely matching to primer sequences (potential PCR artefacts) DRAGEN counts the number of reads aligning specifically to each amplicon. Sample-level QC, to establish that sample collection and the PCR worked as expected, is done using the counts of reads to the human control RNA (e.g. RPP). On samples passing QC, Virus detection was then performed by aggregating read counts from all viral amplicons, compared to the internal human control and evaluated according to thresholds established from calibration studies.

### Realtime RT-PCR

Positive Samples and invalidated samples from the NGS Screening assay were confirmed by Realtime RT-PCR using the ampliCube Coronavirus SARS-CoV-2 (Mikrogen GmbH) assay following the manufacturer's recommendation. The assay was carried out on a BioRad CFX 96 RT-PCR System.

### Results

Following primary data analysis, the read counts of all samples, internal standards, NTC and positive controls for nCoV2, nCov3 and RPP30 were determined
Of the 1571 analyzed samples, two samples and one positive control had to be excluded from the analysis due to a laboratory error.

The mean value and the standard deviation were determined for the control gene RPP30 from all samples. All samples that had fewer reads than the mean minus the standard deviation were considered as not evaluable. 21 samples could not be evaluated and have been reported as invalid (1.3%).

To normalize and determine a comparison value, the counts for nCov2 and nCov3 were added and divided by the counts for RPP30. (counts (nCoV2 + nCoV3) / RPP30). The counts for nCoV1 were excluded from the analysis due to too many unspecific reads that were generated from that amplicon. This normalized comparison value was compared with the values of the dilution series of the reference material.

All samples that achieved a value higher than standard 2 (corresponding to 100 copies / µl) were evaluated as positive. 4 samples showed higher read counts than the standard 2. These samples were the positive controls.

When the value of the lowest standard 7 was used as cutoff (3 copies / µl), 66 samples had more reads than the lowest standard (4.2% false positive reads). All of these samples and all samples that were classified as not evaluable due to insufficient RPP30 values were additionally analyzed by real-time PCR. No sample was confirmed as positive in real-time PCR.

The pilot project has shown that the NGS screening approach according to the invention enables reliable detection of SARS CoV2 Infections. In particular, the approach enables the screening of a very large number of samples simultaneously in a short time.

## Claims

1. A method for preparing an RNA sample for a target specific next generation sequencing comprising performing a one-step target enrichment in a single reaction vessel or in a single reaction mixture, wherein said enrichment comprises the steps:
(i) exposing the RNA to be sequenced in a single reaction vessel to a mixture comprising a reverse transcriptase, a DNA polymerase, and
(a) one or more target-specific reverse primers, suitable for the preparation of a target specific cDNA; and
(b) a forward target specific primer comprising a first forward adaptor sequence, and a reverse target specific primer comprising a first reverse adaptor sequence, and
(c) a forward indexing primer comprising a first forward adaptor sequence, a forward indexing sequence and a second forward adaptor sequence; and a reverse indexing primer, comprising a first reverse adaptor sequence, a reverse indexing sequence, wherein the reverse indexing sequence is different from the forward indexing sequence, and a second reverse adaptor sequence;
and
desoxyribonucleoside triphosphates (dNTPs); and
(ii) subjecting the reaction mixture of (i) to a series of temperature changes under conditions sufficient to yield a first strand cDNA copy of at least a portion of the RNA to be sequenced, preferably a gene sequence, and subsequently a target specific amplicon comprising starting from the 5'- to the 3'-end a second forward adaptor sequence, a forward indexing sequence, a first forward adaptor sequence, a forward target specific primer sequence, target sequence, a reverse target specific primer sequence, a first reverse adaptor sequence, a reverse indexing sequence and a second reverse adaptor sequence.

2. The method of claim 1, additionally comprising as first step the extraction of RNA from a sample obtained from a subject, preferably by sample lysis.

3. The method of claim 1 or 2, wherein the method additionally comprises a control amplification of one or more additional target sequences.

4. The method of any one of claims 1 to 3, additionally comprising a step of sample registration, which is performed previous to the enrichment.

5. The method of any one of claims 1 to 4, wherein said method further comprises a purification of the amplicon as obtained in step (ii) and/or step of quantifying the amplicon.

6. The method of any one of claims 1 to 5, additionally comprising a step of sequencing the amplicon as obtained in step (ii), preferably with a NGS system such as Illumina, Ion Torrent, Oxford Nanopore, or SMRT Sequencing, optionally further comprising assembling sequence reads.

7. A kit for preparing an RNA sample for next generation sequencing in a one-step target enrichment comprising: a) a reverse transcriptase (RT); b) one or more target-specific reverse primers, suitable for the preparation of a target specific cDNA, c) a forward target specific primer comprising a first forward adaptor sequence, and a reverse target specific primer comprising a first reverse adaptor sequence; d) a forward indexing primer comprising a first forward adaptor sequence, a forward indexing sequence and a second forward adaptor sequence; and a reverse indexing primer, comprising a first reverse adaptor sequence, a reverse indexing sequence, wherein the reverse indexing sequence is different from the forward indexing sequence, and a second reverse adaptor sequence; e) desoxyribonucleoside triphosphates (dNTPs); and f) a DNA polymerase.

8. The method of any one of claims 1 to 6, or the kit of claim 7, wherein said adaptor sequence has a length of about 8 to 45 nucleotides; and/or wherein said indexing primer sequence has a length of about 4 to 20 nucleotides.

9. The method of any one of claims 1 to 6, or 8, or the kit of claim 7 to 8, wherein said adaptor sequence is capable of binding to a substrate, preferably a sequence chip or flow cell.

10. The method of any one of claims 1 to 6, or 8 to 9, or the kit of any one of claims 7 to 9, wherein said target-specific primer or said target-specific primer pair is specific for a target sequence, wherein said target sequence is preferably a viral gene or a part of a viral genome, a leukocyte antigen-associated gene, or a blood group antigen associated gene; more preferably wherein said target sequence is a viral gene of a coronavirus, preferably a SARS-CoV-2 virus gene or genomic portion, or a part of it, more preferably the 5'UTR, 3'UTR, ORF1ab, Orf3a, Orf6, Orf7a, Orf7b, Orf8, Orf10, M gene region, E gene region, N gene region, or S gene region of SARS-CoV-2.

11. The method of any one of claims 1 to 6, or 8 to 10, or the kit of any one of claims 7 to 10, wherein said target sequence comprises one or more of the following nucleotide positions according to the nucleotide numbering of the reference genome of SARS-CoV-2 (reference genome with NCBI Reference Sequence No: NC_045512.2; SEQ ID NO: 63): 100, 733, 1264 , 2749, 3267, 3828, 5388, 5648, 6319, 6573, 6613, 6954, 7600, 7851, 10667, 11078, 11288-11296, 11824, 12964, 12778, 13860, 17259, 19602, 19656, 21614, 21621, 21638, 21765-21770, 21974, 21991-21993, 22132, 22812, 23012, 23063, 23271, 23525, 23604, 23709, 24506, 24642, 24914, 26149, 27853, 27972, 28048, 28111, 28167, 28253, 28262, 28280, 28512, 28628, 28877, 28975, 28977, 29722, 29754; or is a leukocyte antigen-associated gene selected from: HLA-A, HLA-B, HLA-C, HLA-E, HLA-F, HLA-G, HLADRA1, HLA-DRB1, HLA-DRB3/4/5, HLA-DQA1, HLA-DQB1, HLA-DPA1, or HLA-DPB1; or is a blood group associated antigen associated with one or more of the ABO, MNS, Rhesus, Lutheran, Kell, Lewis, Duffy, Kidd, Diego, Yt, Scianna, Dombrock, Colton, Cromer, or Vel blood group antigens.

12. The method of any one of claims 1 to 6 or 8 to 11, or the kit of any one of claims 7 to 11, wherein said forward indexing primer is a primer selected from the group comprising primers of SEQ ID NO: 32 to SEQ ID NO: 39; and/or wherein said reverse indexing primer is a primer selected from the group comprising primers of SEQ ID NO: 40 to SEQ ID NO: 51.

13. The method of any one of claims 1 to 6 or 8 to 12, or the kit of any one of claims 7 to 12, wherein 2, 3, 4, 5, 6, 7, 8, 9, 10 or more target sequences are simultaneously amplified, preferably, wherein 2 or 3 target sequences are simultaneously amplified.

14. The method of any one of claims 1 to 6, or 8 to 13, wherein said method allows for a qualitative detection of the target sequence and/or an organism or virus comprising said target sequence or a sequence being highly similar to the target sequence, preferably having a sequence identity of 97% or more.

15. Use of the method of any one of claims 1 to 6 or 8 to 14, or the kit of any one of claims 7 to 13 for an enrichment for a rapid virus detection; or for an enrichment for a rapid leukocyte antigen-associated gene identification; or for an enrichment for a rapid blood group associated gene identification.

## Patentansprüche

1. Ein Verfahren zur Vorbereitung einer RNA-Probe für ein zielspezifisches Next Generation Sequencing, umfassend die Durchführung einer einstufigen Zielanreicherung in einem einzigen Reaktionsgefäß oder in einem einzigen Reaktionsgemisch, wobei die Anreicherung die Schritte umfasst:
(i) Aussetzen der zu sequenzierenden RNA in einem einzigen Reaktionsgefäß einem Gemisch umfassend eine reverse Transkriptase, eine DNA-Polymerase, und
(a) einen oder mehrere zielspezifische Rückwärtsprimer, die für die Herstellung einer zielspezifischen cDNA geeignet sind; und
(b) einen zielspezifischen Vorwärtsprimer umfassend eine erste Vorwärtsadaptersequenz, und einen zielspezifischen Rückwärtsprimer umfassend eine erste Rückwärtsadaptersequenz, und
(c) einen Vorwärtsindexierungsprimer umfassend eine erste Vorwärtsadaptersequenz, eine Vorwärtsindexierungssequenz und eine zweite Vorwärtsadaptersequenz; und einen Rückwärtsindexierungsprimer, umfassend eine erste Rückwärtsadaptersequenz, eine Rückwärtsindexierungssequenz, wobei die Rückwärtsindexierungssequenz von der Vorwärtsindexierungssequenz verschieden ist, und eine zweite Rückwärtsadaptersequenz;
und
Desoxyribonukleosidtriphosphate (dNTPs); und
(ii) Unterziehen des Reaktionsgemisches von (i) einer Reihe von Temperaturänderungen unter Bedingungen, die ausreichen, um eine Erststrang-cDNA-Kopie von mindestens einem Teil der zu sequenzierenden RNA, vorzugsweise einer Gensequenz, und anschließend ein zielspezifisches Amplikon zu erhalten, das ausgehend vom 5'- bis zum 3'-Ende eine zweite Vorwärtsadaptersequenz, eine Vorwärtsindexierungssequenz, eine erste Vorwärtsadaptersequenz, eine zielspezifische Vorwärtsprimersequenz, Zielsequenz, eine zielspezifische Rückwärtsprimersequenz, eine erste Rückwärtsadaptersequenz, eine Rückwärtsindexierungssequenz und eine zweite Rückwärtsadaptersequenz umfasst.

2. Das Verfahren nach Anspruch 1, zusätzlich umfassend als ersten Schritt die Extraktion von RNA aus einer von einer Person erhaltenen Probe, vorzugsweise durch Probenlyse.

3. Das Verfahren nach Anspruch 1 oder 2, wobei das Verfahren zusätzlich eine Kontrollamplifikation von einer oder mehreren zusätzlichen Zielsequenzen umfasst.

4. Das Verfahren nach einem der Ansprüche 1 bis 3, zusätzlich umfassend einen Schritt der Probenregistrierung, welche vor der Anreicherung durchgeführt wird.

5. Das Verfahren nach einem der Ansprüche 1 bis 4, wobei das Verfahren ferner eine Aufreinigung des wie in Schritt (ii) erhaltenen Amplikons und/oder einen Schritt der Quantifizierung des Amplikons umfasst.

6. Das Verfahren nach einem der Ansprüche 1 bis 5, zusätzlich umfassend einen Schritt des Sequenzierens des wie in Schritt (ii) erhaltenen Amplikons, vorzugsweise mit einem NGS-System wie Illumina, Ion Torrent, Oxford Nanopore oder SMRT Sequencing, optional ferner umfassend das Assemblieren von Sequenzier-Reads.

7. Ein Kit zur Vorbereitung einer RNA-Probe für Next Generation Sequencing in einer einstufigen Zielanreicherung, umfassend: a) eine reverse Transkriptase (RT); b) einen oder mehrere zielspezifische Rückwärtsprimer, die für die Herstellung einer zielspezifischen cDNA geeignet sind, c) einen zielspezifischen Vorwärtsprimer, der eine erste Vorwärtsadaptersequenz umfasst, und einen zielspezifischen Rückwärtsprimer, der eine erste Rückwärtsadaptersequenz umfasst; d) einen Vorwärtsindexierungsprimer, der eine erste Vorwärtsadaptersequenz, eine Vorwärtsindexierungssequenz und eine zweite Vorwärtsadaptersequenz umfasst; und einen Rückwärtsindexierungsprimer, der eine erste Rückwärtsadaptersequenz, eine Rückwärtsindexierungssequenz, wobei die Rückwärtsindexierungssequenz von der Vorwärtsindexierungssequenz verschieden ist, und eine zweite Rückwärtsadaptersequenz umfasst; e) Desoxyribonukleosidtriphosphate (dNTPs); und f) eine DNA-Polymerase.

8. Das Verfahren nach einem der Ansprüche 1 bis 6, oder das Kit nach Anspruch 7, wobei die Adaptersequenz eine Länge von etwa 8 bis 45 Nukleotiden hat; und/oder wobei die Indexierungsprimersequenz eine Länge von etwa 4 bis 20 Nukleotiden hat.

9. Das Verfahren nach einem der Ansprüche 1 bis 6, oder 8, oder das Kit nach Anspruch 7 bis 8, wobei die Adaptersequenz in der Lage ist, an ein Substrat, vorzugsweise einen Sequenzchip oder eine Flow Cell, zu binden.

10. Das Verfahren nach einem der Ansprüche 1 bis 6, oder 8 bis 9, oder das Kit nach einem der Ansprüche 7 bis 9, wobei der zielspezifische Primer oder das zielspezifische Primerpaar spezifisch für eine Zielsequenz ist, wobei die Zielsequenz vorzugsweise ein virales Gen oder ein Teil eines viralen Genoms, ein leukozytenantigen-assoziiertes Gen oder ein blutgruppenantigen-assoziiertes Gen ist; noch bevorzugter wobei die Zielsequenz ein virales Gen eines Coronavirus ist, vorzugsweise ein SARS-CoV-2-Virus-Gen oder -Genomabschnitt, oder ein Teil davon, noch bevorzugter die 5'UTR, 3'UTR, ORF1ab, Orf3a, Orf6, Orf7a, Orf7b, Orf8, Orf10, M-Genregion, E-Genregion, N-Genregion, oder S-Genregion von SARS-CoV-2.

11. Das Verfahren nach einem der Ansprüche 1 bis 6, oder 8 bis 10, oder das Kit nach einem der Ansprüche 7 bis 10, wobei die Zielsequenz eine oder mehrere der folgenden Nukleotidpositionen gemäß der Nukleotidnummerierung des Referenzgenoms von SARS-CoV-2 (Referenzgenom mit NCBI-Referenzsequenznummer: NC_045512.2; SEQ ID NO: 63) umfasst: 100, 733, 1264 , 2749, 3267, 3828, 5388, 5648, 6319, 6573, 6613, 6954, 7600, 7851, 10667, 11078, 11288-11296, 11824, 12964, 12778, 13860, 17259, 19602, 19656, 21614, 21621, 21638, 21765-21770, 21974, 21991-21993, 22132, 22812, 23012, 23063, 23271, 23525, 23604, 23709, 24506, 24642, 24914, 26149, 27853, 27972, 28048, 28111, 28167, 28253, 28262, 28280, 28512, 28628, 28877, 28975, 28977, 29722, 29754; oder ein leukozytenantigen-assoziiertes Gen ist, ausgewählt aus: HLA-A, HLA-B, HLA-C, HLA-E, HLA-F, HLA-G, HLA-DRA1, HLA-DRB1, HLA-DRB3/4/5, HLA-DQA1, HLA-DQB1, HLA-DPA1 oder HLA-DPB1; oder ein blutgruppen-assoziiertes Antigen ist, das mit einem oder mehreren der ABO-, MNS-, Rhesus-, Lutheran-, Kell-, Lewis-, Duffy-, Kidd-, Diego-, Yt-, Scianna-, Dombrock-, Colton-, Cromer- oder Vel-Blutgruppenantigene assoziiert ist.

12. Das Verfahren nach einem der Ansprüche 1 bis 6 oder 8 bis 11, oder das Kit nach einem der Ansprüche 7 bis 11, wobei der Vorwärtsindexierungsprimer ein Primer ist, der aus der Gruppe ausgewählt ist, die Primer der SEQ ID NO: 32 bis SEQ ID NO: 39 umfasst; und/oder wobei der Rückwärtsindexierungsprimer ein Primer ist, der aus der Gruppe ausgewählt ist, die Primer der SEQ ID NO: 40 bis SEQ ID NO: 51 umfasst.

13. Das Verfahren nach einem der Ansprüche 1 bis 6 oder 8 bis 12, oder das Kit nach einem der Ansprüche 7 bis 12, wobei 2, 3, 4, 5, 6, 7, 8, 9, 10 oder mehr Zielsequenzen gleichzeitig amplifiziert werden, vorzugsweise, wobei 2 oder 3 Zielsequenzen gleichzeitig amplifiziert werden.

14. Das Verfahren nach einem der Ansprüche 1 bis 6, oder 8 bis 13, wobei das Verfahren einen qualitativen Nachweis der Zielsequenz und/oder eines Organismus oder Virus ermöglicht, der die Zielsequenz oder eine Sequenz, die der Zielsequenz sehr ähnlich ist, vorzugsweise mit einer Sequenzidentität von 97 % oder mehr, umfasst.

15. Verwendung des Verfahrens nach einem der Ansprüche 1 bis 6 oder 8 bis 14, oder des Kits nach einem der Ansprüche 7 bis 13 für eine Anreicherung für einen schnellen Virusnachweis; oder für eine Anreicherung für eine schnelle leukozytenantigenassoziierte Genidentifizierung; oder für eine Anreicherung für eine schnelle blutgruppen-assoziierte Genidentifizierung.

## Revendications

1. Une méthode de préparation d'un échantillon d'ARN pour un séquençage de nouvelle génération spécifique à une cible, comprenant la réalisation d'un enrichissement de cible en une étape dans un récipient de réaction unique ou dans un mélange réactionnel unique, dans lequel ledit enrichissement comprend les étapes:
(i) exposer l'ARN à séquencer dans un seul récipient de réaction à un mélange comprenant une transcriptase inverse, une ADN polymérase, et
(a) une ou plusieurs amorces inverses spécifiques d'une cible, adaptées à la préparation d'un ADNc spécifique d'une cible; et
(b) une amorce spécifique de cible inverse comprenant une première séquence d'adaptateur sens, et une amorce spécifique de cible inverse comprenant une première séquence d'adaptateur inverse, et
(c) une amorce d'indexation directe comprenant une première séquence d'adaptateur direct, une séquence d'indexation directe et une seconde séquence d'adaptateur direct; et une amorce d'indexation inverse, comprenant une première séquence d'adaptateur inverse, une séquence d'indexation inverse, la séquence d'indexation inverse étant différente de la séquence d'indexation avant, et une seconde séquence d'adaptateur inverse ;
et
les désoxyribonucléoside triphosphates (dNTP); et
(ii) soumettre le mélange réactionnel de (i) à une série de changements de température dans des conditions suffisantes pour produire une copie d'ADNc de premier brin d'au moins une partie de l'ARN à séquencer, de préférence une séquence de gène, et ensuite un amplicon spécifique cible comprenant à partir de de l'extrémité 5' à l'extrémité 3' une seconde séquence d'adaptateur avant, une séquence d'indexation avant, une première séquence d'adaptateur avant, une séquence d'amorce spécifique de cible avant, une séquence cible, une séquence d'amorce spécifique de cible inverse, une première séquence d'adaptateur inverse, une séquence d'indexation inverse et une seconde séquence d'adaptateur inverse.

2. La méthode selon la revendication 1, comprenant en outre comme première étape l'extraction de l'ARN d'un échantillon obtenu à partir d'un sujet, de préférence par lyse d'échantillon.

3. La méthode selon la revendication 1 ou 2, dans laquelle la méthode comprend en outre une amplification de contrôle d'une ou plusieurs séquences cibles supplémentaires.

4. La méthode de l'une des revendications 1 à 3, comprenant en outre une étape d'enregistrement de l'échantillon, effectuée avant l'enrichissement.

5. La méthode de l'une des revendications 1 à 4, dans laquelle ladite méthode comprend en outre une purification de l'amplicon obtenu à l'étape (ii) et/ou une étape de quantification de l'amplicon.

6. La méthode de l'une des revendications 1 à 5, comprenant en outre une étape de séquençage de l'amplicon obtenu à l'étape (ii), de préférence avec un système NGS tel que *Illumina, Ion Torrent, Oxford Nanopore,* ou *SMRT Sequencing,* comprenant éventuellement en outre l'assemblage des séquences lues.

7. Kit pour préparer un échantillon d'ARN pour un séquençage de nouvelle génération dans un enrichissement de cible en une étape comprenant : a) une transcriptase inverse (RT) ; b) une ou plusieurs amorces inverses spécifiques d'une cible, adaptées à la préparation d'un ADNc spécifique d'une cible, c) une amorce spécifique d'une cible directe comprenant une première séquence d'adaptateur direct, et une amorce spécifique d'une cible inverse comprenant une première séquence d'adaptateur inverse ; d) une amorce d'indexation directe comprenant une première séquence d'adaptateur direct, une séquence d'indexation directe et une seconde séquence d'adaptateur direct ; et une amorce d'indexation inverse, comprenant une première séquence d'adaptateur inverse, une séquence d'indexation inverse, la séquence d'indexation inverse étant différente de la séquence d'indexation avant, et une seconde séquence d'adaptateur inverse ; e) désoxyribonucléoside triphosphates (dNTP) ; et f) une ADN polymérase.

8. La méthode de l'une des revendications 1 à 6, ou le kit de la revendication 7, dans laquelle ladite séquence adaptatrice a une longueur d'environ 8 à 45 nucléotides ; et/ou dans lequel ladite séquence d'amorce d'indexation a une longueur d'environ 4 à 20 nucléotides.

9. La méthode de l'une des revendications 1 à 6, ou 8, ou le kit de la revendication 7 à 8, dans lequel ladite séquence adaptatrice est capable de se lier à un substrat, de préférence une puce à séquence ou une cellule de flux.

10. La méthode de l'une des revendications 1 à 6, ou 8 à 9, ou le kit de l'une des revendications 7 à 9, dans laquelle ladite amorce spécifique de la cible ou ladite paire d'amorces spécifiques de la cible est spécifique d'une séquence cible, dans laquelle ladite séquence cible est de préférence un gène viral ou une partie d'un génome viral, un gène associé à un antigène leucocytaire, ou un gène associé à un antigène de groupe sanguin ; de préférence, ladite séquence cible est un gène viral d'un coronavirus, de préférence un gène ou une partie génomique du virus SARS-CoV-2, ou une partie de celui-ci, de préférence la région 5'UTR, 3'UTR, ORFlab, Orf3a, Orf6, Orf7a, Orf7b, Orf8, Orf10, la région du gène M, la région du gène E, la région du gène N ou la région du gène S du SARS-CoV-2.

11. La méthode de l'une des revendications 1 à 6, ou 8 à 10, ou le kit de l'une des revendications 7 à 10, dans laquelle ladite séquence cible comprend une ou plusieurs des positions nucléotidiques suivantes selon la numération des nucléotides du génome de référence du SARS-CoV-2 (génome de référence avec NCBI Reference Sequence No : NC_045512.2 ; SEQ ID NO : 63) : 100, 733, 1264, 2749, 3267, 3828, 5388, 5648, 6319, 6573, 6613, 6954, 7600, 7851, 10667, 11078, 11288-11296, 11824, 12964, 12778, 13860, 17259, 19602, 19656, 21614, 21621, 21638, 21765-21770, 21974, 21991-21993, 22132, 22812, 23012, 23063, 23271, 23525, 23604, 23709, 24506, 24642, 24914, 26149, 27853, 27972, 28048, 28111, 28167, 28253, 28262,28280, 28512, 28628, 28877, 28975, 28977, 29722, 29754 ; ou est un gène associé à un antigène leucocytaire choisi parmi : HLA-A, HLA-B, HLA-C, HLA-E, HLA-F, HLA-G, HLADRA1, HLA-DRB1, HLA-DRB3/4/5, HLA-DQA1, HLA-DQB1, HLA-DPA1, ou HLA-DPB1 ; ou est un antigène de groupe sanguin associé à un ou plusieurs des antigènes de groupe sanguin ABO, MNS, Rhésus, Lutheran, Kell, Lewis, Duffy, Kidd, Diego, Vt, Scianna, Dombrock, Colton, Cromer ou Vel.

12. La méthode de l'une des revendications 1 à 6 ou 8 à 11, ou le kit de l'une des revendications 7 à 11, dans laquelle ladite amorce d'indexation avant est une amorce sélectionnée dans le groupe comprenant les amorces de SEQ ID NO : 32 à SEQ ID NO : 39 ; et/ou dans laquelle ladite amorce d'indexation arrière est une amorce sélectionnée dans le groupe comprenant les amorces de SEQ ID NO : 40 à SEQ ID NO : 51.

13. La méthode de l'une des revendications 1 à 6 ou 8 à 12, ou le kit de l'une des revendications 7 à 12, dans lequel 2, 3, 4, 5, 6, 7, 8, 9, 10 séquences cibles ou plus sont amplifiées simultanément, de préférence, dans lequel 2 ou 3 séquences cibles sont amplifiées simultanément.

14. La méthode de l'une des revendications 1 à 6, ou 8 à 13, dans laquelle ladite méthode permet une détection qualitative de la séquence cible et/ou d'un organisme ou d'un virus comprenant ladite séquence cible ou une séquence très similaire à la séquence cible, de préférence ayant une identité de séquence de 97 % ou plus.

15. Utilisation de la méthode de l'une des revendications 1 à 6 ou 8 à 14, ou du kit de l'une des revendications 7 à 13 pour un enrichissement en vue de la détection rapide d'un virus; ou pour un enrichissement en vue de l'identification rapide d'un gène associé à l'antigène leucocytaire; ou pour un enrichissement en vue de l'identification rapide d'un gène associé à un groupe sanguin.
